(19) Europäisches Patentamt
European Patent Office
Office européen des brevets

(11) EP 3 483 234 B1

(12) **EUROPÄISCHE PATENTSCHRIFT**

(45) Veröffentlichungstag und Bekanntmachung des Hinweises auf die Patenterteilung:
**02.06.2021 Patentblatt 2021/22**

(21) Anmeldenummer: **18200972.0**

(22) Anmeldetag: **19.09.2014**

(51) Int Cl.:
***C10M 133/06*** (2006.01) ***C10M 159/12*** (2006.01)
***C10L 1/222*** (2006.01) ***C10L 1/224*** (2006.01)
***C10L 10/04*** (2006.01) ***C10L 10/18*** (2006.01)
***C10M 133/16*** (2006.01) ***C10L 1/22*** (2006.01)
***C07C 209/20*** (2006.01) ***C07C 211/63*** (2006.01)
***C07C 227/14*** (2006.01) ***C10L 10/06*** (2006.01)
***F02M 25/14*** (2006.01) *C10N 30/04* (2006.01)
*C10N 30/08* (2006.01) *C10N 30/00* (2006.01)
*C10N 40/25* (2006.01) *C10N 70/00* (2006.01)

(54) **VERWENDUNG SPEZIELLER DERIVATE QUATERNISIERTER STICKSTOFFVERBINDUNGEN ALS ADDITIVE IN KRAFTSTOFFEN**

USE OF SPECIALISED DERIVATIVES OF QUATERNISED NITROGEN COMPOUNDS AS ADDITIVES IN FUELS

UTILISATION DE DÉRIVÉS SPÉCIAUX DE COMPOSÉS AZOTÉS QUATERNISÉS EN TANT QU'ADDITIFS DANS DES CARBURANTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.09.2013 EP 13185288**
**07.04.2014 EP 14163707**

(43) Veröffentlichungstag der Anmeldung:
**15.05.2019 Patentblatt 2019/20**

(62) Dokumentnummer(n) der früheren Anmeldung(en) nach Art. 76 EPÜ:
**14767022.8 / 3 046 999**

(73) Patentinhaber: **BASF SE**
**67056 Ludwigshafen am Rhein (DE)**

(72) Erfinder:
- **HANSCH, Markus**
  **67346 Speyer (DE)**
- **BÖHNKE, Harald**
  **68163 Mannheim (DE)**
- **GRABARSE, Wolfgang**
  **68163 Mannheim (DE)**
- **VÖLKEL, Ludwig**
  **67117 Limburgerhof (DE)**
- **PERETOLCHIN, Maxim**
  **67466 Lambrecht (DE)**

(56) Entgegenhaltungen:
**WO-A1-2013/087701**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 483 234 B1

## Beschreibung

Die vorliegende Erfindung betrifft die Verwendung spezieller quaternisierter Stickstoff-verbindungen, welche zusätzlich einer speziellen Umesterung oder Amidierung unterzogen wurden, als Kraftstoffadditiv, wie insbesondere als Detergensadditiv; zur Verringerung oder Verhinderung von Ablagerungen in den Einspritzsystemen von direkteinspritzenden Dieselmotoren, insbesondere in Common-Rail-Einspritzsystemen, zur Verringerung des Kraftstoffverbrauches von direkteinspritzenden Dieselmotoren, insbesondere von Dieselmotoren mit Common-Rail-Einspritzsystemen, und zur Minimierung des Leistungsverlustes (power loss) in direkteinspritzenden Dieselmotoren, insbesondere in Dieselmotoren mit Common-Rail-Einspritzsystemen; sowie als Additiv für Ottokraftstoffe, insbesondere zum Betrieb von DISI Motoren.

## Stand der Technik:

Bei direkteinspritzenden Dieselmotoren wird der Kraftstoff durch eine direkt in den Brennraum des Motors reichende Mehrloch-Einspritzdüse eingespritzt und feinst verteilt (vernebelt), anstatt wie beim klassischen (Kammer-) Dieselmotor in eine Vor- oder Wirbelkammer eingeführt zu werden. Der Vorteil der direkteinspritzenden Dieselmotoren liegt in ihrer für Dieselmotoren hohen Leistung und einem dennoch geringen Verbrauch. Außerdem erreichen diese Motoren ein sehr hohes Drehmoment schon bei niedrigen Drehzahlen.

Zurzeit werden im Wesentlichen drei Verfahren eingesetzt, um den Kraftstoff direkt in den Brennraum des Dieselmotors einzuspritzen: die konventionelle Verteilereinspritzpumpe, das Pumpe-Düse-System (Unit-Injector-System bzw. Unit-Pump-System) und das Common-Rail-System.

Beim Common-Rail-System wird der Dieselkraftstoff von einer Pumpe mit Drücken bis zu 2000 bar in eine Hochdruckleitung, die Common-Rail gefördert. Ausgehend von der Common-Rail laufen Stichleitungen zu den verschiedenen Injektoren, die den Kraftstoff direkt in den Brennraum injizieren. Dabei liegt auf der Common-Rail stets der volle Druck an, was eine Mehrfacheinspritzung oder eine spezielle Einspritzform ermöglicht. Bei den anderen Injektionssystemen ist dagegen nur eine geringere Variation der Einspritzung möglich. Die Einspritzung beim Common-Rail wird im Wesentlichen in drei Gruppen unterteilt: (1.) Voreinspritzung, durch die im Wesentlichen eine weichere Verbrennung erreicht wird, so dass harte Verbrennungsgeräusche (" Nageln" ) vermindert werden und der Motorlauf ruhig erscheint; (2.) Haupteinspritzung, die insbesondere für einen guten Drehmomentverlauf verantwortlich ist; und (3.) Nacheinspritzung, die insbesondere für einen geringen $NO_x$-Wert sorgt. Bei dieser Nacheinspritzung wird der Kraftstoff in der Regel nicht verbrannt, sondern durch Restwärme im Zylinder verdampft. Das dabei gebildete Abgas-/Kraftstoffgemisch wird zur Abgasanlage transportiert, wo der Kraftstoff in Gegenwart geeigneter Katalysatoren als Reduktionsmittel für die Stickoxide $NO_x$ wirkt.

Durch die variable, zylinderindividuelle Einspritzung kann beim Common-Rail-Einspritzsystem der Schadstoffausstoß des Motors, z.B. der Ausstoß von Stickoxiden ($NO_x$), Kohlenmonoxid (CO) und insbesondere von Partikeln (Ruß), positiv beeinflusst werden. Dies ermöglicht beispielsweise, dass mit Common-Rail-Einspritzsystemen ausgerüstete Motoren der Euro 4-Norm theoretisch auch ohne zusätzlichen Partikelfilter genügen können.

In modernen Common-Rail-Dieselmotoren können sich unter bestimmten Bedingungen, beispielsweise bei Verwendung von biodieselhaltigen Kraftstoffen oder von Kraftstoffen mit Metall-Verunreinigungen wie Zink-Verbindungen, Kupfer-Verbindungen, Bleiverbindungen und weiteren Metallverbindungen, an den Injektoröffnungen Ablagerungen bilden, die das Einspritzverhalten des Kraftstoffs negativ beeinflussen und dadurch die Performance des Motors beeinträchtigen, d.h. insbesondere die Leistung verringern, aber zum Teil auch die Verbrennung verschlechtern. Die Bildung von Ablagerungen wird durch bauliche Weiterentwicklungen der Injektoren, insbesondere durch die Veränderung der Geometrie der Düsen (engere, konische Öffnungen mit abgerundetem Auslass) noch verstärkt. Für eine dauerhaft optimale Funktionsweise von Motor und Injektoren müssen solche Ablagerungen in den Düsenöffnungen durch geeignete Kraftstoffadditive verhindert oder reduziert werden

In den Einspritzsystemen modernen Dieselmotoren verursachen Ablagerungen signifikante Performance-Probleme. Weit verbreitet ist die Erkenntnis, dass derartige Ablagerungen in den Sprühkanälen zu einer Verringerung des Kraftstoffflusses und damit zu Leistungsverlusten (power loss) führen können. Ablagerungen an der Injektorspitze beeinträchtigen dagegen die optimale Ausbildung von Kraftstoff-Sprühnebel und bedingen dadurch eine verschlechterte Verbrennung und damit verbunden höhere Emissionen und vermehrten Kraftstoffverbrauch. Im Gegensatz zu diesen herkömmlichen, "äußeren" Ablagerungsphänomenen bereiten auch" interne" Ablagerungen (zusammengefasst als innere Diesel-Injektor-Ablagerungen (IDID)) in bestimmten Teilen der Injektoren, wie an der Düsennadel, am Steuerkolben, am Ventilkolben, am Ventilsitz, an der Ansteuereinheit und an den Führungen dieser Komponenten zunehmend Performance-Probleme. Herkömmliche Additive zeigen eine unzureichende Wirkung gegen diese IDIDs.

Aus der EP-A-2 033 945 sind Kaltfließverbesserer bekannt, welche durch Quaternisierung spezieller tertiärer Monoamine, die wenigstens einen $C_8$-$C_{40}$-Alkylrest tragen, mit einem $C_1$-$C_4$-Alkylester spezieller Carbonsäuren hergestellt werden. Beispiele solcher Carbonsäureester sind Dimethyloxalat, Dimethylmaleat, Dimethylphthalat und Dimethylfumarat. Andere Anwendungen als zur Verbesserung des CFPP Werts von Mitteldestillaten sind in der EP-A-2 033 945 nicht belegt.

Quaternäre Ammoniumsalze von alpha-Hydroxycarbonsäuren werden in der EP-A-1 254 889 als Reinigungsmittel für elektronische Bauteile vorgeschlagen.

Weiterhin beschreibt die Japanische Patentanmeldung, Anmeldenummer 61-012197, die Verwendung quaternärer Ammoniumsalze von organischen Carbonsäuren als Oberflächenaktives mittel oder Rohmaterial für Medikamente oder Kosmetika.

Die GB-A-2496514 beschreibt alkylsubstituierte Ammonium-Verbindungen, hergestellt durch Quaternisierung mittels verschiedenen konventionellen Quaternisierungsmitteln, wie insbesondere Chloriden, Nitraten und Acetaten, und deren Verwenduing als Kraftstoffadditiv. Die Verwendung von vollveresterten Polycarbonsäure-Polyalkylestern wird darin nicht offenbart.

Aus der WO2013/087701 der Anmelderin sind quaternisierten Alkylaminen bzw. damit additivierte Kraft- und Schmierstoffzusammensetzungen bekannt, wobei als Quaternisierungsmittel z.B. Dicarbonsäureester vorgeschlagen werden. Die aus dem Stand der Technik bekannten Verbindungen weisen jedoch noch in Bezug auf Handling, Lagerstabilität und Motorölverträglichkeit Nachteile auf.

Es bestand daher die Aufgabe, verbesserte Kraftstoffadditive bereitzustellen, welche oben beschriebene Nachteile nicht mehr aufweisen, jedoch weiterhin Ablagerungen in direkt oder indirekt einspritzenden Dieselmotoren, wie insbesondere Ablagerungen in der Injektorspitze und interne Injektorablagerungen beim Betrieb von Common-Rail-Dieselmotoren in wirksamer Weise verhindern.

## Kurze Beschreibung der Erfindung:

Es wurde nun überraschenderweise gefunden, dass obige Aufgabe durch Bereitstellung spezieller Derivate von mit Polycarbonsäureestern quaternisierten Stickstoffverbindungen, wie z.B. Alkylaminen, bzw. damit additivierter Kraftstoffzusammensetzungen gelöst wird.

Überraschenderweise sind die erfindungsgemäßen Additive, wie insbesondere durch die beiliegenden Anwendungsbeispiele veranschaulicht, überraschend gut handhabbar, besitzen überraschend gute Motoröl-Kompatibilität, und besitzen weiterhin ausgezeichnete Wirksamkeit in direkt und indirekt einspritzenden Dieselmotoren, wie insbesondere als Additiv zur Verringerung von Powerloss und Verringerung von internen Ablagerungen.

## Figurenbeschreibung:

Figur 1 zeigt den Ablauf eines einstündigen Motorentestzyklus gemäß CEC F-098-08.

## Detaillierte Beschreibung der Erfindung:

Die Erfindung wird in den angefügten Patentansprüchen genauer definiert.

### A1) Spezielle Ausführungsformen

Die vorliegende Erfindung betrifft insbesondere folgende spezielle Ausführungsformen:
Ein erster Gegenstand der Erfindung betrifft die Verwendung eines eine quaternisierte Stickstoffverbindung umfassenden Reaktionsprodukts oder einer aus dem Reaktionsprodukt durch Aufreinigung erhaltene, eine quaternisierte Stickstoffverbindung enthaltende Teilfraktion davon, wobei das Reaktionsprodukt erhältlich ist durch

a) Umsetzung einer quaternisierbaren Stickstoffverbindung, wie z.B. eines quaternisierbaren Alkylamins, enthaltend wenigstens eine quaternisierbare, insbesondere tertiäre Aminogruppe mit einem Quaternierungsmittel, das die wenigstens eine quaternisierbare, insbesondere tertiäre Aminogruppe in eine quaternäre Ammoniumgruppe überführt,
wobei das Quaternierungsmittel der insbesondere mehrfach veresterte, bevorzugt vollveresterte Methyl- oder Ethyl-Ester einer aliphatischen, cycloaromatischen oder cycloaliphatischen, insbesondere aliphatischen Polycarbonsäure, insbesondere einer Dicarbonsäure, wie insbesondere der Methyl- oder Ethylester einer cycloaliphatischen oder aliphatischen, insbesondere aliphatischen Dicarbonsäure, ist; und
b) Umesterung der verbleibenden Estergruppe des Quaternisierungsprodukts aus Stufe a) (hierin auch bezeichnet als quatenisierter Polycarbonsäureester; bzw. quaternisiertes Ammoniumsalz eines Polycarbonsäureesters) mit einem wie in Anspruch 1 definierten Polyalkoxylat oder Polyol-gestartetem Polyalkoxylat; als Kraftstoffadditiv in einem Anteil von 10 - 5.000 Gew.-ppm, bezogen auf die Gesamtmenge des Kraftstoffs;
wobei insbesondere die umgeesterte Form (d.h. das Produkt aus Stufe b)) des Quaternisierungsprodukts (d.h. das

Produkt aus Stufe a)) hydrophoberen Charakter als vor der Umesterung besitzt, wodurch insbesondere die Öllöslichkeit der so erhaltenen Produkte weiter verbessert wird.

Insbesondere wird erfindungsgemäß im Quaternisierungsprodukt aus Stufe a) durch die Umesterung wenigstens eine Methyl- oder Ethylestergruppe durch eine Estergruppe mit längerkettigem Hydrocarbylrest ersetzt; wie dies insbesondere durch Umesterung mit geeigneten Polyalkoxylaten und polyolgestarteten Polyalkoxylaten, wie z.B. in folgendem Abschnitt A5) näher erläutert, erreichbar ist.

Eine geeignetes Kriterium zur Auswahl einer Anzahl erfindungsgemäß durch Umesterung einführbarer Gruppen in das Quaternisierungsprodukt der Stufe a) ist der Verteilungskoeffizient ("logPow; oder" logP)" die zur Umesterung bevorzugte eingesetzten Verbindungen (Alkohole und; wie insbesondere die in Abschnitt A5) beschriebenen Verbindungsklassen). Dieser logPow oder logP- Wert geeigneter Verbindungen ist gewöhnlich größer als 0,7, insbesondere größer als 0,8, größer als 0,9, größer als 1, oder größer als 1,1, und nach oben grundsätzlich offen, soweit die Additiveigenschaften dadurch nicht negativ beeinflusst werden und/oder die Öllöslichkeit des Additivs dadurch verbessert wird. Beispielsweise kann der Wert in einem Bereich von 1,2 bis 30, 1,3 bis 25, 1,3 bis 20 oder 1,3 bis 15 oder 1,3 bis 13 oder 2 bis 10 oder 2 bis 5 liegen.

Eine besondere Ausführungsform davon betrifft die Verwendung obiger Reaktionsprodukte oder Teilfunktionen davon als Additiv zur Verringerung des Kraftstoffverbrauches von direkteinspritzenden Dieselmotoren, insbesondere von Dieselmotoren mit Common-Rail-Einspritzsystemen, und/oder zur Minimierung des Leistungsverlustes (powerloss) in direkteinspritzenden Dieselmotoren, insbesondere in Dieselmotoren mit Common-Rail-Einspritzsystemen, wie insbesondere Leisitungsverlust durch Metalle, wie Zn, Na und /oder K und Verbindungen welche dies Metalle enthalten, wie deren Salze.

Eine weitere besondere Ausführungsform betrifft die Verwendung obiger Reaktionsprodukte oder Teilfunktionen davon als Ottokraftstoffadditiv zur Verringerung von Ablagerungen im Einlasssystem eines Ottomotors, wie insbesondere DISI (Direct Injection Spark Ignition) und PFI (Port Fuel Injector) -Motoren .

Eine weitere besondere Ausführungsform betrifft die Verwendung obiger Reaktionsprodukte oder Teilfunktionen davon als Dieselkraftstoffadditiv zur Verringerung und/oder Vermeidung von Ablagerungen in den Einspritzsystemen, wie insbesondere der Internal Diesel Injector Deposits (IDID) und / oder von Ventilkleben in direkteinspritzenden Dieselmotoren, insbesondere in Common-Rail-Einspritzsystemen.

In einer weiteren besonderen Ausführungsform der obigen Verwendungen ist die quaternisierbare Stickstoffverbindung ausgewählt ist unter:

a) wenigstens einem Alkylamin der folgenden allgemeinen Formel 3:

$$R_aR_bR_cN \qquad (3)$$

worin

wenigstens einer der Reste $R_a$, $R_b$ und $R_c$, wie z.B. 1 oder 2 der Reste, für einen geradkettigen oder verzweigte, gesättigten oder ungesättigten $C_8$-$C_{40}$-Hydrocarbylrest (insbesondere geradkettigen oder verzweigte $C_8$ - $C_{40}$-Alkyl) steht und die übrigen Reste für gleiche oder verschiedene, geradkettige oder verzweigte, gesättigte oder ungesättigten $C_1$-$C_6$-Hydrocarbylreste (insbesondere $C_1$-$C_6$-Alkyl) stehen; oder

worin alle Reste $R_a$, $R_b$ und $R_c$ für gleiche oder verschiedene geradkettige oder verzweigte, gesättigten oder ungesättigten $C_8$-$C_{40}$-Hydrocarbylreste, insbesondere geradkettige oder verzweigte $C_8$ - $C_{40}$-Alkyl-Reste stehen;

b) wenigstens einem Polyalken-substituierten Amin, enthaltend wenigstens eine quaternisierbare, insbesondere tertiäre, Aminogruppe;

c) wenigstens einem Polyether-substituierten Amin, enthaltend wenigstens eine quaternisierbare, insbesondere tertiäre, Aminogruppe; und

d) wenigstens einem Reaktionsprodukt eines hydrocarbylsubstituierten Acylierungsmittels und einer Verbindung, enthaltend ein Stickstoff- oder Sauerstoffatom und zusätzlich enthaltend wenigstens eine quaternisierbare Aminogruppe; und

e) Mischungen davon.

In einer weiteren besonderen Ausführungsform der obigen Verwendungen ist das Quaternierungsmittel eine Verbindung der allgemeinen Formel 2

$$R_1OC(O)\text{-}A\text{-}C(O)OR_{1a} \qquad (2)$$

worin

$R_1$ und $R_{1a}$ unabhängig voneinander für einen Methyl- oder Ethylrest, steht und A für eine chemische Bindung (d.h. Kohlenstoff-Kohlenstoff-Einfachbindung) oder einen gegebenenfalls ein- oder mehrfach, wie z.B. 1-, 2-, 3- oder 4-fach, substituiertes Hydrocarbylen, wie insbesondere ein gegebenenfalls ein- oder mehrfach, wie z.B. 2-, 3- oder 4-fach, substituiertes Alkylen oder Alkenylen, oder für einen gegebenenfalls substituierten einkernigen Arylen, insbesondere Phenylen,-oder Cycloalkylen-, insbesondere Cyclopentylen und Cyclohexylen-Rest steht. Substituenten der Gruppe A sind z.B. ausgewählt unter Ketogruppen, - COOH, - COO-Alkyl, insbesondere COO-Niedrigalkyl, - OH, -SH, -CN, Amino, $-NO_2$, Alkyl, insbesondere $C_1$-$C_6$ Alkyl-, oder Alkenyl-, insbesondere $C_2$-$C_6$-AlkenylGruppen.

In einer weiteren besonderen Ausführungsform der obigen Verwendungen ist das quaternisierbare tertiäre Amin eine Verbindung der Formel 3, worin wenigstens zwei der Reste $R_a$, $R_b$ und $R_c$ gleich oder verschieden sind und für einen geradkettigen oder verzweigte, $C_{10}$-$C_{20}$-Alkylrest steht und der übrigen Rest für $C_1$-$C_4$-Alkyl steht.

In einer weiteren besonderen Ausführungsform der obigen Verwendungen ist das Quaternierungsmittel ausgewählt unter Di- methyl- oder ethylphthalaten und insbesondere Dimethyl- oder Diethyl- oxalaten.

In einer weiteren besonderen Ausführungsform der obigen Verwendungen ist der Kraftstoff ausgewählt unter Dieselkraftstoffen, Biodieselkraftstoffen, Ottokraftstoffen, und Alkanol-haltigen Ottokraftstoffen.

Ein weiterer Gegenstand der Erfindung betrifft eine quaternisierte Stickstoffverbindung gemäß obiger Definition.

Ein weiterer Gegenstand der Erfindung betrifft ein Verfahren zur Herstellung derartiger quaternisierter Stickstoffverbindungen, umfassend

a) die Umsetzung einer quaternisierbaren Stickstoffverbindung gemäß obiger Definition, wie z.B. eines quaternisierbaren Alkylamins, enthaltend wenigstens eine quaternisierbare, tertiäre Aminogruppe mit einem Quaternierungsmittel, das die wenigstens eine tertiäre Aminogruppe in eine quaternäre Ammoniumgruppe überführt,
wobei das Quaternierungsmittel der (insbesondere vollveresterte) Methyl- oder Ethylester einer aliphatischen, cycloaromatischen oder cycloaliphatischen Polycarbonsäure, insbesondere einer Dicarbonsäure, ist;
b) die Umesterung der verbleibenden Estergruppe des Quaternisierungsprodukts der Stufe a) ( d.h. des quatenisierten Polycarbonsäureesters bzw. des quaternisierten Ammoniumsalzes eines Polycarbonsäureesters);
wobei insbesondere die umgeesterte Form hydrophoberen Charakter besitzt, und z.B. einen höheren Anteil an Kohlenstoffatomen aufweist, wie oben definiert

Ein weiterer Gegenstand der Erfindung betrifft ein Additivkonzentrat, enthaltend in Kombination mit weiteren Diesel- oder Ottokraftstoffadditiven wenigstens eine quaternisierte Stickstoffverbindung gemäß obiger Definition oder hergestellt nach einem Verfahren gemäß obiger Definition.

Ein weiterer Gegenstand der Erfindung betrifft eine Kraftstoffzusammensetzung, enthaltend in einer Hauptmenge eines üblichen Kraftstoffs d.h. marktüblichen Kraftstoffs, der ggf. bereits andere übliche Additive, wie hierin beschrieben enthält, nicht aber ein erfindungsgemäßes Quaternisierungsprodukt gemäß obiger Definition) einen wirksamen (d.h. den gewünschten Additiveffekt herbeiführenden) Anteil (nämlich 10 bis 5000 Gew.-ppm, vorzugsweise bei 20 bis 1500 Gew.-ppm, insbesondere bei 25 bis 1000 Gew.-ppm, vor allem bei 30 bis 750 Gew.-ppm, jeweils bezogen auf die Gesamtmenge an) wenigstens eines eine quaternisierte Stickstoffverbindung umfassenden Reaktionsprodukts oder eine aus dem Reaktionsprodukt durch Aufreinigung erhaltene, eine quaternisierte Stickstoffverbindung enthaltende Teilfraktion davon, wobei das Reaktionsprodukt erhältlich ist durch

a) die Umsetzung einer quaternisierbaren Stickstoffverbindung gemäß obiger Definition, wie z.B. eines quaternisierbaren Alkylamins, enthaltend wenigstens eine quaternisierbare, tertiäre Aminogruppe mit einem Quaternierungsmittel, das die wenigstens eine tertiäre Aminogruppe in eine quaternäre Ammoniumgruppe überführt,
wobei das Quaternierungsmittel der Methyl- oder Ethylester einer aliphatischen, cycloaromatischen oder cycloaliphatischen Polycarbonsäure, insbesondere einer Dicarbonsäure, ist; und
b) die Umesterung der verbleibenden Estergruppe des Quaternisierungsprodukts der Stufe a) (d.h. des quaternisierten Polycarbonsäureesters bzw. des quaternisierten Ammoniumsalzes eines Polycarbonsäureesters) mit einer wie oben definierten Verbindung;
wobei insbesondere die umgeesterte oder amidierte Form hydrophoberen Charakter besitzt, und z.B. einen höheren Anteil an Kohlenstoffatomen aufweist, wie oben definiert

In einer besonderen Ausführungsform der erfindungsgemäßen Kraftstoffzusammensetzung ist die quaternisierbare Stickstoffverbindung ausgewählt unter

a) wenigstens einem Alkylamin der obigen allgemeinen Formel 3
b) wenigstens einem Polyalken-substituierten Amin, enthaltend wenigstens eine quaternisierbare, insbesondere

tertiären, Aminogruppe;

c) wenigstens einem Polyether-substituierten Amin, enthaltend wenigstens eine quaternisierbare, insbesondere tertiäre, Aminogruppe; und

d) wenigstens einem Reaktionsprodukt eines hydrocarbylsubstituierten Acylierungsmittels und einer Verbindung, enthaltend ein Stickstoff- oder Sauerstoffatom und zusätzlich enthaltend wenigstens eine quaternisierbare Aminogruppe; und

e) Mischungen davon.

In einer weiteren besonderen Ausführungsform der erfindungsgemäßen Kraftstoffzusammensetzung ist das Quaternierungsmittel eine Verbindung der obigen allgemeinen Formel 2.

In einer weiteren besonderen Ausführungsform der erfindungsgemäßen Kraftstoffzusammensetzung erfolgt die Umesterung gemäß Stufe b) der verbleibenden Estergruppe des Quaternisierungsprodukts der Stufe a), d.h. des quatenisierten Polycarbonsäureesters bzw. des quaternisierten Ammoniumsalzes eines Polycarbonsäureesters) durch Umsetzung mit wenigstens einem wie oben definierten Polyalkoxylat oder einem Alkohol-gestarteten Polyalkoxylat ;

wobei insbesondere die umgeesterte Form hydrophoberen Charakter besitzt, wie oben definiert

In einer weiteren besonderen Ausführungsform der erfindungsgemäßen Kraftstoffzusammensetzung ist das quaternisierbare tertiäre Amin eine Verbindung der Formel 3, worin wenigstens zwei der Reste $R_a$, $R_b$ und $R_c$ gleich oder verschieden sind und für einen geradkettigen oder verzweigte, $C_{10}$-$C_{20}$-Alkylrest steht und der übrigen Rest für $C_1$-$C_4$-Alkyl steht.

In einer weiteren besonderen Ausführungsform der erfindungsgemäßen Kraftstoffzusammensetzung ist das Quaternierungsmittel ausgewählt unter Di-Methyl- oder Ethylphthalaten und insbesondere Dimethyl- oder Diethyl- oxalaten.

Jeweils geeignete Testmethoden zur Überprüfung der oben bezeichneten Anwendungen sind den Fachmann bekannt, bzw. in folgenden experimentellen Teil, worauf hiermit ausdrücklich allgemein Bezug genommen wird, beschrieben.

**A2) Allgemeine Definitionen**

Werden keine gegenteiligen Angaben gemacht, so gelten folgende allgemeine Bedeutungen:

" Quaternisierbare" Stickstoffgruppen oder Aminogruppen umfassen insbesondere primäre, sekundäre und, vor allem, tertiäre Aminogruppen.

" Hydrocarbyl" ist breit auszulegen und umfasst sowohl langkettige als auch kurzkettige, gerade oder verzweigte Hydrocarbylreste (Kohlenwasserstoffreste) mit 1 bis 50 Kohlenstoffatomen, welche ggf. zusätzlich Heteroatome, wie z.B. O, N, NH, S, in ihrer Kette enthalten können. Eine spezielle Gruppe von Hydrocarbyl-Resten umfasst sowohl langkettige als auch kurzkettige, geradkettige oder verzweigte Alkylreste mit 1 bis 50 Kohlenstoffatomen

" Langkettige" oder" Hochmolekulare" Hydrocarbylreste stehen für geradkettige oder verzweigte Kohlenwasserstoffreste und weisen 5 bis 50 oder 6 bis 50 oder 7 bis 50 oder 8 bis 50 oder 8 bis 40 oder 10 bis 20 Kohlenstoffatomen, welche ggf. zusätzlich Heteroatome, wie z.B. O, N, NH, S, in ihrer Kette enthalten können. Weiterhin können die Reste ein- oder mehrfach ungesättigt sein und ein oder mehrere nichtkumulierte, wie z.B. 1 bis 5, wie 1, 2 oder 3 C-C Doppelbindungen oder C-C- Dreifachbindungen, insbesondere 1, 2 oder 3 Doppelbindungen, aufweisen. Sie können natürlichen oder synthetischen Ursprungs sein.

Sie können auch ein zahlengemitteltes Molekulargewicht ($M_n$) von 85 bis 20.000, wie z.B. 113 bis 10.000, oder 200 bis 10.000 oder 350 bis 5.000, wie z.B. 350 bis 3.000, 500 bis 2.500, 700 bis 2.500, oder 800 bis 1.500 aufweisen Sie sind dann insbesondere im Wesentlichen aus $C_{2-6}$-, insbesondere $C_{2-4}$-Monomerbausteinen, wie Ethylen, Propylen, n- oder iso-Butylen oder Mischungen davon aufgebaut, wobei die verschiedenen Monomere statistisch verteilt oder als Blöcke einpolymerisiert enthalten sein können. Derartige langkettige Hydrocarbylreste werden auch als Polyalkylenreste oder Poly-$C_{2-6}$- oder Poly-$C_{2-4}$-alkylenreste bezeichnet. Geeignete langkettige Hydrocarbylreste und deren Herstellung sind beispielsweise auch beschreiben in der WO2006/135881 und der dort zitierten Literatur.

Beispiele für besonders brauchbare Polyalkylen-Reste sind Polyisobutenyl-Reste, abgeleitet von sogenannten "hochreaktiven" Polyisobutenen, die sich durch einen hohen Gehalt an terminal angeordneten Doppelbindungen auszeichnen. Terminal angeordnete Doppelbindungen sind dabei alpha-olefinische Doppelbindungen des Typs

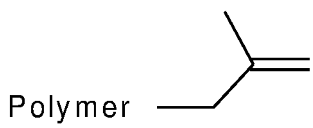

welche zusammen auch als Vinyliden-Doppelbindungen bezeichnet werden. Geeignete hochreaktive Polyisobutene sind beispielsweise Polyisobutene, die einen Anteil an Vinyliden-Doppelbindungen von größer 70 Mol-%, insbesondere größer 80 Mol-% oder größer 85 Mol-% aufweisen. Bevorzugt sind insbesondere Polyisobutene, die einheitliche Polymergerüste aufweisen. Einheitliche Polymergerüste weisen insbesondere solche Polyisobutene auf, die zu wenigstens 85 Gew.-%, vorzugsweise zu wenigstens 90 Gew.-% und besonders bevorzugt zu wenigstens 95 Gew.-% aus Isobuteneinheiten aufgebaut sind. Vorzugsweise weisen solche hochreaktiven Polyisobutene ein zahlenmittleres Molekulargewicht in dem oben genannten Bereich auf. Darüber hinaus können die hochreaktiven Polyisobutene eine Polydispersität im Bereich von 1,05 bis 7, insbesondere von etwa 1,1 bis 2,5, wie z.B. von kleiner 1,9 oder kleiner 1,5, aufweisen. Unter Polydispersität versteht man den Quotienten aus gewichtsmittlerem Molekulargewicht Mw geteilt durch das zahlenmittlere Molekulargewicht Mn.

Besonders geeignete hochreaktive Polyisobutene sind z.B. die Glissopal-Marken der BASF SE, insbesondere Glissopal 1000 (Mn = 1000), Glissopal V 33 (Mn = 550) und Glissopal 2300 (Mn = 2300) und deren Mischungen. Andere zahlenmittlere Molekulargewichte können nach im Prinzip bekannter Weise durch Mischen von Polyisobutenen unterschiedlicher zahlenmittlerer Molekulargewichte oder durch extraktive Anreicherung von Polyisobutenen bestimmter Molekulargewichtsbereiche eingestellt werden.

Eine spezielle Gruppe von langkettigen Hydrocarbyl Resten umfasst geradkettige oder verzweigte Alkylresten ("langkettige" Alkylreste") mit 8 bis 50, wie z.B. 8 bis 40 oder 8 bis 30 oder 10 bis 20 Kohlenstoffatomen.

" Kurzkettiges Hydrocarbyl" oder" niedermolekulares Hydrocarbyl" steht insbesondere für geradkettiges oder verzweigtes Alkyl oder Alkenyl, gegebenenfalls unterbrochen durch eine oder mehrere, wie z.B. 2, 3 oder 4 Heteroatomgruppen, wie -O- oder - NH-. oder gegebenenfalls ein- oder mehrfach, wie z.B. 2, 3 oder 4-fach substituiert.

" Hydrocarbylen" steht für geradkettige oder ein- oder mehrfach verzweigte Brückengruppen mit 1 bis 10 Kohlenstoffatomen, gegebenenfalls unterbrochen durch eine oder mehrere, wie z.B. 2, 3 oder 4 Heteroatomgruppen, wie -O- oder - NH-. oder gegebenenfalls ein- oder mehrfach, wie z.B. 2, 3 oder 4-fach substituiert.

" Alkyl" oder" Niedrigalkyl" steht insbesondere für gesättigte, geradkettige oder verzweigte Kohlenwasserstoffreste mit 1 bis 4, 1 bis 5, 1 bis 6, oder 1 bis 7, Kohlenstoffatomen, wie z. B. Methyl, Ethyl, n-Propyl, 1-Methylethyl, n-Butyl, 1-Methyl-propyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl; sowie n-Heptyl, sowie die ein- oder mehrfach verzweigten Analoga davon.

" Langkettiges Alkyl" steht insbesondere für gesättigte, geradkettige oder verzweigte Kohlenwasserstoffreste mit 8 bis 50, wie z.B. 8 bis 40 oder 8 bis 30 oder 10 bis 20 Kohlenstoffatomen, wie Octyl, Nonyl, Decyl, Undecyl, Dodecyl, Tridecyl, Tetradecyl, Pentadecyl, Hexadecyl, Heptadecyl, Octadecyl, Nonadecyl, Eicosyl, Heneicosyl, Docosyl, Tricosyl, Tetracosyl, Pentacosyl, Hexacosyl, Heptacosyl, Octacosyl, Nonacosyl, Squalyl, Konstitutionsisomere, insbesondere ein oder mehrfach verzweigte Isomere und höhere Homologe davon.

" Hydroxyalkyl" steht insbesondere für die ein- oder mehrfach, insbesondere einfach hydroxylierten Analoga obiger Alkylreste, wie z.B. die monohydroxylierten Analoga obiger geradkettiger oder verzweigter Alkylreste, wie z.B. die linearen Hydroxyalkylgruppen, wie z.B. solchen mit primärer (endständigen) Hydroxylgruppe, wie Hydroxymethyl, 2-Hydroxyethyl, 3-Hydroxypropyl, 4-Hydroxybutyl, oder solchen mit nichtendständigen Hydroxylgruppen, wie 1-Hydroxyethyl, 1- oder 2-Hydroxypropyl, 1- oder 2-Hydroxybutyl oder 1-, 2- oder 3-Hydroxybutyl.

" Alkenyl" steht für ein- oder mehrfach, insbesondere einfach ungesättigte, geradkettige oder verzweigte Kohlenwasserstoffreste mit 2 bis 4, 2 bis 6, oder 2 bis 7 Kohlenstoffatomen und einer Doppelbindung in einer beliebigen Position, z. B. $C_2$-$C_6$-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

" Hydroxyalkenyl" steht insbesondere für die ein- oder mehrfach, insbesondere einfach hydroxylierten Analoga obiger Alkenylreste

" Aminoalkyl" und" Aminoalkenyl" steht insbesondere für die ein- oder mehrfach, insbesondere einfach aminierten Analoga obiger Alkyl- bzw. Alkenylreste, oder Analoga obiger Hydroxyalkyl, wobei die OH-Gruppe durch eine Aminogruppe ersetzt ist.

" Alkylen" steht für geradkettige oder ein- oder mehrfach verzweigte Kohlenwasserstoff-Brückengruppen mit 1 bis 10 Kohlenstoffatomen, wie z.B. $C_1$-$C_7$-Alkylengruppen ausgewählt unter $-CH_2-$, $-(CH_2)_2-$, $-(CH_2)_3-$, $-CH_2-CH(CH_3)-$, $-CH(CH_3)-CH_2-$, $-(CH_2)_4-$, - $(CH_2)_2-CH(CH_3)-$, $-CH_2-CH(CH_3)-CH_2-$ , $(CH_2)_4-$, $-(CH_2)_5-$, $-(CH_2)_6$, $-(CH_2)_7-$, $-CH(CH_3)-CH_2-CH_2-CH(CH_3)-$ oder - $CH(CH_3)-CH_2-CH_2-CH_2-CH(CH_3)-$ oder $C_1$-$C_4$-Alkylengruppen ausgewählt unter $-CH_2-$, $-(CH_2)_2-$, $-(CH_2)_3-$, $-CH_2-CH(CH_3)-$, $-CH(CH_3)-CH_2-$, $-(CH_2)_4-$, $-(CH_2)_2-CH(CH_3)-$, $-CH_2-CH(CH_3)-CH_2-$. oder für $C_2$-$C_6$-Alkylengruppen, wie z.B. $-CH_2-CH(CH_3)-$, $-CH(CH_3)-CH_2-$, $-CH(CH_3)-CH(CH_3)-$, $-C(CH_3)_2-CH_2-$, $-CH_2-C(CH_3)_2-$, $-C(CH_3)_2-CH(CH_3)-$, $-CH(CH_3)-C(CH_3)_2-$, $-CH_2-CH(Et)-$, $-CH(CH_2CH_3)-CH_2-$, $-CH(CH_2CH_3)-CH(CH_2CH_3)-$, $-C(CH_2CH_3)_2-CH_2-$, $-CH_2-C(CH_2CH_3)_2-$, $-CH_2$-CH(n-Propyl)-, -CH(n-Propyl)-$CH_2-$, -CH(n-Propyl)-$CH(CH_3)-$, $-CH_2$-CH(n-Butyl)-, -CH(n-Butyl)-$CH_2-$, $-CH(CH_3)-CH(CH_2CH_3)-$, $-CH(CH_3)$-CH(n-Propyl)-, $-CH(CH_2CH_3)-CH(CH_3)-$, $-CH(CH_3)-CH(CH_2CH_3)-$, oder für $C_2$-$C_4$-Alkylengruppen, wie z.B. ausgewählt unter $-(CH_2)_2-$, $-CH_2-CH(CH_3)-$, $-CH(CH_3)-CH_2-$, $-CH(CH_3)-CH(CH_3)-$, $-C(CH_3)_2-CH_2-$, $-CH_2-C(CH_3)_2-$, $-CH_2-CH(CH_2CH_3)-$, $-CH(CH_2CH_3)-CH_2-$.

" Oxyalkylen-Reste entsprechen der Definition obiger geradkettiger oder ein- oder mehrfach verzweigter Alkylenreste mit 2 bis 10 Kohlenstoffatomen, wobei die Kohlenkette durch ein Sauerstoff-Heteroatom 1- oder mehrfach, insbesondere 1-fach unterbrochen ist. Als nichtlimitierende Beispiele sind zu nennen: $-CH_2-O-CH_2-$, $-(CH_2)_2-O-(CH_2)_2-$, $-(CH_2)_3-O-(CH_2)_3-$, oder $-CH_2-O-(CH_2)_2-$, $-(CH_2)_2-O-(CH_2)_3-$, $-CH_2-O-(CH_2)_3$

" Aminoalkylen" entsprechen der Definition obiger geradkettiger oder ein- oder mehrfach verzweigter Alkylenreste mit 2 bis 10 Kohlenstoffatomen, wobei die Kohlenkette durch eine Stickstoffgruppe (insbesondere - NH-Gruppe) 1- oder mehrfach, insbesondere 1-fach unterbrochen ist. Als nichtlimitierende Beispiele sind zu nennen: $-CH_2-NH-CH_2-$, $-(CH_2)_2-NH-(CH_2)_2-$, $-(CH_2)_3-NH-(CH_2)_3-$, oder $-CH_2-NH-(CH_2)_2-$, $-(CH_2)_2-NH-(CH_2)_3-$, $-CH_2-NH-(CH_2)_3$.

" Alkenylen" steht für die ein- oder mehrfach, insbesondere einfach ungesättigten Analoga obiger Alkylengruppen mit 2 bis 10 Kohlenstoffatomen, insbesondere für $C_2$-$C_7$-Alkenylene oder $C_2$-$C_4$-Alkenylen, wie -CH=CH-, -CH=CH-$CH_2-$, - $CH_2$-CH=CH-, -CH=CH-$CH_2-CH_2-$, $-CH_2$-CH=CH-$CH_2-$, $-CH_2-CH_2$-CH=CH-, $-CH(CH_3)$-CH=CH-, $-CH_2-C(CH_3)$=CH-.

"Cycloalkyl" steht für carbocyclische Reste mit 3 bis 20 Kohlenstoffatomen, wie z.B. $C_3$-$C_{12}$-Cycloalkyl, wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl, Cycloheptyl, sowie Cyclopropyl-methyl, Cyclopropyl-ethyl, Cyclobutyl-methyl, Cyclobutyl-ethyl, Cyclopentyl-methyl, Cyclopentyl-ethyl, Cyclohexyl-methyl oder $C_3$-$C_7$-Cycloalkyl, wie Cyclopropyl, Cyclobutyl, Cyclopentyl, Cyclohexyl, Cycloheptyl, Cyclopropyl-methyl, Cyclopropyl-ethyl, Cyclobutyl-methyl, Cyclopentyl-ethyl, Cyclohexyl-methyl, wobei die Anbindung an den Rest des Moleküls über jegliches geeignetes C-Atom erfolgen kann.

"Cycloalkenyl" oder ein- oder mehrfach ungesättigtes Cycloalkyl" steht insbesondere für monocyclische, ein oder mehrfach ungesättigte Kohlenwasserstoffgruppen mit 5 bis 8, vorzugsweise bis 6 Kohlenstoffringgliedern, wie z.B. die einfach ungesättigten Rests Cyclopenten-1-yl, Cyclopenten-3-yl, Cyclohexen-1-yl, Cyclohexen-3-yl und Cyclohexen-4-yl;

"Cycloalkylen" steht für zweifach gebundenen Analoga obiger carbocyclische Cycloalkyl Reste mit 3 bis 20 Kohlenstoffatomen, wie z.B. $C_3$-$C_{12}$-Cycloalkylen, wobei die Anbindung an den Rest des Moleküls über jegliches geeignetes C-Atom erfolgen kann. Beispiele sind Cyclopropylen, Cyclobutylen, Cyclopentylen, Cyclohexylen, Cycloheptylen und Cyclooctylen.

"Aryl" steht für ein- oder mehrkernige, vorzugsweise ein- oder zweikernige, gegebenenfalls substituierte aromatische Reste mit 6 bis 20 wie z.B. 6 bis 10 RingKohlenstoffatomen, wie z.B. Phenyl, Biphenyl, Naphthyl wie 1- oder 2-Naphthyl, Tetrahydronaphthyl, Fluorenyl, Indenyl und Phenathrenyl. Diese Arylreste können gegebenenfalls 1, 2, 3, 4, 5 oder 6 gleiche oder verschiedene Substituenten tragen.

"Alkylaryl" steht für die in beliebiger Ringposition ein- oder mehrfach, insbesondere 1- oder 2-fach, Alkylsubstituierten Analoga obiger Arylreste, wobei Aryl ebenfalls die oben angegebenen Bedeutungen besitzt, wie z.B. $C_1$-$C_4$-Alkyl-Phenyl-, wobei der $C_1$-$C_4$-Alkylreste in beliebiger Ringposition liegen können.

" Substituenten" für hierin angegebene Reste, sind insbesondere, wenn keine anderen Angaben gemacht werde, ausgewählt sind unter Ketogruppen, - COOH, -COO-Alkyl, - OH, -SH, -CN, Amino, $-NO_2$, Alkyl, insbesondere $C_1$-$C_6$ Alkyl-, oder Alkenyl-, insbesondere $C_2$-$C_6$-Alkenyl-Gruppen.

" Mn" steht für das zahlenmittlere Molekulargewicht und wird in herkömmlicher Weise bestimmt; insbesondere beziehen sich solche Angaben auf Mn-Werte, bestimmt durch Relativmethoden, wie der Gelpermeationschromatographie mit THF als Eluent und Polystyrol-Standards oder Absolutmethoden, wie der Dampfphasenosmometrie unter Verwendung von Toluol als Lösungsmittel.

"Mw" steht für das gewichtsmittlere Molekulargewicht und wird in herkömmlicher Weise bestimmt; insbesondere beziehen sich solche Angaben auf Mw-Werte, bestimmt durch Relativmethoden, wie der Gelpermeationschromatographie mit THF als Eluent und Polystyrol-Standards oder Absolutmethoden, wie der Lichtstreuung.

Der " Polymerisationsgrad" bezeichnet gewöhnich den zahlenmäßigen mittleren Polymerisationsgrad (Bestimmungsmethode Gelpermeationschromatographie mit THF als Eluent und Polystyrol-Standards; oder GC-MS Kopplung).

Der Verteilungskoeffizient wir je nach Wert der mittels verschiedener Methoden bestimmt. Der Verteilungskoeffizient "logPow" einer Verbindung bis zu einem Wert von etwa 6 ist bestimmbar nach OECD Richtlinie 117 bei 25 °C in n-Octanol/Wasser; nach dieser Methode besitzt n-Butanol einen Wert von 1, Pentanol einen Wert von 1,3 oder 2-Ethylhexanol einen Wert von 2,9.

Höhere Verteilungskoeffizienten (d.h. Wert >6; hier bezeichnet als "logP" ) werden nach der ACD/LogP Methode (Perauskas, A.A. et al., Perspectives in Drug Discovery and Design, 19: 99-116 (2000) bestimmt

**A3) Quatemisierbare Stickstoffverbindungen**

Quaternisierbare Stickstoffverbindungen sind insbesondere:

**A3.1) Tertiäre Amine**

Tertiäre Amine sind insbesondere Verbindungen der obigen Formel 3 und sind an sich bekannte Verbindungen, wie z.B. beschrieben in der EP-A-2 033 945.

Das tertiäre Amin-Edukt 3 trägt vorzugsweise ein Segment der Formel $NR_a$ $R_b$ wobei einer der Reste eine Alkylgruppe mit 8 bis 40 Kohlenstoffatomen und der andere eine Alkylgruppe mit bis zu 40, besonders bevorzugt 8 bis 40 Kohlenstoffatomen aufweist. Der Rest $R_c$ ist dabei insbesondere ein kurzkettiger $C_1$-$C_6$-Alkylrest, wie eine Methyl-, Ethyl- oder Propyl-Gruppe. $R_a$ und $R_b$ können geradkettig oder verzweigt sein, und / oder können gleich oder verschieden sein. Beispielsweise können $R_a$ und $R_b$ eine geradkettige $C_{12}$-$C_{24}$-Alkylgruppe sein. Alternativ kann nur einer der beiden Reste langkettig (z.B. mit mit 8 bis 40 Kohlenstoffatomen) sein und der andere eine Methyl-, Ethyl- oder Propyl-Gruppe darstellen.

Zweckmäßig ist das Segment $NR_aR_b$ von einem sekundären Amin abgeleitet, wie Dioctadecylamin, Di-Kokosamin, di-hydriertes Talgamin und Methylbehenylamin. Amingemische, wie sie aus natürlichen Materialien erhältlich sind, eignen sich ebenfalls. Beispielsweise ist zu nennen ein sekundäres hydriertes Talg-Amin, wobei die Alkylgruppen von hydriertem Talgfett abgeleitet sind, und etwa 4 Gew.-% $C_{14}$, 31 Gew.-% $C_{16}$ und 59 Gew.-% $C_{18}$-Alkylgruppen aufweisen. Entsprechende tertiäre Amine der Formel 3 werden beispielsweise von der Firma Akzo Nobel unter der Bezeichnung Armeen® M2HT oder Armeen® M2C vertrieben.

Das tertiäre Amin-Edukt 3 kann aber auch so ausgebildet sein, dass die Reste $R_a$, $R_b$ und $R_c$ gleich oder verschiedene langkettige Alkylreste insbesondere geradkettige oder verzweigte Alkylgruppen mit 8 bis 40 Kohlenstoffatomen aufweisen.

Weitere nichtlimitierende Beispiele für geeignete Amine sind:
N,N-Dimethyl-N-(2-ethylhexyl)amin, N,N-Dimethyl-N-(2-propylheptyl)amin, Dodecyldimethylamin, Hexadecyldimethylamin, Oleyldimethylamin, Cocoyldimethylamin, Dicocoylmethylamin, Talgfettdimethylamin, Ditalgfettmethylamin, Tridodecylamin, Trihexadecylamin, Trioctadecylamin, Sojadimethylamin, Tris(2-ethylhexyl)amin, sowie Alamine 336 (Tri-n-octylamin).

**A3.2) Quatemisierbare, Polyether-substituierte Amin, enthaltend wenigstens eine quatemisierbare, insbesondere tertiäre, Aminogruppe;**

Derartige Verbindungen sind z.B. beschrieben in der WO2013/064689 der Anmelderin, worauf hiermit ausdrücklich Bezug genommen wird.

Derartige substituierte Amine besitzen insbesondere wenigstens einen, insbesondere einen Polyether-Substituenten mit Monomereinheiten der allgemeinen Formel Ic

$$-[-CH(R_3)-CH(R_4)-O-]- \qquad (Ic)$$

worin
$R_3$ und $R_4$ gleich oder verschieden sind und für H, Alkyl, Alkylaryl oder Aryl stehen.

Das Polyether-substituierte Amin kann dabei ein zahlenmittleres Molekulargewicht im Bereich von 500 bis 5000, insbesondere 800 bis 3000 oder 900 bis 1500 aufweisen.

Die quaternisierbaren, Polyether-substituierten Amine sind insbesondere Stickstoffverbindung der allgemeinen Formel Ia-1 oder Ib-2

$$(R_1)(R_2)N-A-O-[-CH(R_3)-CH(R_4)-O-]_n-H \qquad (Ia-1)$$

$$R_6-O-[-CH(R_3)-CH(R_4)-O-]_{n-1}CH(R_3)-CH(R_4)-N(R_1)(R_2) \qquad (Ib-2)$$

worin

$R_1$ und $R_2$ gleich oder verschieden sind und für Alkyl, Alkenyl, Hydroxyalkyl, Hydroxyalkenyl, Aminoalkyl oder Aminoalkenyl stehen, oder $R_1$ und $R_2$ zusammen für Alkylen, Oxyalkylen oder Aminoalkylen stehen;
$R_3$ und $R_4$ gleich oder verschieden sind und für H, Alkyl, Alkylaryl oder Aryl stehen;
$R_6$ für Alkyl, Alkenyl, ggf ein oder mehrfach ungesättigtes Cycloalkyl, Aryl, jeweils gegebenenfalls substituiert, wie z.B. mit wenigstens einem Hydroxylrest oder Alkylrest, oder unterbrochen durch wenigstens ein Heteroatom, steht;
A für einen geradkettigen oder verzweigten Alkylenrest steht, der gegebenenfalls durch ein oder mehrere Heteroatome, wie N, O und S, unterbrochen ist; und
n für einen ganzzahligen Wert von 1 bis 50 steht;

Insbesondere sind solche Stickstoffverbindungen der Formeln Ia-1 und Ib-2 zu nennen, worin
$R_1$ und $R_2$ gleich oder verschieden sind und für $C_1$-$C_6$-Alkyl, Hydroxy-$C_1$-$C_6$-alkyl, Hydroxy-$C_1$-$C_6$-alkenyl, oder Amino-$C_1$-$C_6$-alkyl stehen, oder $R_1$ und $R_2$ zusammen eine $C_2$-$C_6$-Alkylen-, $C_2$-$C_6$-Oxyalkylen- oder $C_2$-$C_6$-Aminoalkylen-Rest bilden;
$R_3$ und $R_4$ gleich oder verschieden sind und für H, $C_1$-$C_6$-Alkyl oder Phenyl stehen;
$R_6$ für $C_1$-$C_{20}$-Alkyl, wie z.B. $C_{10}$-$C_{20}$-, $C_{11}$-$C_{20}$- oder $C_{12}$-$C_{20}$-Alkyl oder Aryl oder Alkylaryl, wobei Alkyl insbesondere für $C_1$-$C_{20}$- bedeutet, steht;
A für einen geradkettigen oder verzweigten $C_2$-$C_6$-Alkylenrest steht, der gegebenenfalls durch ein oder mehrere Heteroatome, wie N, O und S, unterbrochen ist; und
n für einen ganzzahligen Wert von 1 bis 30.

Weiterhin besonders zu nennen sind Umsetzungsprodukte von N,N-Dimethylethanolamin und Propylenoxid wie beschrieben in Synthesebeispiel 1 der WO 2013/064689. Diese Umsetzung kann auch unkatalysiert oder mit einem Amin (beispielsweise Imidazol) als Katalysator durchgeführt werden, wie z.B. beschrieben in M. Ionescu, Chemistry and Technology of Polyols for Polyurethanes, 2005, ISBN 978-85957-501-7.

Stickstoffverbindungen der allgemeinen Formel Ia-1, sind herstellbar wobei man ein Aminoalkanol der allgemeinen Formel II

$(R_1)(R_2)$N-A-OH (II)

worin

$R_1$, $R_2$ und A die oben angegebenen Bedeutungen besitzen,
mit einem Epoxid der allgemeinen Formel III

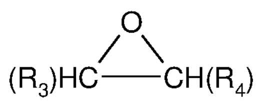

(III)

worin

$R_3$ und $R_4$ die oben angegebenen Bedeutungen besitzen,
alkoxyliert, wobei man ein alkoxyliertes Amin der Formel

$(R_1)(R_2)$N-A-O-[-CH($R_3$)-CH($R_4$)-O-]$_n$-H (Ia-1)

erhält, worin $R_1$ bis $R_4$, A und n die oben angegebenen Bedeutungen besitzen.

Stickstoffverbindungen der allgemeinen Formel Ia-2, sind herstellbar wobei man wobei man einen Alkohol der allgemeinen Formel V

$R_e$-OH (V)

worin
$R_6$ die oben angegebenen Bedeutungen besitzt, mit einem Epoxid der allgemeinen Formel III

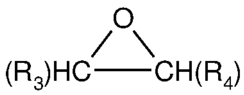

(III)

worin
$R_3$ und $R_4$ die oben angegebenen Bedeutungen besitzen, alkoxyliert, wobei man einen Polyether der Formel Ib-1 erhält;

$$R_6\text{-O-[-CH(}R_3\text{)-CH(}R_4\text{)-O-]}_{n-1}\text{CH(}R_3\text{)-CH(}R_4\text{)OH} \qquad \text{(Ib-1)}$$

erhält; worin $R_3$, $R_4$ und $R_6$, A und n die oben angegebenen Bedeutungen besitzen und
b) anschließend den so erhaltenen Polyether der Formel Ib-1 mit einem Amin der allgemeinen Formel

$$NH(R_1)(R_2) \qquad \text{(VII)}$$

worin $R_1$ und $R_2$ die oben angegebenen Bedeutungen besitzen,
aminiert, wobei man ein Amin der Formel Ib-2 erhält.

Ausgangsverbindungen zur Herstellung Polyether-substituierter, quaternisierbarer Stickstoffverbindungen sind somit:

1) Alkohole
wie z.B. der allgemeinen Formel V

$$R_6\text{-OH} \qquad \text{(V)}$$

worin $R_6$ für Alkyl, Alkenyl, ggf ein oder mehrfach ungesättigtes Cycloalkyl, Aryl, jeweils gegebenenfalls substituiert, wie z.B. mit wenigstens einem Hydroxylrest oder Alkylrest, oder unterbrochen durch wenigstens ein Heteroatom, steht; und

2) Aminoalkanole
wie z.B. der allgemeinen Formel II

$$(R_1)(R_2)\text{N-A-OH} \qquad \text{(II)}$$

worin

$R_1$ und $R_2$ gleich oder verschieden sind und für Alkyl, Alkenyl, Hydroxyalkyl, Hydroxyalkenyl, Aminoalkyl oder Aminoalkenyl stehen, oder $R_1$ und $R_2$ zusammen für Alkylen, Oxyalkylen oder Aminoalkylen stehen; und
A für einen geradkettigen oder verzweigten Alkylen- oder Alkenylenrest steht, der gegebenenfalls durch ein oder mehrere Heteroatome, wie N, O und S, unterbrochen ist;
Als weitere geeignete Gruppe von quaternisierbaren Aminoalkoholen sind zu nennen Verbindungen, ausgewählt unter Hydroxyalkyl-substituierten Mono- oder Polyaminen mit wenigstens einer quaternisierbaren, primären, sekundären oder tertiären Aminogruppe und wenigstens einer Hydroxylgruppe, welche mit einem Polyetherrest verknüpfbar ist.

Insbesondere ausgewählt sind die quaternisierbaren Stickstoff-Verbindung unter Hydroxyalkyl-substituierten primären, sekundären, und insbesondere tertiären Monoaminen und Hydroxyalkyl-substituierten primären, sekundären und insbesondere tertiären Diaminen.

Beispiele für geeignete " hydroxyalkyl-substituierte Mono- oder Polyamine" sind solche, die mit wenigstens einem, wie z.B. 1, 2, 3, 4, 5 oder 6, Hydroxyalkyl-Substituierten ausgestattet sind.

Ala Beispiele für " Hydroxyalkyl-substituierte Monoamine" können genannt werden: N-Hydroxyalkyl-monoamine, N,N-Dihydroxyalkyl-monoamine und N,N,N-Trihydroxyalkyl-monoamine, wobei die Hydroxyalkylgruppen gleich oder verschieden sind und außerdem wie oben definiert sind. Hydroxyalkyl steht dabei insbesondere für 2-Hydroxyethyl, 3-Hydroxypropyl oder 4-Hydroxybutyl.

Beispielsweise können folgende " Hydroxyalkyl-substituierte Polyamine" und insbesondere " Hydroxyalkyl-substituierte Diamine" genannt werden: (N-Hydroxyalkyl)-alkylendiamine, N,N-Dihydroxyalkyl-alkylendiamine, wobei die Hydroxyalkylgruppen gleich oder verschieden sind und außerdem wie oben definiert sind. Hydroxyalkyl steht dabei insbesondere für 2-Hydroxyethyl, 3-Hydroxypropyl oder 4-Hydroxybutyl; Alkylen steht dabei insbesondere für Ethylen,

Propylen oder Butylen.

Folgende quaternisierbare Stickstoffverbindungen seien insbesondere genannt:

| NAME | FORMEL |
|---|---|
| ***Alkohole mit primärem und sekundärem Amin*** | |
| Ethanolamin | 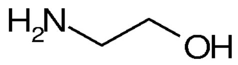 |
| 3-Hydroxy-1-propylamin | 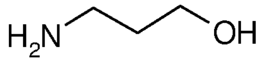 |
| Diethanolamin | 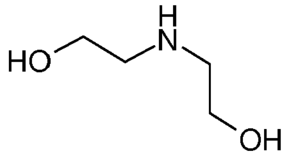 |
| Diisopropanolamin | 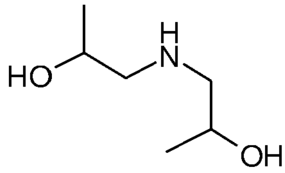 |
| N-(2-Hydroxyethyl)ethylendiamin | 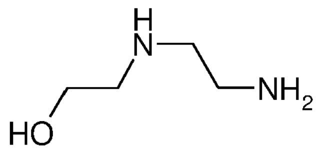 |
| ***Alkohole mit tertiärem Amin*** | |
| Triethanolamin, (2,2$^{I}$,2$^{II}$-Nitrilotriethanol) | 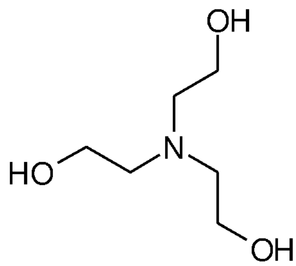 |
| 1 -(3-Hydroxypropyl)imidazol | 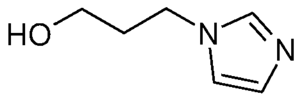 |
| Tris(hydroxymethyl)amin | 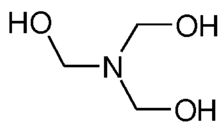 |
| 3-Dimethylamino-1-propanol | 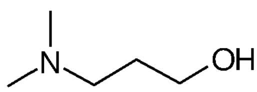 |
| 3-Diethylamino-1-propanol | 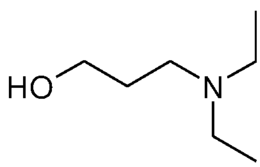 |

(fortgesetzt)

| ***Alkohole mit tertiärem Amin*** | |
|---|---|
| 2-Dimethylamino-1-ethanol | 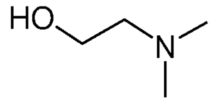 |
| 4-Diethylamino-1-butanol | 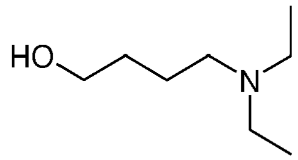 |

Zur Herstellung der Polyethersubstituierten quaternisierbaren Verbindungen (la-1 und Ib-1) kann wie folgt verfahren werden:

a1) Ausgehend von Aminoalkoholen der Formel II:

Die Aminoalkohole der allgemeinen Formel II können in prinzipiell bekannter Art und Weise alkoxyliert werden, wobei alkoxylierte Amine der allgemeinen Formel Ia-1 erhalten werden.

Die Durchführung von Alkoxylierungen ist dem Fachmann prinzipiell bekannt. Es ist dem Fachmann ebenfalls bekannt, dass man durch die Reaktionsbedingungen, insbesondere die Wahl des Katalysators, die Molekulargewichtsverteilung der Alkoxylate beeinflussen kann.

Zur Alkoxylierung werden $C_2$-$C_{16}$-Alkylenoxide eingesetzt, beispielsweise Ethylenoxid, Propylenoxid, oder Butylenoxid. Bevorzugt sind jeweils die 1,2-Alkylenoxide.

Bei der Alkoxylierung kann es sich um eine basenkatalysierte Alkoxylierung handeln. Dazu können die Aminoalkohole (II) in einem Druckreaktor mit Alkalimetallhydroxiden, bevorzugt Kaliumhydroxid oder mit Alkalialkoholaten wie beispielsweise Natriummethylat versetzt werden. Durch verminderten Druck (bspw. <100 mbar) und/oder Erhöhung der Temperatur (30 bis 150°C) kann noch in der Mischung vorhandenes Wasser abgezogen werden. Der Alkohol liegt danach als das entsprechende Alkoholat vor. Anschließend wird mit Inertgas (z.B. Stickstoff) inertisiert und das(die) Alkylenoxid(e) bei Temperaturen von 60 bis 180°C bis zu einem Druck von max. 10 bar schrittweise zugegeben. Am Ende der Reaktion kann der Katalysator durch Zugabe von Säure (z.B. Essigsäure oder Phosphorsäure) neutralisiert und kann bei Bedarf abfiltriert werden. Der basische Katalysator kann aber auch durch Zugabe handelsüblicher Mg-Silikate neutralisiert werden, welche anschließend abfiltriert werden. Optional kann die Alkoxylierung auch in Gegenwart eines Lösungsmittels durchgeführt werden. Dies kann z.B. Toluol, Xylol, Dimethylformamid oder Ethylencarbonat sein.

Die Alkoxylierung der Aminoalkohole kann aber auch mittels anderer Methoden vorgenommen werden, beispielsweise durch säurekatalysierte Alkoxylierung. Weiterhin können beispielsweise Doppelhydroxidtone wie in DE 43 25 237 A1 beschrieben eingesetzt werden, oder es können Doppelmetallcyanid-Katalysatoren (DMC-Katalysatoren) verwendet werden. Geeignete DMC-Katalysatoren sind beispielsweise in der DE 102 43 361 A1, insbesondere den Abschnitten [0029] bis [0041] sowie der darin zitierten Literatur offenbart. Beispielsweise können Katalysatoren vom Zn-Co-Typ eingesetzt werden. Zur Durchführung der Reaktion kann der Aminoalkohol mit dem Katalysator versetzt, die Mischung wie oben beschrieben entwässert und mit den Alkylenoxiden wie beschrieben umgesetzt werden. Es werden üblicherweise nicht mehr als 1000 ppm Katalysator bezüglich der Mischung eingesetzt, und der Katalysator kann aufgrund dieser geringen Menge im Produkt verbleiben. Die Katalysatormenge kann in der Regel geringer sein als 1000 ppm, beispielsweise 250 ppm und weniger.

Die Alkoxylierung kann alternativ auch durch Reaktion der Verbindungen (IV) und (V) mit cyclischen Carbonaten wie beispielsweise Ethylencarbonat vorgenommen werden.

a2) Ausgehend von Alkanolen der Formel V:

Wie unter dem vorherigen Abschnitt a1) für Aminoalkohole (II) beschrieben können analog auch Alkanole $R_6OH$ in prinzipiell bekannter Weise zu Polyethern (Ib-1) alkoxyliert werden. Die so erhaltenen Polyether können anschließend durch reduktive Aminierung mit Ammoniak, primären Aminen oder sekundären Aminen (VII) nach üblichen Methoden in kontinuierlichen oder diskontinuierlichen Verfahren unter Verwendung hierfür üblicher Hydrier- bzw. Aminierungskatalysatoren wie beispielsweise solchen, die katalytisch aktive Bestandteile auf Basis der Elemente Ni, Co, Cu, Fe, Pd, Pt, Ru, Rh, Re, Al, Si, Ti, Zr, Nb, Mg, Zn, Ag, Au, Os, Ir, Cr, Mo" W oder Kombinationen dieser Elemente untereinander

enthalten, in üblichen Mengen zu den entsprechenden Polyetheraminen (Ib-2) umgesetzt werden. Die Umsetzung kann ohne Lösungsmittel oder bei hohen Polyetherviskositäten in Gegenwart eines Lösungsmittels, vorzugsweise in Gegenwart verzweigter Aliphaten wie beispielsweise Isododekan durchgeführt werden. Die Aminkomponente (VII) wird dabei im Allgemeinen im Überschuss, z.B. im 2- bis 100-fachen Überschuss, vorzugsweise 10- bis 80-fachem Überschuss, eingesetzt. Die Reaktion wird bei Drücken von 10 bis 600 bar durchgeführt über einen Zeitraum von 10 Minuten bis 10 Stunden. Nach dem Abkühlen trennt man den Katalysator durch Filtrieren ab, verdampft überschüssige Aminkomponente (VII) und destilliert das Reaktionswasser azeotrop oder unter einem leichten Stickstoffstrom ab.

Sollte das resultierende Polyetheramin (Ib-2) primäre oder sekundäre Aminfunktionalitäten aufweisen ($R_1$ und/oder $R_2$ gleich H), kann dieses nachfolgend in ein Polyetheramin mit tertiärer Aminfunktion überführt werden ($R_1$ und $R_2$ ungleich H). Die Alkylierung kann in prinzipiell bekannter Weise durch Umsetzung mit Alkylierungsmitteln erfolgen. Geeignet sind prinzipiell alle Alkylierungsmittel wie beispielsweise Alkylhalogenide, Alkylarylhalogenide, Dialkylsulfate, Alkylenoxide ggf. in Kombination mit Säure; aliphatische oder aromatische Carbonsäureester, wie insbesondere Dialkylcarboxylate; Alkanoate; cyclische nichtaromatische oder aromatische Carbonsäureester; Dialkylcarbonate; und Mischungen davon. Die Umsetzungen zum tertiären Polyetheramin können auch durch reduktive Aminierung durch Umsetzung mit einer Carbonylverbindung wie beispielsweise Formaldehyd in Gegenwart eines Reduktionsmittels stattfinden. Geeignete Reduktionsmittels sind Ameisensäure oder Wasserstoff in Gegenwart eines geeigneten heterogenen oder homogenen Hydrierkatalysators. Die Reaktionen können ohne Lösungsmittel oder in Gegenwart von Lösungsmitteln durchgeführt werden. Geeignete Lösungsmittel sind beispielsweise $H_2O$, Alkanole wie Methanol oder Ethanol, oder 2-Ethylhexanol, aromatische Lösungsmitteln wie Toluol, Xylol oder Lösungsmittelgemischen der Solvesso-Serie, oder aliphatische Lösungsmittel, insbesondere Gemische verzweigter aliphatischer Lösungsmittel. Die Reaktionen werden bei Temperaturen von 10°C bis 300°C bei Drücken von 1 bis 600 bar über einen Zeitraum von 10 Minuten bis 10 h durchgeführt. Das Reduktionsmittel wird dabei mindestens stöchiometrisch, vorzugsweise im Überschuss eingesetzt, insbesondere im 2- bis 10-fachen Überschuss.

Das so gebildete Reaktionsprodukt (Polyetheramin Ib-1 oder Ib-2) kann theoretisch weiter aufgereinigt oder das Lösungsmittel entfernt werden. Gewöhnlich ist dies aber nicht zwingend notwendig, so dass das Reaktionsprodukt ohne weitere Aufreinigung in den nächsten Syntheseschritt, der Quaternisierung, überführt werden kann.

**A3.3) Polyalkensubstituierte Amine mit wenigstens einer tertiären, quatemisierbaren Stickstoffgruppe**

Weiterhin als quaternisierbare Sticksatoffverbindungen geeignet sind polyalkensubstituierte Amine mit wenigstens einer tertiären Stickstoffgruppe. Diese Verbindungsgruppe ist ebenfalls bekannt und z.B. beschrieben in der WO 2008/060888 oder US 2008/0113890 und dem darin genannten weiteren Stand dter Technik, worauf hiermit ausdrücklich Bezug genommen wird.

Derartige Polyalken-substituierten Amine mit mindestens einer tertiären Aminogruppe sind aus einem Olefin-Polymer und einem Amin wie Ammoniak, Monoaminen, Polyaminen oder Gemischen davon, ableitbar. Sie können durch eine Vielzahl von Verfahren hergestellt werden, wie z.B. die folgenden beispielhaft aufgeführten Verfahren:
Ein Verfahren zur Herstellung eines Polyalken-substituierten Amins umfasst die Umsetzung eines halogenierten Olefin-Polymers mit einem Amin, wie in den US-Patenten 3,275,554, 3,438,757, 3,454,555, 3,565,804, 3,755,433 und 3,822,289 beschrieben.

Ein weiteres Verfahren zur Herstellung eines Polyalken-substituierten Amins umfasst die Umsetzung eines hydroformylierten Olefins mit einem Polyamin und Hydrierung des Reaktionsprodukts, wie in US 5,567,845 und 5,496,383 beschrieben

Ein weiteres Verfahren zur Herstellung eines Polyalken-substituierten Amins umfasst die Umwandlung eines Polyalkens mit Hilfe eines herkömmlichen Epoxidierungsreagenz mit oder ohne Katalysator, in das entsprechende Epoxid und die Umsetzung des Epoxids zum Polyalken-substituierten Amin durch Umsetzung mit Ammoniak oder einem Amin unter den Bedingungen der reduktiven Animation, wie in US 5,350,429 beschrieben.

Ein weiteres Verfahren zur Herstellung von Polyalken-substituierten Amins umfasst die Hydrierung eines β-Aminonitrils, das durch Umsetzung eines Amins mit einem Nitril hergestellt wurde, wie in US 5,492,641, beschrieben.

Ein weiteres Verfahren zur Herstellung eines Polyalken-substituierten Amins umfasst Hydroformylierung eines Polybutens oder Polyisobutylens mit einem Katalysator, wie Rhodium oder Kobalt, in Gegenwart von CO und und Wasserstoff bei erhöhten Drücken und Temperaturen, wie in US 4,832,702 beschrieben.

In einer Ausführungsform der Erfindung sind die zur Herstellung verwendeten Polyalkene von Olefin-Polymeren abgeleitet. Die Olefin-Polymere können Homopolymere und Copolymere von polymerisierbaren Olefinmonomeren mit 2 bis etwa 16 Kohlenstoffatomen, 2 bis etwa 6 Kohlenstoffatomen oder 2 bis etwa 4 Kohlenstoffatomen umfassen.

Interpolymere sind solche, in denen zwei oder mehr Olefinmonomere nach bekannten herkömmlichen Methoden interpolymerisiert werden, wobei Polyalkene anfallen mit Einheiten innerhalb ihrer Struktur, die von jedem der der zwei oder mehr Olefinmonomeren abgeleitetet sind.

Somit umfassen "Interpolymere" Copolymere, Terpolymere und Tetrapolymere.

" Polyalkene" , von denen die Polyalken-substituierten Amine abgeleitet sind, werden herkömmlich häufig auch als "Polyolefine" bezeichnet.

Die Olefinmonomeren, von denen die Olefinpolymere abgeleitet sind, sind polymerisierbare Olefinmonomere, die eine oder mehrere ethylenisch ungesättigte Gruppen (d.h. >C=C<) aufweisen., das heißt, sie sind monoolefinische Monomeren, wie Ethylen, Propylen , 1-Buten, Isobuten (2-methyl-1-buten), 1-Octen oder polyolefinische Monomere (gewöhnlich diolefinische Monomere) wie 1,3-Butadien und Isopren.

Die Olefinmonomere sind gewöhnlich polymerisierbare endständige Olefine, das heißt, Olefine, die in ihrer Struktur die Gruppe > $C=CH_2$ aufweisen. Es können aber auch polymerisierbare innenständige Olefinmonomere eingesetzt werden, die durch Gruppen der Formel >C-C=C-C< gekennzeichnet sind

Spezifische Beispiele für endständige und innenständige Olefinmonomere, die verwendet werden können, um die Polyalkene nach herkömmlichen Methoden herzustellen, sind: Ethylen, Propylen, die Butene (Butylen), insbesondere 1-Buten, 2-Buten und Isobutylen, 1-Penten, 1-Hexen, 1-Hepten, 1-Octen, 1-Nonen, 1-Decen, 2-Penten, Propylen-Tetramer, Diisobutylen, Isobutylen-Trimer, 1,2-Butadien, 1,3-Butadien , 1,2-Pentadien, 1,3-Pentadien, 1,4-Pentadien, Isopren, 1,5-Hexadien, 2-Methyl-5-propyl-1-hexen, 3-Penten, 4-Octen und 3, 3-Dimethyl-1-penten.

In einer anderen Ausführungsform ist das Olefin-Polymer durch Polymerisation eines $C_4$-Raffineriestroms mit einem Butengehalt von etwa 35 bis etwa 75 Gewichtsprozent und einem Isobutengehalt von etwa 30 bis etwa 60 Gewichtsprozent in Gegenwart einer Lewis-Säure Katalysators wie Aluminiumtrichlorid oder Bortrifluorid herstellbar. Diese Polybutene enthalten üblicherweise vorwiegend (mehr als etwa 80% der gesamten Wiederholungseinheiten) Isobuten-Wiederholungseinheiten des Typs ($-CH_2-C(CH_3)_2-$).

In einer weiteren Ausführungsform ist der Polyalken-Substituent des Polyalken-substituierte Amin von einem Polyisobutylen abgeleitet.

In einer anderen Ausführungsform umfassen die Amine, die verwendet werden können, um das Polyalken-substituierte Amin zu bilden, Ammoniak, Monoaminen, Polyaminen oder Gemischen davon, einschließlich Mischungen von verschiedenen Monoamine, Mischungen verschiedener Polyamine und Mischungen von Monomaminen und Polyaminen (die Diamine). Die Amine umfassenaliphatische, aromatische, heterocyclische und carbocyclische Amine. Monoamine und Polyamine sind durch die Anwesenheit in ihrer Struktur von mindestens einer HN <-Gruppe gekennzeichnet. Die Amine können aliphatisch, cycloaliphatisch, aromatisch oder heterocyclisch sein.

Die Monoamine sind im Allgemeinen durch eine Kohlenwasserstoffgruppe mit 1 bis 50 Kohlenstoffatomen substituiert. Diese Kohlenwasserstoffgruppen können insbesondere aliphatisch und frei von acetylenisch ungesättigten Gruppen sein und 1 bis etwa 30 Kohlenstoffatome aufweisen. Insbesondere sind zu nenenn gesättigte aliphatische Kohlenwasserstoffreste mit 1 bis 30 Kohlenstoffatomen.

In einer weiteren Ausführungsform können die Monoamine die Formel $HNR_1R_2$ aufweisen, wobei $R_1$ eine Kohlenwasserstoffgruppe mit bis zu 30 Kohlenstoffatomen und $R_2$ Wasserstoff oder eine Hydrocarbylgruppe von bis zu etwa 30 Kohlenstoffatomen ist. Beispiele für geeignete Monoamine sind Methylamin, Ethylamin, Diethylamin, 2-Ethylhexylamin, Di-(2-ethylhexyl)-amin, n-Butylamin, Di-n-butylamin, Allylamin, Isobutylamin, Kokosamin, Stearylamin, Laurylamin, Methyllaurylamine und Oleylamin.

Aromatische Monoamine sind jene Monoamine, bei denen ein Kohlenstoffatom der aromatischen Ringstruktur direkt an das Aminstickstoffatom gebunden ist. Der aromatische Ring wird gewöhnlich ein einkerniger aromatischer Ring (d.h. von Benzol abgeleitet) sein, kann aber kondensierte aromatische Ringe aufweisen, und insbesondere solche von Naphthalin abgeleitet sein. Beispiele für aromatische Monoamine sind Anilin, Di (para-methylphenyl) amin, Naphthylamin, N-(n-Butyl) anilin. Beispiele für aliphatisch-substituierte, cycloaliphatisch-substituierte und heterocyclisch-substituierte aromatische Monoamine sind: para-Dodecylanilin, Cyclohexyl-substituiertes Naphthylamin und Thienyl-substituiertes Anilin.

Hydroxyamine sind ebenfalls geeigneter Monoamine. Derartige Verbindungen sind die Hydroxyhydrocarbyl-substituierten Analoga der vorstehend erwähnten Monoamine.

In einer Ausführungsform kann die Hydroxy-Monoamine der Formel $HNR_3R_4$, wobei $R_3$ eine hydroxysubstituierte Alkylgruppe mit bis zu etwa 30 Kohlenstoffatomen darstellt, und in einer Ausführungsform bis zu etwa 10 Kohlenstoffatome aufweist; und $R_4$ eine hydroxysubstituierte Alkylgruppe mit bis zu etwa 30 Kohlenstoffatomen, Wasserstoff oder eine Hydrocarbylgruppe mit bis zu etwa 10 Kohlenstoffatomen darstellt. Beispiele für Hydroxy-substituierte Monoamine umfassen: Ethanolamin, Di-3-Propanolamin, 4-Hydroxybutylamin, Diethanolamin und N-Methyl-2-hydroxypropylamin.

In einer anderen Ausführungsform kann das Amin der Polyalken-substituierten Amine ein Polyamin sein. Das Polyamin kann aliphatisch, cycloaliphatisch, heterocyclisch oder aromatisch sein. Beispiele für die Polyamine umfassen: Alkylenpolyamine, Hydroxygruppen-enthaltende Polyamine, Arylpolyamine und heterocyclische Polyamine.

Die Alkylenpolyamine umfassen solche der folgenden Formel

$$HN(R^5)\text{-}(Alkylen\text{-}N(R^5))_n\text{-}(R^5)$$

worin n im Bereich von 1 bis etwa 10 und z.B. im Bereich von 2 bis etwa 7, oder von 2 bis etwa 5 liegt, und die "Alkylen"-Gruppe 1 bis etwa 10 Kohlenstoffatome, wie z.B. 2 bis etwa 6, oder 2 bis etwa 4 Kohlenstoffatome aufweist; die Reste $R^5$ unabhängig voneinander für Wasserstoff, eine aliphatische Gruppe, eine Hydroxyl-oder Amin-substituierte aliphatische Gruppe von jeweils bis zu etwa 30 Kohlenstoffatome stehen. Typischerweise bedeutet $R^5$ H oder Niedrigalkyl (eine Alkylgruppe mit 1 bis etwa 5 Kohlenstoffatome), insbesondere H. Solche Alkylenpolyamine umfassen: Methylenpolyamine, Ethylenpolyamine, Butylenpolyamine, Propylenpolyamine, Pentylenpolyamine, Hexylenpolyamine und Heptylenpolyamine. Die höheren Homologen solcher Amine und verwandte Aminoalkyl-substituierte Piperazine sind ebenfalls eingeschlossen.

Spezifische Alkylenpolyamine zur Herstellung der Polyalken-substituierte Amine sind die folgenden: Ethylendiamin, Diethylentriamin, Triethylentetramin, Tetraethylenpentamin, Propylendiamin, 3-Dimethylaminopropylamin, Trimethylendiamin, Hexamethylendiamin, Decamethylendiamin, Octamethylendiamin, Di (Heptamethylen) triamin, Tripropylentetramin, Pentaethylenhexamin, Di (trimethylen-triamin), N-(2-Aminoethyl) piperazin und 1,4-Bis-(2-Aminoethyl) piperazin.

Ethylenpolyamine, wie die vorstehend genannten, sind besonders aus Gründen der Kosten und Effektivität geeignet. Solche Polyamine sind im Detail im Kapitel "Diamine und höhere Amine" in Encyclopedia of Chemical Technology, zweite Ausgabe, Kirk - Othemer, Band 7, Seiten 27-39, Interscience Publishers, Abteilung von John Wiley & Sons, 1965 beschrieben. Solche Verbindungen werden am bequemsten durch die Umsetzung eines Alkylenchlorids mit Ammoniak oder durch Umsetzung eines Ethylenimins mit einem ringöffnenden Reagenz wie Ammoniak, hergestellt. Diese Reaktionen führen zur Herstellung von komplexen Gemischen von Alkylenpolyaminen, einschließlich cyclischer Kondensationsprodukte wie Piperazinen.

Andere geeignete Typen von Polyamingemischen sind die durch Stripping der vorstehend beschriebenen Polyamingemische als Rückstand gebildeten und häufig als "Polyamin-Sumpf" bezeichneten Produkte. Im allgemeinen Alkylenpolyamin-Sumpfprodukte solche, die weniger als zwei, gewöhnlich weniger als 1 Gew.% Material enthalten das unter etwa 200 °C siedet. Eine typisches Beispiel für solche Ethylenpolyaminsümpfe sind mit " E-100" bezeichneten Produkte von Dow Chemical Company in Freeport, Texas. Diese Alkylenpolyamin-Sümpfe umfassen cyclische Kondensationsprodukte wie Piperazin und höhere Analoga von Diethylentriamin, Triethylenetetriamine und dergleichen.

Hydroxylgruppen-enthaltende Polyamine umfassen: Hydroxyalkylalkylenpolyamine mit einem oder mehreren Hydroxyalkyl-Substituenten an den Stickstoffatomen. Solche Polyamine können durch Umsetzen der vorstehend beschriebenen Alkylenpolyamine mit einem oder mehreren Alkylenoxiden (z. B. Ethylenoxid, Propylenoxid und Butylenoxid) hergestellt werden. Ähnliche Alkylenoxid-Alkanolamin-Reaktionsprodukte können auch beispielsweise die Produkte der Umsetzung von primären, sekundären oder tertiären Alkanolamine mit Ethylen, Propylen oder höheren Epoxiden in einem MolVerhältnis von 1:1 bis 1:2 sein. Reaktantenverhältnisse und Temperaturen zur Durchführung solcher Umsetzungen sind dem Fachmann bekannt.

In einer anderen Ausführungsform kann das Hydroxyalkyl-substituierte Alkylenpolyamin, eine Verbindung sein in der die Hydroxyalkylgruppe eine Hydroxy-Niedrigalkylgruppe ist, d.h. weniger als acht Kohlenstoffatomen aufweist. Beispiele für solche Hydroxyalkyl-substituierten Polyamine umfassen N-(2-Hydroxyethyl) ethylendiamin (auch bekannt als 2 - (2-Aminoethylamino) ethanol), N, N-Bis (2-hydroxyethyl) ethylendiamin, 1 - (2 - Hydroxyethyl) piperazin, monohydroxypropyl substituiertes Diethylentriamin, Dihydroxypropyl-substituiertes Tetraethylenpentamin und N-(3-Hydroxybutyl) tetramethylendiamin.

Arylpolyamine sind Analoga zu den oben genannten aromatischen Monoaminen. Beispiele für Arylpolyamine umfassen: N, N’-Di-n-butyl-para-phenylendiamin und Bis-(para-aminophenyl) methan.

Heterocyclische Mono-und Polyamine können, umfassen: Aziridine, Azetidine, Azolidine, Pyridine, Pyrrole, Indole, Piperidine, Imidazole, Piperazine, Isoindole, Purine, Morpholine, Thiomorpholine, N-Aminoalkylmorpholine, N-aminoalkylthiomorpholines, N-Aminoalkylpiperazine, N, N’-Diamino-Alkylpiperazine, Azepine, Azocine, Azonine, Azecine und Tetra-, Di-und Perhydroderivate von jeder der vorstehenden Verbindungen und Gemische von zwei oder mehreren dieser heterocyclischen Amine. Typische heterocyclische Amine sind gesättigte 5 - und 6-gliedrige heterocyclische Amine, die nur Stickstoff, Sauerstoff und / oder Schwefel im Heterocyclus aufweisen, insbesondere Piperidine, Piperazine, Thiomorpholine, Morpholine, Pyrrolidine und dergleichen. Piperidin, Aminoalkyl-substituierte Piperidine, Piperazin, Aminoalkyl-substituierte Piperazine, Morpholin, Aminoalkyl-substituierte Morpholine, Pyrrolidin und Aminoalkyl-substituierte Pyrrolidine sind besonders bevorzugt. Gewöhnlich sind die Aminoalkylsubstituenten an einem Stickstoffatom gebunden, das Teil des Heterocyclus ist

Spezielle Beispiele solcher heterocyclischer Amine umfassen N-Aminopropylmorpholin, N-Aminoethylpiperazin und N, N’-Diaminoethylpiperazin. Hydroxy-heterocyclische Polyamine sind auch geeignet, Beispiele umfassen: N-(2-Hydroxyethyl) cyclohexylamin, 3-Hydroxycyclopentylamin, parahydroxy-anilin und N-Hydroxyethylpiperazin.

Beispiele von Polyalken-substituierten Amine sind die folgenden: Poly (propylen) amin, Poly (buten)-amin, N, N-dimethylpolyisobutyleneamine; polybutenemorpholine N-, N-Poly (buten)-ethylendiamin, N-Poly (propylen) trimethylendiamin, N-Poly (buten), Diethylentriamin, N ’, N’-Poly (buten) Tetraethylenpentamin und N, N-Dimethyl-N’poly (propylen) -1,3-propylendiamin.

Das zahlenmittlere Molekulargewichts solcher Polyalken-substituierten Amine beträgt etwa 500 bis etwa 5000, wie z.B. 1000 bis etwa 1500 oder etwa 500 bis etwa 3000.

Jedes der oben genannten Polyalken-substituierten Amine, die sekundäre oder primäre Amine sind, können zu tertiären Aminen mit Alkylierungsmitteln alkyliert werden, welche auch als Quaternisierungsmittel bekannt sind, wie Dialkylsulfate, Alkylhalogenide, Hydrocarbyl-substituierte Carbonate; Hydrocarbylepoxiden in Kombination mit einer Säure und Mischungen davon. Wenn bestimmte Quaternisierungsmittel, wie Alkylhalogenide oder Dialkylsulfate verwendet werden, so kann es erforderlich sein, eine Base oder basische Mittel, wie Natriumcarbonat oder Natriumhydroxid, bereitzustellen, um die freie tertiäre Aminform zu ergeben. Primäre Amine erfordern zwei Äquivalenten Alkylierungsmittel und zwei Äquivalente Base, um ein tertiäres Amin zu erhalten. In einer anderen Ausführungsform kann die Alkylierung von primären Aminen häufig in vier aufeinanderfolgenden Schritten durchgeführt werden, zunächst eine Behandlung mit dem Alkylierungsmittel und zweiten Behandlung mit einer Base und dann Wiederholen der beiden Schritte. In einer anderen Ausführungsform wird die Alkylierung eines primären Amins in einem Schritt erfolgen, beispielsweise unter Verwendung von zwei Mol Alkylhalogenid in Gegenwart eines Überschusses von heterogenen Base, wie Natriumcarbonat. Das Polyamin kann in an sich bekannter Weise erschöpfend oder partiell alkyliert sein.

In einer anderen Ausführungsform kann die Alkylierung primärer Amine und sekundärer Amine zu tertiären Aminen mit Epoxiden erfolgen. Anders als mit den Alkylhalogeniden, ist bei Verwendung eines Epoxids keine Behandlung mit Base erforderlich, um das freie Amin zu erhalten. Typischerweise wird bei Alkylierung von Aminen mit Epoxiden mindestens ein Mol Epoxid für jedes Wasserstoffatom am Amin eingesetzt. Bei der Alkylierung zum tertiären Amin mit einem Epoxid sind weder zusätzliche Säure noch Base erforderlich.

Weiterhin besonders bevorzugt ist Polyisobutendimethylamin erhältlich durch Hydroformylierung von Polyisobuten (Mn 1000) und anschließende reduktive Aminierung mit Dimethylamin, siehe Example B der WO 2008/060888.

**A3.4) Reaktionsprodukte eines hydrocarbylsubstituierten Acylierungsmittels und einer Verbindung enthaltend ein Stickstoff- oder Sauerstoffatom und zusätzlich enthaltend wenigstens eine quaternisierbare Aminogruppe**

Derartige Verbindungen sind z.B. beschrieben in der WO2013/000997 der Anmelderin, worauf hiermit ausdrücklich Bezug genommen wird.

Als geeignete Hydrocarbyl-substituierte Polycarbonsäure-Verbindungen, bzw. hydrocarbylsubstituierten Acylierungsmittels sind zu nennen:

Die eingesetzten Polycarbonsäure-Verbindungen sind aliphatische zwei- oder mehrwertige (wie z.B. 3- oder 4-wertig), insbesondere von Di-, Tri- oder Tetracarbonsäuren, sowie Analoga davon, wie Anhydride oder Niedrigalkylester (teilweise oder vollständig verestert), und gegebenenfalls durch einen oder mehrere (wie z.B. 2 oder 3), insbesondere einem langkettigen Alkylrest und/oder einem hochmolekularen Hydrocarbylrest, insbesondere einem Polyalkylenrest substituiert. Beispiele sind $C_3$ - $C_{10}$ Polycarbonsäuren, wie die Dicarbonsäuren Malonsäure, Bernsteinsäure, Glutarsäure, Adipinsäure, Pimelinsäure, Suberinsäure, Azelainsäure und Sebacinsäure, und deren verzweigte Analoga; sowie die Tricarbonsäure Citronensäure; sowie Anhydride oder Niedrigalkylester davon von. Die Polycarbonsäure-Verbindungen können auch aus den entsprechenden einfach ungesättigten Säuren und Addition wenigstens eines langkettigen Alkylrests und/oder hochmolekularen Hydrocarbylrests erzeugt werden. Beispiele geeigneter einfach ungesättigter Säuren sind Fumarsäure, Maleinsäure, Itaconsäure.

Der hydrophobe " langkettige" oder " hochmolekulare" Hydrocarbylrest, welcher für die ausreichende Löslichkeit des quaternisierten Produkts im Kraftstoff sorgt, hat ein zahlengemitteltes Molekulargewicht ($M_n$) von 85 bis 20.000, wie z.B. 113 bis 10.000, oder 200 bis 10.000 oder 350 bis 5.000, wie z.B. 350 bis 3.000, 500 bis 2.500, 700 bis 2.500, oder 800 bis 1.500. Als typische hydrophobe Hydrocarbylreste sind zu nennen Polypropenyl-, Polybutenyl- und Polyisobutenylreste, z.B. mit einem zahlenmittleren Molekulargewicht $M_n$ von 3.500 bis 5.000, 350 bis 3.000, 500 bis 2.500, 700 bis 2.500 und 800 bis 1.500.

Geeignete Hydrocarbyl substituierte Verbindungen sind z.B. beschrieben in der DE 43 19 672 und der WO2008/138836.

Geeignete Hydrocarbyl-substituierte Polycarbonsäure-Verbindungen umfassen auch polymere, insbesondere dimere Formen solcher Hydrocarbyl-substituierten Polycarbonsäure-Verbindungen. Dimere Formen enthalten z.B. zwei Säureanhydridgruppen welche unabhängig voneinander im erfindungsgemäßen Herstellungsverfahren mit der quaternisierbaren Stickstoffverbindung umgesetzt werden können.

Die mit obiger Polycarbonsäure-Verbindung reaktiven, quaternisierbaren Stickstoff-Verbindungen sind ausgewählt unter

a. Hydroxyalkyl-substituierten Mono- oder Polyaminen mit wenigstens einer quarternisierten (z.B. Cholin) oder quaternisierbaren, primären, sekundären oder tertiären Aminogruppe,

b. geradkettigen oder verzweigtem, cyclischen, heterocyclischen, aromatischen oder nichtaromatischen Polyaminen

mit wenigstens einer primären oder sekundären (anhydridreaktiven) Aminogruppe und mit wenigstens einer quarternisierten oder quaternisierbaren, primären, sekundären oder tertiären Aminogruppe;
c. Piperazinen.
Insbesondere ausgewählt sind die quaternisierbaren Stickstoff-Verbindungen unter
d. Hydroxyalkyl-substituierten primären, sekundären, tertiären oder quartären Monoaminen und Hydroxyalkyl-substituierten primären, sekundären, tertiären oder quartären Diaminen.
e. geradkettigen oder verzweigten aliphatischen Diaminen mit zwei primären Aminogruppen; Di- oder Polyaminen mit wenigstens einer primären und wenigstens einer sekundären Aminogruppe; Di- oder Polyaminen mit wenigstens einer primären und wenigstens einer tertiären Aminogruppe; Di- oder Polyaminen mit wenigstens einer primären und wenigstens einer quartären Aminogruppe; aromatischen carbo-cyclischen Diaminen mit zwei primären Aminogruppen; aromatischen heterocyclischen Polyaminen mit zwei primären Aminogruppen; aromatischen oder nichtaromatischen Heterozyklen mit einer primären und einer tertiären Aminogruppe;
Beispiele für geeignete " hydroxyalkyl-substituierte Mono- oder Polyamine" sind solche die mit wenigstens einem, wie z.B. 1, 2, 3, 4, 5 oder 6, Hydroxyalkyl-Substituierten ausgestattet sind.

Als Beispiele für " Hydroxyalkyl-substituierte Monoamine" können genannt werden: N-Hydroxyalkyl-monoamine, N,N-Dihydroxyalkyl-monoamine und N,N,N-Trihydroxyalkyl-monoamine, wobei die Hydroxyalkylgruppen gleich oder verschieden sind und außerdem wie oben definiert sind. Hydroxyalkyl steht dabei insbesondere für 2-Hydroxyethyl, 3-Hydroxypropyl oder 4-Hydroxybutyl.

Beispielsweise können folgende " Hydroxyalkyl-substituierte Polyamine" und insbesondere " Hydroxyalkyl-substituierte Diamine" genannt werden: (N-Hydroxyalkyl)-alkylendiamine, N,N-Dihydroxyalkyl-alkylendiamine, wobei die Hydroxyalkylgruppen gleich oder verschieden sind und außerdem wie oben definiert sind. Hydroxyalkyl steht dabei insbesondere für 2-Hydroxyethyl, 3-Hydroxypropyl oder 4-Hydroxybutyl; Alkylen steht dabei insbesondere für Ethylen, Propylen oder Butylen.

Geeignete " Diamine" sind Alkylendiamine, sowie die N-alkylsubstituierten Analoga davon, wie N-monoalkylierten Alkylendiamine und die N,N- oder N, N'-dialkylierten Alkylendiamine. Alkylen steht insbesondere für geradkettiges oder verzweigtes $C_{1-7}$ oder $C_{1-4}$-Alkylen, wie oben definiert. Alkyl steht insbesondere für $C_{1-4}$-Alkyl gemäß obiger Definition. Beispiele sind insbesondere Ethylendiamin, 1,2-Propylendiamin, 1,3-Propylendiamin, 1,4-Butylendiamin und Isomere davon, Pentandiamin und Isomere davon, Hexandiamin und Isomere davon, Heptandiamin und Isomere davon, sowie ein- oder mehrfach, wie z.B. ein - oder zweifach $C_1$-$C_4$-alkylierte, wie z.B. methylierte, Derivate der vorher genannten Diamin-Verbindungen, wie 3-Dimethylamino-1-propylamin (DMAPA), N,N-Diethylaminopropylamin, und N,N-Dimethylaminoethylamin.

Geeignete geradkettige " Polyamine" sind beispielsweise Dialkylentriamin, Trialkylentetramin, Tetraalkylenpentamin, Pentaalkylenhexamin, sowie die N-alkylsubstituierten Analoga davon, wie N-monoalkylierten und die N,N-oder N, N' - dialkylierten Alkylenpolyamine. Alkylen steht insbesondere für geradkettiges oder verzweigtes $C_{1-7}$ oder $C_{1-4}$-Alkylen, wie oben definiert. Alkyl steht insbesondere für $C_{1-4}$-Alkyl gemäß obiger Definition.

Beispiele sind insbesondere Diethylentriamin, Triethylentetramin, Tetraethylenpentamin, Pentaethylenhexamin, Dipropylentriamin, Tripropylenetetramin, Tetrapropylenpentamin, Pentapropylenhexamin, Dibutylentriamin, Tributylentetramin, Tetrabutylenpentamin, Pentabutylenhexamin; sowie die N,N-Dialkylderivate davon, insbesondere die N,N-Di-$C_{1-4}$-alkylderivate davon. Als Beispiele können genannt werden: N,N-Dimethyldimethylentriamin, N,N-Diethyldimethylentriamin, N,N-Dipropyldimethylentriamin, N,N-Dimethyldiethylen-1,2-triamin, N,N-Diethyldiethylen-1,2-triamin, N,N-Dipropyldiethylen-1,2-triamin, N,N-Dimethyldipropylen-1,3-triamin (i.e. DMAPAPA), N,N-Diethyldipropylen-1,3-triamin, N,N-Dipropyldipropylen-1,3-triamin, N,N-Dimethyldibutylen-1,4-triamin, N,N-Diethyldibutylen-1,4-triamin, N,N-Dipropyldibutylen-1,4-triamin, N,N-Dimethyldipentylen-1,5-triamin, N,N-Diethyldipentylen-1,5-triamin, N,N-Dipropyldipentylen-1,5-triamin, N,N-Dimethyldihexylen-1,6-triamin, N,N-Diethyldihexylen-1,6-triamin und N,N-Dipropyldihexylen-1,6-triamin,

" Aromatische carbocyclischen Diamine" mit zwei primären Aminogruppen sind die di-aminosubstituierten Derivate von Benzol, Biphenyl, Naphthalin, Tetrahydronaphthalin, Fluoren, Inden und Phenanthren.

" Aromatische oder nicht-aromatische heterocyclische Polyamine" mit zwei primären Aminogruppen sind die mit zwei Aminogruppen substituierten Derivate von folgenden Heterocyclen:

- 5- oder 6-gliedriges gesättigtes oder einfach ungesättigtes Heterocyclen, enthaltend ein bis zwei Stickstoffatome und/oder ein Sauerstoff- oder Schwefelatom oder ein oder zwei Sauerstoff- und/oder Schwefelatome als Ringglieder, z. B. Tetrahydrofuran, Pyrrolidin, Isoxazolidin, Isothiazolidin, Pyrazolidin, Oxazolidin, Thiazolidin, Imidazolidin, Pyrrolin, Piperidin, Piperidinyl, 1,3- Dioxan, Tetrahydropyran, Hexahydropyridazin, Hexahydropyrimidin, Piperazin;

- 5-gliedrige aromatische Heterocyclen, enthaltend neben Kohlenstoffatomen ein, zwei oder drei Stickstoffatome oder ein oder zwei Stickstoffatome und ein Schwefel- oder Sauerstoffatom als Ringglieder, z. B. Furan, Thian, Pyrrol,

Pyrazol, Oxazol, Thiazol, Imidazol und 1,3,4-Triazol; Isoxazol, Isothiazol, Thiadiazol, Oxadiazol

- 6-gliedrige Heterocyclen enthaltend neben Kohlenstoffatomen ein oder zwei bzw. ein, zwei oder drei Stickstoffatome als Ringglieder, z. B. Pyridinyl, Pyridazin, Pyrimidin, Pyrazinyl, 1,2,4-Triazin, 1,3,5-Triazin-2-yl;

" Aromatische oder nichtaromatische Heterozyklen mit einer primären und einer tertiären Aminogruppe" sind beispielsweise die oben genannten N-Heterocyclen, welche an wenigstens einem Ring-N-Atom aminoalkyliert sind, und insbesondere eine Amino-$C_{1-4}$-alkylgruppe tragen.

" Aromatischen oder nichtaromatischen Heterozyklen mit einer tertiären Aminogruppe und einer Hydroxyalkylgruppe" sind beispielsweise die oben genannten N-Heterocyclen, welche an wenigstens einem Ring-N-Atom hydroxyalkyliert sind, und insbesondere eine Hydroxy-$C_{1-4}$-alkylgruppe tragen.

Folgende Gruppen einzelner Verbindungsklassen quaternisierbarer Stickstoffverbindungen seien insbesondere genannt:

Gruppe 1:

| NAME | FORMEL |
|---|---|
| ***Diamine mit primärem zweiten N-Atom*** | |
| Ethylendiamin | 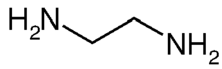 |
| 1,2-Propylendiamin | 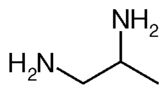 |
| 1,3-Propylendiamin | 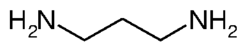 |
| Isomere Butylendiamine, wie z.B. | 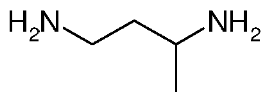 |
| 1,5-Pentylendiamin | 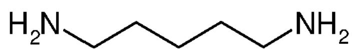 |
| Isomere Pentandiamine, wie z.B. | 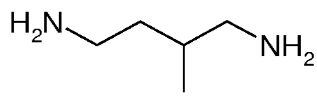 |
| Isomere Hexandiamine, wie z.B. | 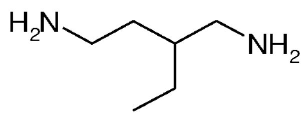 |
| Isomere Heptandiamine, wie z.B. | 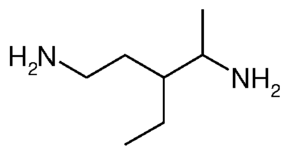 |
| ***Di- und Polyamine mit sekundärem zweiten N-Atom*** | |
| Diethylentriamin (DETA) | 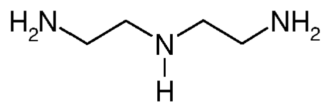 |
| Dipropylentriamin (DPTA), 3,3'-Iminobis(N,N-dimethylpropylamin) | 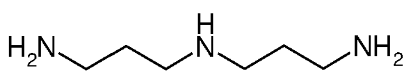 |

(fortgesetzt)

| *Di- und Polyamine mit sekundärem zweiten N-Atom* | |
|---|---|
| Triethylentetramin (TETA) | 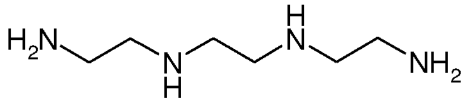 |
| Tetraethylenpentamin (TEPA) | 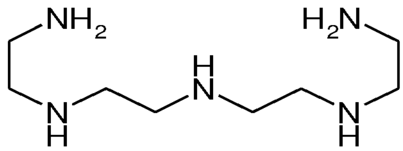 |
| Pentaethylenhexamin | 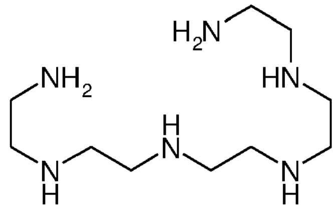 |
| N-M ethyl-3-amino-1-propylamin | 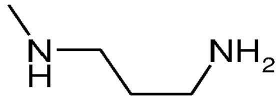 |
| Bishexamethylentriamin | 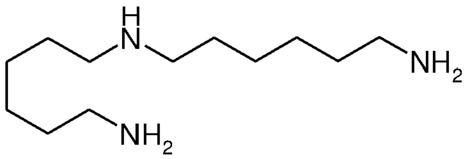 |
| ***Aromaten*** | |
| Diaminobenzole, wie z.B. | 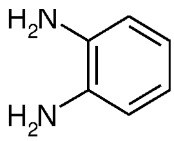 |
| Diaminopyridine, wie z.B. | 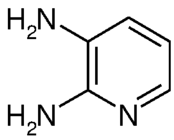 |

Gruppe 2:

| NAME | FORMEL |
|---|---|
| ***Heterozyklen*** | |
| 1-(3-Aminopropyl)imidazol | 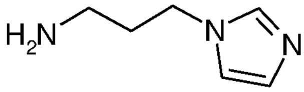 |
| 4-(3-Aminopropyl)-morpholin | 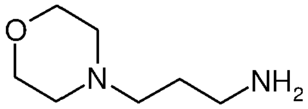 |

(fortgesetzt)

| NAME | FORMEL |
|---|---|
| ***Heterozyklen*** | |
| 1-(2-Aminoethylpiperidin) | 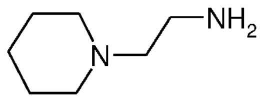 |
| 2-(1-Piperazinyl)ethylamin (AEP) | 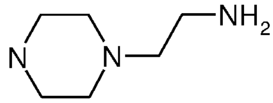 |
| N-M ethylpiperazin | 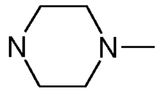 |
| ***Amine mit tertiärem zweiten N-Atom*** | |
| 3,3-Diamino-N-methyldipropylamin | 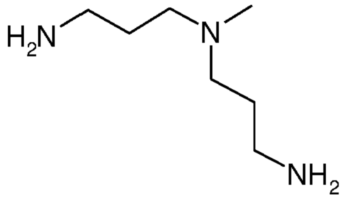 |
| 3-Dimethylamino-1-propylamin (DMAPA) | 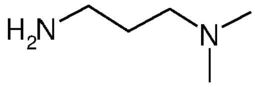 |
| N,N-Diethylaminopropylamin | 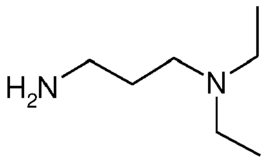 |
| N,N-Dimethylaminoethylamin | 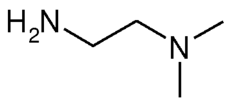 |

Gruppe 3:

| NAME | FORMEL |
|---|---|
| ***Alkohole mit primärem und sekundärem Amin*** | |
| Ethanolamin | 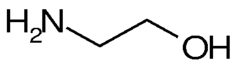 |
| 3-Hydroxy-1-propylamin | 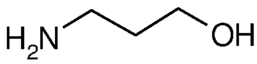 |
| Diethanolamin | 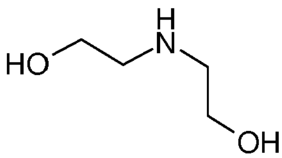 |

(fortgesetzt)

| NAME | FORMEL |
|---|---|
| ***Alkohole mit primärem und sekundärem Amin*** | |
| Diisopropanolamin | 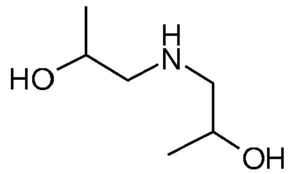 |
| N-(2-Hydroxyethyl)ethylendiamin | 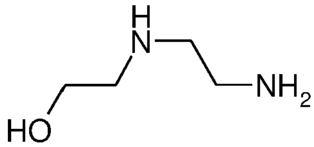 |
| ***Alkohole mit tertiärem Amin*** | |
| Triethanolamin, (2,2$^{I}$,2$^{II}$-Nitrilotriethanol) | 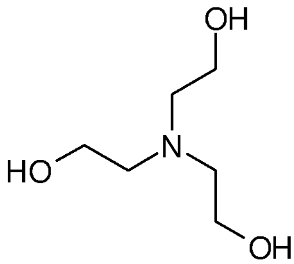 |
| 1-(3-Hydroxypropyl)imidazol | 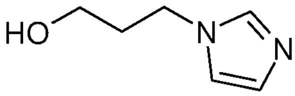 |
| Tris(hydroxymethyl)amin | 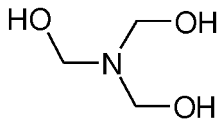 |
| 3-Dimethylamino-1-propanol | 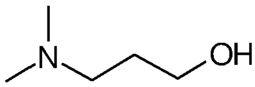 |
| 3-Diethylamino-1-propanol | 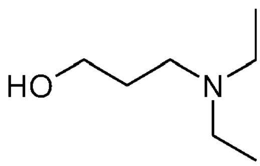 |
| 2-Dimethylamino-1-ethanol | 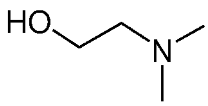 |
| 4-Diethylamino-1-butanol | 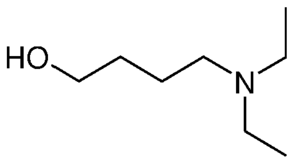 |

Die Umsetzung der Hydrocarbyl-substituierten Polycarbonsäure-Verbindung mit der quaternisierbaren Stickstoffverbindung kann unter thermisch kontrollierten Bedingungen erfolgen, so dass im Wesentlichen keine Kondensationsreaktion erfolgt. Insbesondere ist dann keine Bildung von Reaktionswasser zu beobachten. Insbesondere erfolgt eine derartige Reaktion bei einer Temperatur im Bereich von 10 bis 80, insbesondere 20 bis 60 oder 30 bis 50 °C. Die Reaktionsdauer kann dabei im Bereich von wenigen Minuten oder einigen Stunden, wie z.B. etwa 1 Minute bis zu etwa 10 Stunden liegen. Der Umsetzung kann dabei bei etwa 0,1 bis 2 atm Druck, insbesondere aber etwa bei Normaldruck erfolgen. Beispielsweise ist eine Inertgas-Atmosphäre, wie z.B. Stickstoff, zweckmäßig

Insbesondere kann die Reaktion auch unter erhöhten, eine Kondensation begünstigenden Temperaturen, z. B. im Bereich von oder 90 bis 100 °C oder 100 bis 170 °C erfolgen. Die Reaktionsdauer kann dabei im Bereich von wenigen Minuten oder einigen Stunden, wie z.B. etwa 1 Minute bis zu etwa 10 Stunden liegen. Der Umsetzung kann dabei bei etwa 0,1 bis 2 atm Druck, insbesondere aber etwa bei Normaldruck erfolgen.

Die Reaktanden werden insbesondere in etwa äquimolaren Mengen vorgelegt, gegebenenfalls ist ein geringer, z. B. 0,05 bis 0,5-facher, wie z.B. 0,1- bis 0,3-facher, molarer Überschuss der Polycarbonsäureverbindung wünschenswert. Falls erforderlich können die Reaktanden in einem geeigneten inerten organischen aliphatischen oder aromatischen Lösungsmittel oder einem Gemisch davon, vorgelegt werden. Typischen Beispiele sind z.B. Lösungsmittel der Solvesso Serie, Toluol oder Xylol. Das Lösungsmittel kann beispielsweise auch dazu dienen, Kondensationswasser azeotrop aus dem Reaktionsgemisch zu entfernen. Insbesondere werden die Reaktionen aber ohne Lösungsmittel durchgeführt.

Das so gebildete Reaktionsprodukt kann theoretisch weiter aufgereinigt oder das Lösungsmittel entfernt werden. Gewöhnlich ist dies aber nicht zwingend notwendig, so dass das Reaktionsprodukt ohne weitere Aufreinigung in den nächsten Syntheseschritt, der Quaternisierung, überführt werden kann.

Besonders zu nennen ist das Kondensationsprodukt von Polyisobutylenbernsteinsäureanhydrid (Glissopal® SA der Fa. BASF hergestellt aus Polyisobuten (Mn 1000) und Maleinsäureanhydrid in bekannter Weise) und N,N-Dimethyl-1,3-diaminopropan (CAS 109-55-7), siehe Herstellungsbeispiel 1 der WO 2013/000997.

**A4) Quatemisierungsmittel:**

Als Quaternisierungsmittel kommen im Prinzip alle als solche geeigneten Methyl- und Ethylester einer aliphatischen, cycloaromatischen oder cycloaliphatischen Polycarbonsäureester (teil- oder insbesondere vollverestert) in Betracht. Insbesondere sind zu nennen teil oder insbesondere vollveresterte aliphatische oder aromatische Polycarbonsäuremethyl- oder ethyl-ester, wie insbesondere Di-methyl- oder ethyl-carboxylate; cyclischen nichtaromatischen oder aromatischen Polycarbonsäuremethyl- oder ethyl-ester.

Beispiele sind insbesondere Verbindungen der obigen allgemeinen Formel 2

$$R_1OC(O)\text{-}A\text{-}C(O)OR_{1a} \qquad (2)$$

worin $R_1$, $R_{1a}$ und A wie oben definiert sind.

Geeignet sind z.B. Alkylester, abgeleitet von Carbonsäuren obiger Formel 2, deren $pK_s$-Wert kleiner als 3,5 ist.

Beispiele sind weiterhin insbesondere Verbindungen obiger Formel 2, und zwar vollveresterte Methyl- oder Ethyl- ester, abgeleitet von gesättigten, unsubstituierten Dicarbonsäuren, wie Oxalsäure, Malonsäure, Bernsteinsäure, Glutarsäure, Adipinsäure, Pimelinsäure und Korksäure;
gesättigten substituierten Dicarbonsäuren, wie Alkyl- oder Alkenylbernsteinsäuren, 2-Methylbutandisäure, 2-Ethylpentandisäure, 2-n-Dodecylbutandisäure, 2-n-Dodecenylbutandisäure, 2-Phenylbutandisäure und 2-(p-Methylphenyl)butandisäure ; polysubstituierte Alkyldicarbonsäuren, wie 2,2-Dimethylbutandisäure; 2,3-Dimethylbutandisäure; 2,3,4-Trimethylpentandisäure; 2,2,3-Trimethylpentandisäure und 2-Ethyl-3-methylbutandisäure;
ungesättigten Dicarbonsäuren, wie Maleinsäure, Fumarsäure, Pent-2-endisäure, Hex-2-endisäure, Hex-3-endisäure, 5-Methylhex-2-endisäure, 2,3-Dimethylpent-2-endisäure, 2-Methylbut-2-endisäure, 2-Dodecylbut-2-endisäure und 2-Polyisobutylbut-2-endisäure;
aromatischen Dicarbonsäuren, wie Phthalsäure, Isophthalsäure, Terephthalsäure und substituierte Phthalsäuren oder 3-Methylbenzol-1,2-dicarbonsäure; 4-Phenylbenzol-1,3-dicarbonsäure; 2-(1-Propenyl)benzol-1,4-dicarbonsäure und 3,4-Dimethylbenzol-1,2-dicarbonsäure; und
aromatischen oder nichtaromatischen Polycarbonsäuren, wie Citronensäure, 1,2,4-Benzoltricarboxylat, 1,2,4,5-Benzoltetracarboxylat.

Insbesondere sind zu nennen die vollveresterten Methyl- oder Ethylester, der Oxalsäure, Phthalsäure, Maleinsäure, Malonsäure und Citronensäure.

In einer besonderen Ausführungsform erfolgt die Quaternisierung des mindestens einen quaternisierbaren tertiären Stickstoffatoms jedoch mit mindestens einem Quaternisierungsmittel ausgewählt unter
Verbindungen der allgemeinen Formel 2

$$R_1OC(O)\text{-}A\text{-}C(O)OR_{1a} \qquad (2)$$

worin

$R_1$ und $R_{1a}$ unabhängig voneinander für Methyl- oder Ethyl-, steht und
A für eine chemische Bindung oder für gegebenenfalls ein- oder mehrfach substituiertes Hydrocarbylen (wie insbesondere ein gegebenenfalls ein- oder mehrfach substituiertes $C_1$-$C_7$-Alkylen oder $C_2$-$C_7$-Alkenylen) steht; wobei geeignete Substituenten z.B. ausgewählt sind unter OH, $NH_2$, $NO_2$, oder $C(O)OR_3$, insbesondere OH und $C(O)OR_3$, wobei $R_3$ für H oder insbesondere Niedrigalkyl steht.

Oben genannte Ester werden typischerweise in Gegenwart von Säuren, insbesondere in Gegenwart von freien Protonensäuren, wie vor allem mit $C_{1-12}$-Monocarbonsäuren, wie Ameisensäure, Essigsäure oder Propionsäure oder $C_{2-12}$-Dicarbonsäuren wie Oxalsäure oder Adipinsäure; oder auch in Gegenwart von Sulfonsäuren, wie Benzolsulfonsäure oder Toluolsulfonsäure oder wässrigen Mineralsäuren, wie Schwefelsäure oder Salzsäure, eingesetzt.

**A5) Alkohole und Amine zur Umesterung bzw. Amidierung**

Für den erfindungsgemäßen Derivatisierungsschritt b) (d.h. Umesterung der freien Säure/Estergruppe der quaternisierten Verbindungen) eignen sich an sich bekannte Alkohole. Durch die erfindungsgemäße Umesterung werden insbesondere kurzkettige Methyl- oder Ethyl-estergruppen in längerkettige Homologe überführt.

a) Umesterung:

Nach folgendem allgemeinem Reaktionsschema

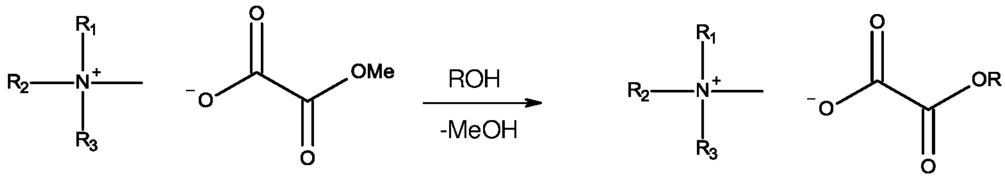


Für die Umesterung sind z.B. insbesondere folgende Alkohole der Formel ROH worin R für einen Hydrocarbyl-Rest, insbesondere langkettigen Hydrocarbyl-Rest steht, geeignet. Beispiele sind:

- Polyalkoxylate, insbesondere als Homopolymere oder Copolymere (statistisch oder block) der allgemeinen Strukturformel 1

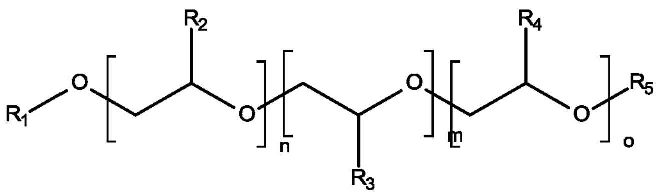

(1)

worin:

$R_1$ und $R_5$ unabhängig voneinander für H, Alkyl, Alkenyl oder Aryl stehen, wobei wenigstens einer der beiden Reste für H steht.
$R_2$, $R_3$ und $R_4$ unabhängig voneinander für H, $C_1$-$C_{30}$-Alkyl, insbesondere $C_1$-$C_5$ Alkyl wie Methyl oder Ethyl steht;
n, m, o unanbhängig voneinander für ganzzahlige Werte von 0 bis 100 stehen;
mit Molekularmassen im Bereich von 40 bis 10.000 g/mol ermittelt durch Bestimmung der OH-Zahl (DIN 53240-2), siehe auch M. Ionescu, Chemistry and Technology of Polyols for Polyurethanes, 2005, ISBN 978-1-85957-501-7, Kapitel 3. , wie nsbesondere Trägeröle wie beispielsweise beschrieben in folgendem Abschnitt B).

- Polyolgestartete Polyalkoxylate insbesondere unter Verwendung von Ethylenoxid oder Propylenoxid oder Butylenoxid (Homopolymere oder Copolymere (statistisch oder block)) mit Molekularmassen im Bereich von 100 bis

10000 g/mol ermittelt durch Bestimmung der OH-Zahl (DIN 53240-2), siehe auch M. Ionescu, Chemistry and Technology of Polyols for Polyurethanes, 2005, ISBN 978-1-85957-501-7, Kapitel 3, jeweils gemäß der Definition in Anspruch 1.

**A6) Herstellung erfindungsgemäßer Additive:**

a) Quaternisierung

Die Quaternisierung wird in an sich bekannter Weise durchgeführt

Zur Durchführung der Quaternisierung versetzt man das tertiäre Amin mit wenigstens einer Verbindung obiger Formel 2, insbesondere in den erforderlichen stöchiometrischen Mengen, um die gewünschte Quaternisierung zu erreichen. Pro Äquivalent an quaternisierbarem tertiären Stickstoffatom kann man z.B. 0,1 bis 5,0, oder 0,2 bis 3,0, oder 0,5 bis 2,5 Äquivalente, an Quaternisierungsmittel einsetzen. Insbesondere werden aber etwa 1 bis 2 Äquivalente Quaternisierungsmittel in Relation zum tertiären Amin eingesetzt, um die tertiäre Amingruppe vollständig zu quaternisieren

Man arbeitet hierbei typischerweise bei Temperaturen im Bereich von 50 bis 180°C, wie z.B. 90 bis 160 °C oder 100 bis 140 °C. Die Reaktionsdauer kann dabei im Bereich von wenigen Minuten oder einigen Stunden, wie z.B. etwa 10 Minuten bis zu etwa 24 Stunden liegen. Der Umsetzung kann dabei bei etwa 0,1 bis 20 bar, wie z.B. 1 bis 10 oder 1,5 bis 3 bar Druck, insbesondere aber etwa bei Normaldruck erfolgen.

Falls erforderlich können die Reaktanden in einem geeigneten inerten organischen aliphatischen oder aromatischen Lösungsmittel oder einem Gemisch davon, für die Quaternisierung vorgelegt werden. Typischen Beispiele sind z.B. Lösungsmittel der Solvesso Serie, Toluol oder Xylol oder Ethylhexanol. Die Quaternisierung kann aber auch in Abwesenheit eines Lösungsmittels durchgeführt werden

Zur Durchführung der Quaternisierung kann die Zugabe katalytisch wirksamer Mengen einer Säure zweckmäßig sein. Bevorzugt sind dabei aliphatische Monocarbonsäuren, wie z.B. $C_1$-$C_{18}$-Monocarbonsäuren wie insbesondere Laurinsäure, Isononansäure oder 3,5,5--Trimethylhexansäure oder Neodecansäure, aber auch aliphatische Dicarbonsäuren oder höherwertige aliphatische Carbonsäuren mit einer C-Atomzahl im oben angegebenen Bereich. Die Quaternisierung kann auch in Anwesenheit einer Lewis-Säure durchgeführt werden. Die Quaternisierung kann aber auch in Abwesenheit jeglicher Säure durchgeführt werden.

b) Umesterung

Geeignete Bedingungen sind allgemein bekannt und z.B. beschrieben in Standardlehrbüchern der organischen Chemie, wie z.B. Vollhardt, Organische Chemie, Wiley Verlag, Morrison Boyd, Lehrbuch der organischen Chemie VCh, Otera, Esterification, Wiley Verlag..

c) Aufarbeitung des Reaktionsgemisches

Das so gebildete Reaktionsendprodukt kann theoretisch weiter aufgereinigt oder das Lösungsmittel kann entfernt werden. Gegebenenfalls kann überschüssiges Reagenz, wie beispielsweise überschüssiges Epoxid, Amin und/oder Alkohol entfernt werden. Dies kann beispielsweise durch Einleiten von Stickstoff bei Normaldruck oder unter vermindertem Druck geschehen. Um die weitere Prozessierbarkeit der Produkte zu verbessern kann aber auch nach der Reaktion Lösungsmittel zugesetzt werden, wie z.B. Lösungsmittel der Solvesso Reihe, 2-Ethylhexanol, oder im Wesentlichen aliphatische Lösungsmittel. Gewöhnlich ist dies aber nicht zwingend notwendig, so dass das Reaktionsprodukt ohne weitere Aufreinigung als Additiv, gegebenenfalls nach Abmischung mit weiteren Additivkomponenten (s. unten) einsetzbar ist.

**B) Weitere Additivkomponenten**

Der mit dem erfindungsgemäßen quaternisierten Additiv additivierte Kraftstoff ist ein Ottokraftstoff oder insbesondere ein Mitteldestillat-Kraftstoff, vor allem ein Dieselkraftstoff.

Der Kraftstoff kann weitere übliche Additive zur Wirksamkeitsverbesserung und/oder Verschleißunterdrückung enthalten.

Im Falle von Dieselkraftstoffen sind dies in erster Linie übliche Detergenz-Additive, Trägeröle, Kaltfließverbesserer, Schmierfähigkeitsverbesserer (Lubricity Improver), Korrosionsinhibitoren, Demulgatoren, Dehazer, Antischaummittel, Cetanzahlverbesserer, Verbrennungsverbesserer, Antioxidantien oder Stabilisatoren, Antistatika, Metallocene, Metalldeaktivatoren, Farbstoffe und/oder Lösungsmittel.

Im Falle von Ottokraftstoffen sind dies vor allem Schmierfähigkeitsverbesserer (Friction Modifier), Korrosionsinhibitoren, Demulgatoren, Dehazer, Antischaummittel, Verbrennungsverbesserer, Antioxidantien oder Stabilisatoren, Antistatika, Metallocene, Metalldeaktivatoren, Farbstoffe und/oder Lösungsmittel.

Typische Beispiele geeigneter Co-Additive sind im folgenden Abschnitt aufgeführt:

B1) Detergenz-Additive

Vorzugsweise handelt es sich bei den üblichen Detergenz-Additiven um amphiphile Substanzen, die mindestens einen hydrophoben Kohlenwasserstoffrest mit einem zahlengemittelten Molekulargewicht ($M_n$) von 85 bis 20.000 und mindestens eine polare Gruppierung besitzen, die ausgewählt ist unter:

(Da) Mono- oder Polyaminogruppen mit bis zu 6 Stickstoffatomen, wobei mindestens ein Stickstoffatom basische Eigenschaften hat;

(Db) Nitrogruppen, gegebenenfalls in Kombination mit Hydroxylgruppen;

(Dc) Hydroxylgruppen in Kombination mit Mono- oder Polyaminogruppen, wobei mindestens ein Stickstoffatom basische Eigenschaften hat;

(Dd) Carboxylgruppen oder deren Alkalimetall- oder Erdalkalimetallsalzen;

(De) Sulfonsäuregruppen oder deren Alkalimetall- oder Erdalkalimetallsalzen;

(Df) Polyoxy-$C_2$- bis $C_4$-alkylengruppierungen, die durch Hydroxylgruppen, Mono- oder Polyaminogruppen, wobei mindestens ein Stickstoffatom basische Eigenschaften hat, oder durch Carbamatgruppen terminiert sind;

(Dg) Carbonsäureestergruppen;

(Dh) aus Bernsteinsäureanhydrid abgeleiteten Gruppierungen mit Hydroxy- und/oder Amino- und/oder Amido- und/oder Imidogruppen; und/oder

(Di) durch Mannich-Umsetzung von substituierten Phenolen mit Aldehyden und Mono- oder Polyaminen erzeugten Gruppierungen.

Der hydrophobe Kohlenwasserstoffrest in den obigen Detergenz-Additiven, welcher für die ausreichende Löslichkeit im Kraftstoff sorgt, hat ein zahlengemitteltes Molekulargewicht ($M_n$) von 85 bis 20.000, vorzugsweise von 113 bis 10.000, besonders bevorzugt von 300 bis 5.000, stärker bevorzugt von 300 bis 3.000, noch stärker bevorzugt von 500 bis 2.500 und insbesondere von 700 bis 2.500, vor allem von 800 bis 1500. Als typischer hydrophober Kohlenwasserstoffrest, insbesondere in Verbindung mit den polaren Gruppierungen, kommen insbesondere Polypropenyl-, Polybutenyl- und Polyisobutenylreste mit einem zahlenmittleren Molekulargewicht $M_n$ von vorzugsweise jeweils 300 bis 5.000, besonders bevorzugt 300 bis 3.000, stärker bevorzugt 500 bis 2.500 noch stärker bevorzugt 700 bis 2.500 und insbesondere 800 bis 1.500 in Betracht.

Als Beispiele für obige Gruppen von Detergenz-Additiven seien die folgenden genannt:

Mono- oder Polyaminogruppen (Da) enthaltende Additive sind vorzugsweise Polyalkenmono- oder Polyalkenpolyamine auf Basis von Polypropen oder von hochreaktivem (d.h. mit überwiegend endständigen Doppelbindungen) oder konventionellem (d.h. mit überwiegend mittenständigen Doppelbindungen) Polybuten oder Polyisobuten mit $M_n$ = 300 bis 5000, besonders bevorzugt 500 bis 2500 und insbesondere 700 bis 2500. Derartige Additive auf Basis von hochreaktivem Polyisobuten, welche aus dem Polyisobuten, das bis zu 20 Gew.-% n-Buten-Einheiten enthalten kann, durch Hydroformylierung und reduktive Aminierung mit Ammoniak, Monoaminen oder Polyaminen wie Dimethylaminopropylamin, Ethylendiamin, Diethylentriamin, Triethylentetramin oder Tetraethylenpentamin hergestellt werden können, sind insbesondere aus der EP-A 244 616 bekannt. Geht man bei der Herstellung der Additive von Polybuten oder Polyisobuten mit überwiegend mittenständigen Doppelbindungen (meist in der $\beta$- und $\gamma$-Position) aus, bietet sich der Herstellweg durch Chlorierung und anschließende Aminierung oder durch Oxidation der Doppelbindung mit Luft oder Ozon zur Carbonyl- oder Carboxylverbindung und anschließende Aminierung unter reduktiven (hydrierenden) Bedingungen an. Zur Aminierung können hier Amine, wie z. B. Ammoniak, Monoamine oder die oben genannten Polyamine, eingesetzt werden. Entsprechende Additive auf Basis von Polypropen sind insbesondere in der WO-A 94/24231 beschrieben.

Weitere besondere Monoaminogruppen (Da) enthaltende Additive sind die Hydrierungsprodukte der Umsetzungsprodukte aus Polyisobutenen mit einem mittleren Polymerisationsgrad P = 5 bis 100 mit Stickoxiden oder Gemischen aus Stickoxiden und Sauerstoff, wie sie insbesondere in der WO-A 97/03946 beschrieben sind.

Weitere besondere Monoaminogruppen (Da) enthaltende Additive sind die aus Polyisobutenepoxiden durch Umsetzung mit Aminen und nachfolgender Dehydratisierung und Reduktion der Aminoalkohole erhältlichen Verbindungen, wie sie insbesondere in der DE-A 196 20 262 beschrieben sind.

Nitrogruppen (Db), gegebenenfalls in Kombination mit Hydroxylgruppen, enthaltende Additive sind vorzugsweise Umsetzungsprodukte aus Polyisobutenen des mittleren Polymerisationsgrades P = 5 bis 100 oder 10 bis 100 mit Stickoxiden oder Gemischen aus Stickoxiden und Sauerstoff, wie sie insbesondere in der WO-A96/03367 und in der WO-A 96/03479 beschrieben sind. Diese Umsetzungsprodukte stellen in der Regel Mischungen aus reinen Nitropolyisobutenen (z. B. $\alpha,\beta$ -Dinitropolyisobuten) und gemischten Hydroxynitropolyisobutenen (z. B. $\alpha$-Nitro-$\beta$ -hydroxypolyisobuten) dar.

Hydroxylgruppen in Kombination mit Mono- oder Polyaminogruppen (Dc) enthaltende Additive sind insbesondere Umsetzungsprodukte von Polyisobutenepoxiden, erhältlich aus vorzugsweise überwiegend endständige Doppelbindungen aufweisendem Polyisobuten mit $M_n$ = 300 bis 5000 mit Ammoniak, Mono- oder Polyaminen, wie sie insbesondere in der EP-A 476 485 beschrieben sind.

Carboxylgruppen oder deren Alkalimetall- oder Erdalkalimetallsalze (Dd) enthaltende Additive sind vorzugsweise Copolymere von $C_2$- bis $C_{40}$-Olefinen mit Maleinsäureanhydrid mit einer Gesamt-Molmasse von 500 bis 20.000, deren Carboxylgruppen ganz oder teilweise zu den Alkalimetall- oder Erdalkalimetallsalzen und ein verbleibender Rest der Carboxylgruppen mit Alkoholen oder Aminen umgesetzt sind. Solche Additive sind insbesondere aus der EP-A 307 815 bekannt. Derartige Additive dienen hauptsächlich zur Verhinderung von Ventilsitzverschleiß und können, wie in der WO-A 87/01126 beschrieben, mit Vorteil in Kombination mit üblichen Kraftstoffdetergenzien wie Poly(iso)-butenaminen oder Polyetheraminen eingesetzt werden.

Sulfonsäuregruppen oder deren Alkalimetall- oder Erdalkalimetallsalze (De) enthaltende Additive sind vorzugsweise Alkalimetall- oder Erdalkalimetallsalze eines Sulfobernsteinsäurealkylesters, wie er insbesondere in der EP-A 639 632 beschrieben ist. Derartige Additive dienen hauptsächlich zur Verhinderung von Ventilsitzverschleiß und können mit Vorteil in Kombination mit üblichen Kraftstoffdetergenzien wie Poly(iso)buten-aminen oder Polyetheraminen eingesetzt werden.

Polyoxy-$C_2$-$C_4$-alkylengruppierungen (Df) enthaltende Additive sind vorzugsweise Polyether oder Polyetheramine, welche durch Umsetzung von $C_2$- bis $C_{60}$-Alkanolen, $C_6$-bis $C_{30}$-Alkandiolen, Mono- oder Di-$C_2$- bis $C_{30}$-alkylaminen, $C_1$- bis $C_{30}$-Alkylcyclo-hexanolen oder $C_1$- bis $C_{30}$-Alkylphenolen mit 1 bis 30 mol Ethylenoxid und/oder Propylenoxid und/oder Butylenoxid pro Hydroxylgruppe oder Aminogruppe und, im Falle der Polyetheramine, durch anschließende reduktive Aminierung mit Ammoniak, Monoaminen oder Polyaminen erhältlich sind. Derartige Produkte werden insbesondere in der EP-A 310 875, EP-A 356 725, EP-A 700 985 und US-A 4 877 416 beschrieben. Im Falle von Polyethern erfüllen solche Produkte auch Trägeröleigenschaften. Typische Beispiele hierfür sind Tridecanol- oder Isotridecanolbutoxylate, Isononylphenolbutoxylate sowie Polyisobutenolbutoxylate und -propoxylate sowie die entsprechenden Umsetzungsprodukte mit Ammoniak.

Carbonsäureestergruppen (Dg) enthaltende Additive sind vorzugsweise Ester aus Mono-, Di- oder Tricarbonsäuren mit langkettigen Alkanolen oder Polyolen, insbesondere solche mit einer Mindestviskosität von 2 $mm^2/s$ bei 100 °C, wie sie insbesondere in der DE-A 38 38 918 beschrieben sind. Als Mono-, Di- oder Tricarbonsäuren können aliphatische oder aromatische Säuren eingesetzt werden, als Esteralkohole bzw. -polyole eignen sich vor allem langkettige Vertreter mit beispielsweise 6 bis 24 C-Atomen. Typische Vertreter der Ester sind Adipate, Phthalate, iso-Phthalate, Terephthalate und Trimellitate des iso-Octanols, iso-Nonanols, iso-Decanols und des iso-Tridecanols. Derartige Produkte erfüllen auch Trägeröleigenschaften.

Aus Bernsteinsäureanhydrid abgeleitete Gruppierungen mit Hydroxy- und/oder Amino- und/oder Amido- und/oder insbesondere Imidogruppen (Dh) enthaltende Additive sind vorzugsweise entsprechende Derivate von Alkyl- oder Alkenyl-substituiertem Bernsteinsäureanhydrid und insbesondere die entsprechenden Derivate von Polyisobutenylbernsteinsäureanhydrid, welche durch Umsetzung von konventionellem oder hochreaktivem Polyisobuten mit $M_n$ = vorzugsweise 300 bis 5000, besonders bevorzugt 300 bis 3000, stärker bevorzugt 500 bis 2500, noch stärker bevorzugt 700 bis 2500 und insbesondere 800 bis 1500, mit Maleinsäureanhydrid auf thermischem Weg in einer En-Reaktion oder über das chlorierte Polyisobuten erhältlich sind. Bei den Gruppierungen mit Hydroxy- und/oder Amino- und/oder Amido- und/oder Imidogruppen handelt es sich beispielsweise um Carbonsäuregruppen, Säureamide von Monoaminen, Säureamide von Di- oder Polyaminen, die neben der Amidfunktion noch freie Amingruppen aufweisen, Bernsteinsäurederivate mit einer Säure- und einer Amidfunktion, Carbonsäureimide mit Monoaminen, Carbonsäureimide mit Di- oder Polyaminen, die neben der Imidfunktion noch freie Amingruppen aufweisen, oder Diimide, die durch die Umsetzung von Di- oder Polyaminen mit zwei Bernsteinsäurederivaten gebildet werden. Beim Vorliegen von Imidogruppierungen D(h) wird das weitere Detergenz-Additiv im Sinne der vorliegenden Erfindung jedoch nur bis maximal 100 % der Gewichtsmenge an Verbindungen mit Betainstruktur eingesetzt. Derartige Kraftstoffadditive sind allgemein bekannt und beispielsweise in den Dokumenten (1) und (2) beschrieben. Bevorzugt handelt es sich um die Umsetzungsprodukte von Alkyl- oder Alkenyl-substituierten Bernsteinsäuren oder Derivaten davon mit Aminen und besonders bevorzugt um die Umsetzungsprodukte von Polyisobutenyl-substituierten Bernsteinsäuren oder Derivaten davon mit Aminen. Von besonderem Interesse sind hierbei Umsetzungsprodukte mit aliphatischen Polyaminen (Polyalkylenimine) wie insbesondere Ethylendiamin, Diethylentriamin, Triethylentetramin, Tetraethylenpentamin, Pentaethylenhexamin und Hexaethylenheptamin, welche eine Imidstruktur aufweisen.

Durch Mannich-Umsetzung von substituierten Phenolen mit Aldehyden und Mono- oder Polyaminen erzeugte Gruppierungen (Di) enthaltende Additive sind vorzugsweise Umsetzungsprodukte von Polyisobuten-substituierten Phenolen mit Formaldehyd und Mono- oder Polyaminen wie Ethylendiamin, Diethylentriamin, Triethylentetramin, Tetraethylenpentamin oder Dimethylaminopropylamin. Die Polyisobutenyl-substituierten Phenole können aus konventionellem oder hochreaktivem Polyisobuten mit $M_n$ = 300 bis 5000 stammen. Derartige "Polyisobuten-Mannichbasen" sind insbesondere in der EP-A 831 141 beschrieben.

Dem Kraftstoff können ein oder mehrere der genannten Detergenz-Additive in solch einer Menge zugegeben werden, dass die Dosierrate an diesen Detergenz-Additiven vozugsweise 25 bis 2500 Gew.-ppm, insbesondere 75 bis 1500 Gew.-ppm, vor allem 150 bis 1000 Gew.-ppm, beträgt.

B2) Trägeröle

Mitverwendete Trägeröle können mineralischer oder synthetischer Natur sein. Geeignete mineralische Trägeröle sind bei der Erdölverarbeitung anfallende Fraktionen, wie Brightstock oder Grundöle mit Viskositäten wie beispielsweise aus der Klasse SN 500 bis 2000, aber auch aromatische Kohlenwasserstoffe, paraffinische Kohlenwasserstoffe und Alkoxyalkanole. Brauchbar ist ebenfalls eine als "hydrocrack oil" bekannte und bei der Raffination von Mineralöl anfallende Fraktion (Vakuumdestillatschnitt mit einem Siedebereich von etwa 360 bis 500 °C, erhältlich aus unter Hochdruck katalytisch hydriertem und isomerisiertem sowie entparaffiniertem natürlichen Mineralöl). Ebenfalls geeignet sind Mischungen oben genannter mineralischer Trägeröle.

Beispiele für geeignete synthetische Trägeröle sind Polyolefine (Polyalphaolefine oder Polyinternalolefine), (Poly)ester, (Poly)alkoxylate, Polyether, aliphatische Polyetheramine, alkylphenolgestartete Polyether, alkylphenolgestartete Polyetheramine und Carbonsäureester langkettiger Alkanole.

Beispiele für geeignete Polyolefine sind Olefinpolymerisate mit $M_n$ = 400 bis 1800, vor allem auf Polybuten- oder Polyisobuten-Basis (hydriert oder nicht hydriert).

Beispiele für geeignete Polyether oder Polyetheramine sind vorzugsweise Polyoxy-$C_2$-bis $C_4$-alkylengruppierungen enthaltende Verbindungen, welche durch Umsetzung von $C_2$- bis $C_{60}$-Alkanolen, $C_6$- bis $C_{30}$-Alkandiolen, Mono- oder Di-$C_2$- bis $C_{30}$-alkylaminen, $C_1$- bis $C_{30}$-Alkyl-cyclohexanolen oder $C_1$- bis $C_{30}$-Alkylphenolen mit 1 bis 30 mol Ethylenoxid und/ oder Propylenoxid und/oder Butylenoxid pro Hydroxylgruppe oder Aminogruppe und, im Falle der Polyetheramine, durch anschließende reduktive Aminierung mit Ammoniak, Monoaminen oder Polyaminen erhältlich sind. Derartige Produkte werden insbesondere in der EP-A 310 875, EP-A 356 725, EP-A 700 985 und der US-A 4,877,416 beschrieben. Beispielsweise können als Polyetheramine Poly-$C_2$- bis $C_6$-Alkylenoxidamine oder funktionelle Derivate davon verwendet werden. Typische Beispiele hierfür sind Tridecanol- oder Isotridecanolbutoxylate, Isononylphenolbutoxylate sowie Polyisobutenolbutoxylate und -propoxylate sowie die entsprechenden Umsetzungsprodukte mit Ammoniak.

Beispiele für Carbonsäureester langkettiger Alkanole sind insbesondere Ester aus Mono-, Di- oder Tricarbonsäuren mit langkettigen Alkanolen oder Polyolen, wie sie insbesondere in der DE-A 38 38 918 beschrieben sind. Als Mono-, Di- oder Tricarbonsäuren können aliphatische oder aromatische Säuren eingesetzt werden, als Esteralkohole bzw. -polyole eignen sich vor allem langkettige Vertreter mit beispielsweise 6 bis 24 Kohlenstoffatomen. Typische Vertreter der Ester sind Adipate, Phthalate, iso-Phthalate, Terephthalate und Trimellitate des Isooctanols, Isononanols, Isodecanols und des Iso-tridecanols, z. B. Di-(n- oder Isotridecyl)phthalat.

Weitere geeignete Trägerölsysteme sind beispielsweise in der DE-A 38 26 608, DE-A 41 42 241, DE-A 43 09 074, EP-A 452 328 und der EP-A 548 617 beschrieben.

Beispiele für besonders geeignete synthetische Trägeröle sind alkoholgestartete Polyether mit etwa 5 bis 35, vorzugsweise etwa 5 bis 30, besonders bevorzugt 10 bis 30 und insbesondere 15 bis 30 $C_3$- bis $C_6$-Alkylenoxideinheiten, z. B. Propylenoxid-, n-Butylenoxid- und Isobutylenoxid-Einheiten oder Gemischen davon, pro Alkoholmolekül. Nichtlimitierende Beispiele für geeignete Starteralkohole sind langkettige Alkanole oder mit langkettigem Alkyl-substituierte Phenole, wobei der langkettige Alkylrest insbesondere für einen geradkettigen oder verzweigten $C_6$- bis $C_{18}$-Alkylrest steht. Als besondere Beispiele sind zu nennen Tridecanol und Nonylphenol. Besonders bevorzugte alkoholgestartete Polyether sind die Umsetzungsprodukte (Polyveretherungsprodukte) von einwertigen aliphatischen $C_6$- bis $C_{18}$-Alkoholen mit $C_3$- bis $C_6$-Alkylenoxiden. Beispiele für einwertige aliphatische $C_6$-$C_{18}$-Alkohole sind Hexanol, Heptanol, Octanol, 2-Ethylhexanol, Nonylalkohol, Decanol, 3-Propylheptanol, Undecanol, Dodecanol, Tridecanol, Tetradecanol, Pentadecanol, Hexadecanol, Octadecanol und deren Konstitutions- und Stellungsisomere. Die Alkohole können sowohl in Form der reinen Isomere als auch in Form technischer Gemische eingesetzt werden. Ein besonders bevorzugter Alkohol ist Tridecanol. Beispiele für $C_3$- bis $C_6$-Alkylenoxide sind Propylenoxid, wie 1,2-Propylenoxid, Butylenoxid, wie 1,2-Butylenoxid, 2,3-Butylenoxid, Isobutylenoxid oder Tetrahydrofuran, Pentylenoxid und Hexylenoxid. Besonders bevorzugt

sind hierunter $C_3$- bis $C_4$-Alkylenoxide, d.h. Propylenoxid wie 1,2-Propylenoxid und Butylenoxid wie 1,2-Butylenoxid, 2,3-Butylenoxid und Isobutylenoxid. Speziell verwendet man Butylenoxid.

Weitere geeignete synthetische Trägeröle sind alkoxylierte Alkylphenole, wie sie in der DE-A 10 102 913 beschrieben sind.

Besondere Trägeröle sind synthetische Trägeröle, wobei die zuvor beschriebenen alkoholgestarteten Polyether besonders bevorzugt sind.

Das Trägeröl bzw. das Gemisch verschiedener Trägeröle wird dem Kraftstoff in einer Menge von vorzugsweise 1 bis 1000 Gew.-ppm, besonders bevorzugt von 10 bis 500 Gew.-ppm und insbesondere von 20 bis 100 Gew.-ppm zugesetzt.

B3) Kaltfließverbesserer

Geeignete Kaltfließverbesserer sind im Prinzip alle organischen Verbindungen, welche in der Lage sind, das Fließverhalten von Mitteldestillat-Kraftstoffen bzw. Dieselkraftstoffen in der Kälte zu verbessern. Zweckmäßigerweise müssen sie eine ausreichende Öllöslichkeit aufweisen. Insbesondere kommen hierfür die üblicherweise bei Mitteldestillaten aus fossilem Ursprung, also bei üblichen mineralischen Dieselkraftstoffen, eingesetzten Kaltfließverbesserer (" middle distillate flow improvers" , " MDFI" ) in Betracht. Jedoch können auch organische Verbindungen verwendet werden, die beim Einsatz in üblichen Dieselkraftstoffen zum Teil oder überwiegend die Eigenschaften eines Wax Anti-Settling Additivs (" WASA") aufweisen. Auch können sie zum Teil oder überwiegend als Nukleatoren wirken. Es können aber auch Mischungen aus als MDFI wirksamen und/oder als WASA wirksamen und/oder als Nukleatoren wirksamen organischen Verbindungen eingesetzt werden.

Typischerweise wird der Kaltfließverbesserer ausgewählt aus:

(K1) Copolymeren eines $C_2$- bis $C_{40}$-Olefins mit wenigstens einem weiteren ethyle-nisch ungesättigten Monomer;
(K2) Kammpolymeren;
(K3) Polyoxyalkylenen;
(K4) polaren Stickstoffverbindungen;
(K5) Sulfocarbonsäuren oder Sulfonsäuren oder deren Derivaten; und
(K6) Poly(meth)acrylsäureestern.

Es können sowohl Mischungen verschiedener Vertreter aus einer der jeweiligen Klassen (K1) bis (K6) als auch Mischungen von Vertretern aus verschiedenen Klassen (K1) bis (K6) eingesetzt werden.

Geeignete $C_2$- bis $C_{40}$-Olefin-Monomere für die Copolymeren der Klasse (K1) sind beispielsweise solche mit 2 bis 20, insbesondere 2 bis10 Kohlenstoffatomen sowie mit 1 bis 3, vorzugsweise mit 1 oder 2, insbesondere mit einer Kohlenstoff-Kohlenstoff-Doppelbindung. Im zuletzt genannten Fall kann die Kohlenstoff-Kohlenstoff-Doppelbindung sowohl terminal ($\alpha$ -Olefine) als auch intern angeordnet sein kann. Bevorzugt sind jedoch $\alpha$ -Olefine, besonders bevorzugt $\alpha$ -Olefine mit 2 bis 6 Kohlenstoffatomen, beispielsweise Propen, 1-Buten, 1-Penten, 1-Hexen und vor allem Ethylen.

Bei den Copolymeren der Klasse (K1) ist das wenigstens eine weitere ethylenisch ungesättigte Monomer vorzugsweise ausgewählt unter Carbonsäurealkenylestern, (Meth)Acrylsäureestern und weiteren Olefinen.

Werden weitere Olefine mit einpolymerisiert, sind dies vorzugsweise höhermolekulare als das oben genannte $C_2$- bis $C_{40}$-Olefin-Basismonomere. Setzt man beispielsweise als Olefin-Basismonomer Ethylen oder Propen ein, eignen sich als weitere Olefine insbesondere $C_{10}$- bis $C_{40}$-$\alpha$ -Olefine. Weitere Olefine werden in den meisten Fällen nur dann mit einpolymerisiert, wenn auch Monomere mit Carbonsäureester-Funktionen eingesetzt werden.

Geeignete (Meth)Acrylsäureester sind beispielsweise Ester der (Meth)Acrylsäure mit $C_1$- bis $C_{20}$-Alkanolen, insbesondere $C_1$- bis $C_{10}$-Alkanolen, vor allem mit Methanol, Ethanol, Propanol, Isopropanol, n-Butanol, sec.-Butanol, Isobutanol, tert.-Butanol, Pentanol, Hexanol, Heptanol, Octanol, 2-Ethylhexanol, Nonanol und Decanol sowie Strukturisomeren hiervon.

Geeignete Carbonsäurealkenylester sind beispielsweise $C_2$- bis $C_{14}$-Alkenylester, z.B. die Vinyl- und Propenylester, von Carbonsäuren mit 2 bis 21 Kohlenstoffatomen, deren Kohlenwasserstoffrest linear oder verzweigt sein kann. Bevorzugt sind hierunter die Vinylester. Unter den Carbonsäuren mit verzweigtem Kohlenwasserstoffrest sind solche bevorzugt, deren Verzweigung sich in der $\alpha$-Position zur Carboxylgruppe befindet, wobei das $\alpha$-Kohlenstoffatom besonders bevorzugt tertiär ist, d. h. die Carbonsäure eine sogenannte Neocarbonsäure ist. Vorzugsweise ist der Kohlenwasserstoffrest der Carbonsäure jedoch linear.

Beispiele für geeignete Carbonsäurealkenylester sind Vinylacetat, Vinylpropionat, Vinylbutyrat, Vinyl-2-ethylhexanoat, Neopentansäurevinylester, Hexansäurevinylester, Neononansäurevinylester, Neodecansäurevinylester und die entsprechenden Propenyl-ester, wobei die Vinylester bevorzugt sind. Ein besonders bevorzugter Carbonsäurealkenylester ist Vinylacetat; typische hieraus resultierende Copolymere der Gruppe (K1) sind die mit am häufigsten eingesetzten Ethylen-Vinylacetat-Copolymere (" EVA").

Besonders vorteilhaft einsetzbare Ethylen-Vinylacetat-Copolymere und ihre Herstellung sind in der WO 99/29748 beschrieben.

Als Copolymere der Klasse (K1) sind auch solche geeignet, die zwei oder mehrere voneinander verschiedene Carbonsäurealkenylester einpolymerisiert enthalten, wobei diese sich in der Alkenylfunktion und/oder in der Carbonsäuregruppe unterscheiden. Ebenfalls geeignet sind Copolymere, die neben dem/den Carbonsäurealkenylester(n) wenigstens ein Olefin und/oder wenigstens ein (Meth)Acrylsäureester einpolymerisiert enthalten.

Auch Terpolymere aus einem $C_2$- bis $C_{40}$-$\alpha$ -Olefin, einem $C_1$- bis $C_{20}$-Alkylester einer ethylenisch ungesättigten Monocarbonsäure mit 3 bis 15 Kohlenstoffatomen und einem $C_2$- bis $C_{14}$-Alkenylester einer gesättigten Monocarbonsäure mit 2 bis 21 Kohlenstoffatomen sind als Copolymere der Klasse (K1) geeignet. Derartige Terpolymere sind in der WO 2005/054314 beschrieben. Ein typisches derartiges Terpolymer ist aus Ethylen, Acrylsäure-2-ethylhexylester und Vinylacetat aufgebaut.

Das wenigstens eine oder die weiteren ethylenisch ungesättigten Monomeren sind in den Copolymeren der Klasse (K1) in einer Menge von vorzugsweise 1 bis 50 Gew.-%, insbesondere von 10 bis 45 Gew.-% und vor allem von 20 bis 40 Gew.-%, bezogen auf das Gesamtcopolymer, einpolymerisiert. Der gewichtsmäßige Hauptanteil der Monomereinheiten in den Copolymeren der Klasse (K1) stammt somit in der Regel aus den $C_2$- bis $C_{40}$-Basis-Olefinen.

Die Copolymere der Klasse (K1) weisen vorzugsweise ein zahlenmittleres Molekulargewicht $M_n$ von 1000 bis 20.000, besonders bevorzugt von 1000 bis 10.000 und insbesondere von 1000 bis 8000 auf.

Typische Kammpolymere der Komponente (K2) sind beispielsweise durch die Copolymerisation von Maleinsäureanhydrid oder Fumarsäure mit einem anderen ethylenisch ungesättigten Monomer, beispielsweise mit einem $\alpha$ -Olefin oder einem ungesättigten Ester wie Vinylacetat, und anschließende Veresterung der Anhydrid- bzw. Säurefunktion mit einem Alkohol mit wenigstens 10 Kohlenstoffatomen erhältlich. Weitere geeignete Kammpolymere sind Copolymere von $\alpha$ -Olefinen und veresterten Comonomeren, beispielsweise veresterte Copolymere von Styrol und Maleinsäureanhydrid oder veresterte Copolymere von Styrol und Fumarsäure. Geeignete Kammpolymere können auch Polyfumarate oder Polymaleinate sein. Außerdem sind Homo- und Copolymere von Vinylethern geeignete Kammpolymere. Als Komponente der Klasse (K2) geeignete Kammpolymere sind beispielsweise auch solche, die in der WO 2004/035715 und in " Comb-Like Polymers. Structure and Properties" , N. A. Plate und V. P. Shibaev, J. Poly. Sci. Macromolecular Revs. 8, Seiten 117 bis 253 (1974)" beschrieben sind. Auch Gemische von Kammpolymeren sind geeignet.

Als Komponente der Klasse (K3) geeignete Polyoxyalkylene sind beispielsweise Polyoxyalkylenester, Polyoxyalkylenether, gemischte Polyoxyalkylenesterether und Gemische davon. Bevorzugt enthalten diese Polyoxyalkylenverbindungen wenigstens eine, vorzugsweise wenigstens zwei lineare Alkylgruppen mit jeweils 10 bis 30 Kohlenstoffatomen und eine Polyoxyalkylengruppe mit einem zahlenmittleren Molekulargewicht von bis zu 5000. Derartige Polyoxyalkylenverbindungen sind beispielsweise in der EP-A 061 895 sowie in der US 4 491 455 beschrieben. Besondere Polyoxyalkylenverbindungen basieren auf Polyethylenglykolen und Polypropylenglykolen mit einem zahlenmittleren Molekulargewicht von 100 bis 5000. Weiterhin sind Polyoxyalkylenmono- und -diester von Fettsäuren mit 10 bis 30 Kohlenstoffatomen wie Stearinsäure oder Behensäure geeignet.

Als Komponente der Klasse (K4) geeignete polare Stickstoffverbindungen können sowohl ionischer als auch nicht ionischer Natur sein und besitzen vorzugsweise wenigstens einen, insbesondere wenigstens zwei Substituenten in Form eines tertiären Stickstoffatoms der allgemeinen Formel $>NR^7$, worin $R^7$ für einen $C_8$- bis $C_{40}$-Kohlenwasserstoffrest steht. Die Stickstoffsubstituenten können auch quaternisiert, das heißt in kationischer Form, vorliegen. Beispiele für solche Stickstoffverbindungen sind Ammoniumsalze und/oder Amide, die durch die Umsetzung wenigstens eines mit wenigstens einem Kohlenwasserstoffrest substituierten Amins mit einer Carbonsäure mit 1 bis 4 Carboxylgruppen bzw. mit einem geeigneten Derivat davon erhältlich sind. Vorzugsweise enthalten die Amine wenigstens einen linearen $C_8$- bis $C_{40}$-Alkylrest. Zur Herstellung der genannten polaren Stickstoffverbindungen geeignete primäre Amine sind beispielsweise Octylamin, Nonylamin, Decylamin, Undecylamin, Dodecylamin, Tetradecylamin und die höheren linearen Homologen, hierzu geeignete sekundäre Amine sind beispielsweise Dioctadecylamin und Methylbehenylamin. Geeignet sind hierzu auch Amingemische, insbesondere großtechnisch zugängliche Amingemische wie Fettamine oder hydrierte Tallamine, wie sie beispielsweise in Ullmanns Encyclopedia of Industrial Chemistry, 6. Auflage, im Kapitel " Amines, aliphatic" beschrieben werden. Für die Umsetzung geeignete Säuren sind beispielsweise Cyclohexan-1,2-dicarbonsäure, Cyclohexen-1,2-dicarbonsäure, Cyclopentan-1,2-dicarbonsäure, Naphthalindicarbonsäure, Phthalsäure, Isophthalsäure, Terephthalsäure und mit langkettigen Kohlenwasserstoffresten substituierte Bernsteinsäuren.

Insbesondere ist die Komponente der Klasse (K4) ein öllösliches Umsetzungsprodukt aus mindestens eine tertiäre Aminogruppe aufweisenden Poly($C_2$- bis $C_{20}$-Carbon-säuren) mit primären oder sekundären Aminen. Die diesem Umsetzungsprodukt zugrundeliegenden mindestens eine tertiäre Aminogruppe aufweisenden Poly($C_2$- bis $C_{20}$-Carbonsäuren) enthalten vorzugsweise mindestens 3 Carboxylgruppen, insbesondere 3 bis 12, vor allem 3 bis 5 Carboxylgruppen. Die Carbonsäure-Einheiten in den Polycarbonsäuren weisen vorzugsweise 2 bis 10 Kohlenstoffatome auf, insbesondere sind es Essigsäure-Einheiten. Die Carbonsäure-Einheiten sind in geeigneter Weise zu den Polycarbonsäuren verknüpft, meist über ein oder mehrere Kohlenstoff- und/oder Stickstoffatome. Vorzugsweise sind sie an tertiäre Stickstoffatome angebunden, die im Falle mehrerer Stickstoffatome über Kohlenwasserstoffketten verbunden sind.

Vorzugsweise ist die Komponente der Klasse (K4) ein öllösliches Umsetzungsprodukt auf Basis von mindestens eine tertiäre Aminogruppe aufweisenden Poly($C_2$- bis $C_{20}$-Carbonsäuren) der allgemeinen Formel IIa oder IIb

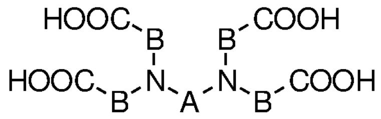

(IIa)

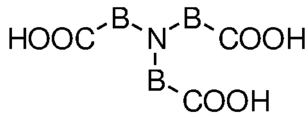

(IIb)

in denen die Variable A eine geradkettige oder verzweigte $C_2$- bis $C_6$-Alkylengruppe oder die Gruppierung der Formel III

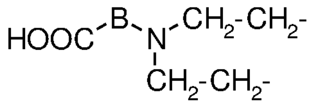

(III)

darstellt und die Variable B eine $C_1$- bis $C_{19}$-Alkylengruppe bezeichnet. Die Verbindungen der allgemeinen Formel IIa und IIb weisen insbesondere die Eigenschaften eines WASA auf.

Weiterhin ist das bevorzugte öllösliche Umsetzungsprodukt der Komponente (K4), insbesondere das der allgemeinen Formel IIa oder IIb, ein Amid, ein Amidammoniumsalz oder ein Ammoniumsalz, in dem keine, eine oder mehrere Carbonsäuregruppen in Amidgruppen übergeführt sind.

Geradkettige oder verzweigte $C_2$- bis $C_6$-Alkylengruppen der Variablen A sind beispielsweise 1,1-Ethylen, 1,2-Propylen, 1,3-Propylen, 1,2-Butylen, 1,3-Butylen, 1,4-Butylen, 2-Methyl-1,3-propylen, 1,5-Pentylen, 2-Methyl-1,4-butylen, 2,2-Dimethyl-1,3-propylen, 1,6-Hexylen (Hexamethylen) und insbesondere 1,2-Ethylen. Vorzugsweise umfasst die Variable A 2 bis 4, insbesondere 2 oder 3 Kohlenstoffatome.

$C_1$- bis $C_{19}$-Alkylengruppen der Variablen B sind vor beispielsweise 1,2-Ethylen, 1,3-Propylen, 1,4-Butylen, Hexamethylen, Octamethylen, Decamethylen, Dodecamethylen, Tetradecamethylen, Hexadecamethylen, Octadecamethylen, Nonadecamethylen und insbesondere Methylen. Vorzugsweise umfasst die Variable B 1 bis 10, insbesondere 1 bis 4 Kohlenstoffatome.

Die primären und sekundären Amine als Umsetzungspartner für die Polycarbonsäuren zur Bildung der Komponente (K4) sind üblicherweise Monoamine, insbesondere aliphatische Monoamine. Diese primären und sekundären Amine können aus einer Vielzahl von Aminen ausgewählt sein, die - gegebenenfalls miteinander verbundene - Kohlenwasserstoffreste tragen.

Meist sind diese den öllöslichen Umsetzungsprodukten der Komponente (K4) zugrundeliegenden Amine sekundären Amine und weisen die allgemeine Formel $HN(R^8)_2$ auf, in der die beiden Variablen $R^8$ unabhängig voneinander jeweils geradkettige oder verzweigte $C_{10}$- bis $C_{30}$-Alkylreste, insbesondere $C_{14}$- bis $C_{24}$-Alkylreste bedeuten. Diese längerkettigen Alkylreste sind vorzugsweise geradkettig oder nur in geringem Grade verzweigt. In der Regel leiten sich die genannten sekundären Amine hinsichtlich ihrer längerkettigen Alkylreste von natürlich vorkommenden Fettsäuren bzw. von deren Derivaten ab. Vorzugsweise sind die beiden Reste $R^8$ gleich.

Die genannten sekundären Amine können mittels Amidstrukturen oder in Form der Ammoniumsalze an die Polycarbonsäuren gebunden sein, auch kann nur ein Teil als Amidstrukturen und ein anderer Teil als Ammoniumsalze vorliegen. Vorzugsweise liegen nur wenige oder keine freien Säuregruppen vor. Vorzugsweise liegen die öllöslichen Umsetzungsprodukte der Komponente (K4) vollständig in Form der Amidstrukturen vor.

Typische Beispiele für derartige Komponenten (K4) sind Umsetzungsprodukte der Nitrilotriessigsäure, der Ethylendiamintetraessigsäure oder der Propylen-1,2-diamintetraessigsäure mit jeweils 0,5 bis 1,5 Mol pro Carboxylgruppe, insbesondere 0,8 bis 1,2 Mol pro Carboxylgruppe, Dioleylamin, Dipalmitinamin, Dikokosfettamin, Distearylamin, Dibehenylamin oder insbesondere Ditalgfettamin. Eine besonders bevorzugte Komponente (K4) ist das Umsetzungsprodukt aus 1 Mol Ethylendiamintetraessigsäure und 4 Mol hydriertem Ditalgfettamin.

Als weitere typische Beispiele für die Komponente (K4) seien die N,N-Dialkylammoni-umsalze von 2-N' ,N' -Dialkylamidobenzoaten, beispielsweise das Reaktionsprodukt aus 1 Mol Phthalsäureanhydrid und 2 Mol Ditalgfettamin, wobei letzteres hydriert oder nicht hydriert sein kann, und das Reaktionsprodukt von 1 Mol eines Alkenylspirobislactons mit 2 Mol eines Dialkylamins, beispielsweise Ditalgfettamin und/oder Talgfettamin, wobei die beiden letzteren hydriert

oder nicht hydriert sein können, genannt.

Weitere typische Strukturtypen für die Komponente der Klasse (K4) sind cyclische Verbindungen mit tertiären Aminogruppen oder Kondensate langkettiger primärer oder sekundärer Amine mit carbonsäurehaltigen Polymeren, wie sie in der WO 93/18115 beschrieben sind.

Als Kaltfließverbesserer der Komponente der Klasse (K5) geeignete Sulfocarbonsäuren, Sulfonsäuren oder deren Derivate sind beispielsweise die öllöslichen Carbonsäureamide und Carbonsäureester von ortho-Sulfobenzoesäure, in denen die Sulfonsäurefunktion als Sulfonat mit alkylsubstituierten Ammoniumkationen vorliegt, wie sie in der EP-A 261 957 beschrieben werden.

Als Kaltfließverbesserer der Komponente der Klasse (K6) geeignete Poly(meth)acrylsäureester sind sowohl Homo- als auch Copolymere von Acryl- und Methacrylsäureestern. Bevorzugt sind Copolymere von wenigstens zwei voneinander verschiedenen (Meth)Acrylsäureestern, die sich bezüglich des einkondensierten Alkohols unterscheiden. Gegebenenfalls enthält das Copolymer noch ein weiteres, davon verschiedenes olefinisch ungesättigtes Monomer einpolymerisiert. Das gewichtsmittlere Molekulargewicht des Polymers beträgt vorzugsweise 50.000 bis 500.000. Ein besonders bevorzugtes Polymer ist ein Copolymer von Methacrylsäure und Methacrylsäureestern von gesättigten $C_{14}$- und $C_{15}$-Alkoholen, wobei die Säuregruppen mit hydriertem Tallamin neutralisiert sind. Geeignete Poly(meth)acrylsäureester sind beispielsweise in der WO 00/44857 beschrieben.

Dem Mitteldestillat-Kraftstoff bzw. Dieselkraftstoff wird der Kaltfließverbesserer bzw. das Gemisch verschiedener Kaltfließverbesserer in einer Gesamtmenge von vorzugsweise 10 bis 5000 Gew.-ppm, besonders bevorzugt von 20 bis 2000 Gew.-ppm, stärker bevorzugt von 50 bis 1000 Gew.-ppm und insbesondere von 100 bis 700 Gew.-ppm, z.B. von 200 bis 500 Gew.-ppm, zugegeben.

B4) Schmierfähigkeitsverbesserer

Geeignete Schmierfähigkeitsverbesserer (Lubricity Improver bzw. Friction Modifier) basieren üblicherweise auf Fettsäuren oder Fettsäureestern. Typische Beispiele sind Tallölfettsäure, wie beispielsweise in der WO 98/004656 beschrieben, und Glycerinmonooleat. Auch die in der US 6 743 266 B2 beschriebenen Reaktionsprodukte aus natürlichen oder synthetischen Ölen, beispielsweise Triglyceriden, und Alkanolaminen sind als solche Schmierfähigkeitsverbesserer geeignet.

B5) Korrosionsinhibitoren

Geeignete Korrosionsinhibitoren sind z.B. Bernsteinsäureester, vor allem mit Polyolen, Fettsäurederivate, z.B. Ölsäureester, oligomerisierte Fettsäuren, substituierte Ethanolamine und Produkte, die unter dem Handelsnamen RC 4801 (Rhein Chemie Mannheim, Deutschland) oder HiTEC 536 (Ethyl Corporation) vertrieben werden.

B6) Demulgatoren

Geeignete Demulgatoren sind z.B. die Alkali- oder Erdalkalisalze von Alkyl-substituierten Phenol- und Naphthalinsulfonaten und die Alkali- oder Erdalkalisalze von Fettsäuren, außerdem neutrale Verbindungen wie Alkoholalkoxylate, z.B. Alkoholethoxylate, Phenolalkoxylate, z.B. tert-Butylphenolethoxylat oder tert-Pentylphenolethoxylat, Fettsäuren, Alkylphenole, Kondensationsprodunkte von Ethylenoxid (EO) und Propylenoxid (PO), z.B. auch in Form von EO/PO-Blockcopolymeren, Polyethylenimine oder auch Polysiloxane.

B7) Dehazer

Geeignete Dehazer sind z.B. alkoxylierte Phenol-Formaldehyd-Kondensate, wie beispielsweise die unter dem Handelsnamen erhältlichen Produkte NALCO 7D07 (Nalco) und TOLAD 2683 (Petrolite).

B8) Antischaummittel

Geeignete Antischaummittel sind z.B. Polyether-modifizierte Polysiloxane, wie beispielsweise die unter dem Handelsnamen erhältlichen Produkte TEGOPREN 5851 (Goldschmidt), Q 25907 (Dow Corning) und RHODOSIL (Rhone Poulenc).

B9) Cetanzahlverbesserer

Geeignete Cetanzahlverbesserer sind z.B. aliphatische Nitrate wie 2-Ethylhexylnitrat und Cyclohexylnitrat sowie Peroxide wie Di-tert-butylperoxid.

B10) Antioxidantien

Geeignete Antioxidantien sind z.B. substituierte Phenole, wie 2,6-Di-tert.-butylphenol und 6-Di-tert.-butyl-3-methylphenol sowie Phenylendiamine wie N,N'-Di-sec.-butyl-p-phenylendiamin.

B11) Metalldeaktivatoren

Geeignete Metalldeaktivatoren sind z.B. Salicylsäurederivate wie N,N'-Disalicyliden-1,2-propandiamin.

B12) Lösungsmittel

Geeignete sind z.B. unpolare organische Lösungsmittel wie aromatische und aliphatische Kohlenwasserstoffe, beispielsweise Toluol, Xylole, "white spirit" und Produkte, die unter dem Handelsnamen SHELLSOL (Royal Dutch/Shell Group) und EXXSOL (ExxonMobil) vertrieben werden, sowie polare organische Lösungsmittel, beispielsweise Alkohole wie 2-Ethylhexanol, Decanol und Isotridecanol. Derartige Lösungsmittel gelangen meist zusammen mit den vorgenannten Additiven und Co-Additiven, die sie zur besseren Handhabung lösen oder verdünnen sollen, in den Dieselkraftstoff.

**C) Kraftstoffe**

Das erfindungsgemäße Additiv eignet sich in hervorragender Weise als Kraftstoffzusatz und kann im Prinzip in jeglichen Kraftstoffen eingesetzt werden. Es bewirkt eine ganze Reihe von vorteilhaften Effekten beim Betrieb von Verbrennungsmotoren mit Kraftstoffen. Bevorzugt wird das erfindungsgemäße quaternisierte Additiv in Mitteldestillat-Kraftstoffen, insbesondere Dieselkraftstoffen, eingesetzt.

Gegenstand der vorliegenden Erfindung sind daher auch Kraftstoffe, insbesondere Mitteldestillat-Kraftstoffe, mit einem als Zusatzstoff zur Erzielung von vorteilhaften Effekten beim Betrieb von Verbrennungsmotoren, beispielsweise von Dieselmotoren, insbesondere von direkteinspritzenden Dieselmotoren, vor allem von Dieselmotoren mit Common-Rail-Einspritzsystemen, wirksamen Gehalt an dem erfindungsgemäßen quaternisierten Additiv. Dieser wirksame Gehalt (Dosierrate) liegt in der Regel bei 10 bis 5000 Gew.-ppm, vorzugsweise bei 20 bis 1500 Gew.-ppm, insbesondere bei 25 bis 1000 Gew.-ppm, vor allem bei 30 bis 750 Gew.-ppm, jeweils bezogen auf die Gesamtmenge an Kraftstoff.

Bei Mitteldestillat-Kraftstoffen wie Dieselkraftstoffen oder Heizölen handelt es sich vorzugsweise um Erdölraffinate, die üblicherweise einen Siedebereich von 100 bis 400°C haben. Dies sind meist Destillate mit einem 95%-Punkt bis zu 360°C oder auch darüber hinaus. Dies können aber auch so genannte "Ultra Low Sulfur Diesel" oder "City Diesel" sein, gekennzeichnet durch einen 95%-Punkt von beispielsweise maximal 345°C und einem Schwefelgehalt von maximal 0,005 Gew.-% oder durch einen 95%-Punkt von beispielsweise 285°C und einem Schwefelgehalt von maximal 0,001 Gew.-%. Neben den durch Raffination erhältlichen mineralischen Mitteldestillat-Kraftstoffen bzw. Dieselkraftstoffen sind auch solche, die durch Kohlevergasung oder Gasverflüssigung ["gas to liquid" (GTL)-Kraftstoffe] oder durch Biomasse-Verflüssigung ["biomass to liquid" (BTL)-Kraftstoffe] erhältlich sind, geeignet. Geeignet sind auch Mischungen der vorstehend genannten Mitteldestillat-Kraftstoffe bzw. Dieselkraftstoffe mit regenerativen Kraftstoffen, wie Biodiesel oder Bioethanol.

Die Qualitäten der Heizöle und Dieselkraftstoffe sind beispielsweise in DIN 51603 und EN 590 näher festgelegt (vgl. auch Ullmann' s Encyclopedia of Industrial Chemistry, 5. Auflage, Band A12, S. 617 ff.).

Das erfindungsgemäße quaternisierte Additiv kann neben seiner Verwendung in den oben genannten Mitteldestillat-Kraftstoffen aus fossilem, pflanzlichem oder tierischem Ursprung, die im wesentlichen Kohlenwasserstoffmischungen darstellen, auch in Mischungen aus solchen Mitteldestillaten mit Biobrennstoffölen (Biodiesel) eingesetzt werden. Derartige Mischungen werden im Sinne der vorliegenden Erfindung auch von dem Begriff" Mitteldestillat-Kraftstoff" umfasst. Sie sind handelsüblich und enthalten meist die Biobrennstofföle in untergeordneten Mengen, typischerweise in Mengen von 1 bis 30 Gew.-% insbesondere von 3 bis 10 Gew.-%, bezogen auf die Gesamtmenge aus Mitteldestillat fossilen, pflanzlichem oder tierischen Ursprungs und Biobrennstofföl.

Biobrennstofföle basieren in der Regel auf Fettsäureestern, vorzugsweise im wesentlichen auf Alkylester von Fettsäuren, die sich von pflanzlichen und/oder tierischen Ölen und/oder Fetten ableiten. Unter Alkylestern werden üblicherweise Niedrigalkylester, insbesondere $C_1$- bis $C_4$-Alkylester, verstanden, die durch Umesterung der in pflanzlichen und/oder tierischen Ölen und/oder Fetten vorkommenden Glyceride, insbesondere Triglyceride, mittels Niedrigalkoholen, beispielsweise Ethanol oder vor allem Methanol (" FAME"), erhältlich sind. Typische Niedrigalkylester auf Basis von pflanzlichen und/oder tierischen Ölen und/oder Fetten, die als Biobrennstofföl oder Komponenten hierfür Verwendung finden, sind beispielsweise Sonnenblumenmethylester, Palmölmethylester ("PME"), Sojaölmethylester ("SME") und insbesondere Rapsölmethylester ("RME").

Besonders bevorzugt handelt es sich bei den Mitteldestillat-Kraftstoffen bzw. Dieselkraftstoffen um solche mit niedrigem Schwefelgehalt, das heißt mit einem Schwefelgehalt von weniger als 0,05 Gew.-%, vorzugsweise von weniger

als 0,02 Gew.-%, insbesondere von weniger als 0,005 Gew.-% und speziell von weniger als 0,001 Gew.-% Schwefel.

Als Ottokraftstoffe kommen alle handelsüblichen Ottokraftstoffzusammensetzungen in Betracht. Als typischer Vertreter soll hier der marktübliche Eurosuper Grundkraftstoff gemäß EN 228 genannt werden. Weiterhin sind auch Ottokraftstoffzusammensetzungen der Spezifikation gemäß WO 00/47698 mögliche Einsatzgebiete für die vorliegende Erfindung.

Das erfindungsgemäße quaternisierte Additiv eignet sich insbesondere als Kraftstoffzusatz in Kraftstoffzusammensetzungen, insbesondere in Dieselkraftstoffen, zur Überwindung der eingangs geschilderten Probleme bei direkteinspritzenden Dieselmotoren, vor allem bei solchen mit Common-Rail-Einspritzsystemen.

Die Erfindung wird nun anhand der folgenden Ausführungsbeispiele näher beschrieben. Insbesondere die im Folgenden genannten Testmethoden sind Teil der allgemeine Offenbarung der Anmeldung und nicht auf die konkreten Ausführungsbeispiele beschränkt.

**Experimenteller Teil:**

### A. Allgemeine Testmethoden

#### Motorentests

#### 1. XUD9 Test - Bestimmung der Flow Restriction

Die Durchführung erfolgt nach den Standardbestimmungen gemäß CEC F-23-01.

#### 2. DW10 Test - Bestimmung des Leistungsverlusts durch Injektorablagerungen im Common Rall Dieselmotor

#### 2.1. DW10- KC - Keep Clean Test

Der Keep Clean Test lehnt sich an die CEC Test Prozedur F-098-08 Issue 5 an. Dabei kommen der gleiche Testaufbau und Motorentyp (PEUGEOT DW10) wie in der CEC Prozedur zum Einsatz.

**Änderung und Besonderheiten:**

Bei den Versuchen kamen gereinigte Injektoren zum Einsatz. Die Reinigungsdauer im Ultraschallbad in 60°C Wasser + 10% Superdecontamine (Intersciences, Brüssel) betrug 4h.

**Testlaufzeiten:**

Der Testzeitraum betrug 12h ohne Abstellphasen. Der in Figur 2 dargestellte einstündige Testzyklus aus der CEC F-098-08 wurde dabei 12-mal durchfahren.

**Leistungsbestimmung:**

Die Anfangsleistung P0,KC [kW] wird aus dem gemessenen Drehmoment bei 4000/min Volllast direkt nach Teststart und Warmlauf des Motors berechnet. Die Vorgehensweise ist in der Issue 5 der Testprozedur (CEC F-98-08) beschrieben. Dabei wird der gleiche Testaufbau und der Motorentyp PEUGEOT DW10 verwendet.

Die Endleistung (Pend,KC) wird im 12. Zyklus in Stufe 12, (siehe Tabelle, Figur 1) bestimmt. Auch hier ist der Betriebspunkt 4000/min Volllast. Pend,KC [kW] errechnet sich aus dem gemessenen Drehmoment.

Der Leistungsverlust im KC Test wird wie folgt berechnet:

$$\mathbf{Powerloss,KC\,[\%]} = \left(1 - \frac{Pend,KC}{P0,KC}\right) * 100$$

#### 2.2. DW10-Dirty Up - Clean Up-(DU-CU)

Der DU-CU Test lehnt sich an die CEC Test Prozedur F-098-08 Issue 5 an. Die Vorgehensweise ist in der Issue 5 der Testprozedur (CEC F-98-08) beschrieben. Dabei wird der gleiche Testaufbau und der Motorentyp PEUGEOT DW10 verwendet.

Der DU - CU Test besteht aus zwei einzelnen Tests, die hintereinander gefahren werden. Der erste Test dient

zur Ablagerungsbildung (DU), der zweite zum Entfernen der Ablagerungen (CU). Nach dem DU wird der Leistungsverlust (Powerloss) bestimmt. Nach Ende des DU Laufs wird der Motor für mindestens 8 Stunden nicht betrieben und auf Umgebungstemperatur abgekühlt. Danach wird mit dem CU Kraftstoff der CU gestartet, ohne die Injektoren auszubauen und zu reinigen. Die Ablagerungen und der powerloss gehen im Idealfall im CU-Testverlauf zurück.

**Änderung und Besonderheiten:**

Gereinigte Injektoren wurden vor jedem DU Test in den Motor eingebaut. Die Reinigungsdauer im Ultraschallbad bei 60°C , Wasser + 10% Superdecontamine (Intersciences, Brüssel) betrug 4h.

**Testlaufzeiten:**

Der Testzeitraum betrug 12h für den DU und 12h für den CU. Der Motor wurde im DU und CU Test ohne Abstellphasen betrieben.

Der in Figur 2 dargestellte einstündige Testzyklus aus der CEC F-098-08 wurde dabei jeweils 12-mal durchfahren.

**Leistungsbestimmung:**

Die Anfangsleistung P0,du [kW] wird aus dem gemessenen Drehmoment bei 4000/min Volllast direkt nach Teststart und Warmlauf des Motors berechnet. Die Vorgehensweise ist ebenfalls in der Issue 5 der Testprozedur beschrieben.

Die Endleistung (Pend,du) wird im 12. Zyklus in Stufe 12, (siehe Tabelle oben) bestimmt. Auch hier ist der Betriebspunkt 4000/min Volllast. Pend,du [kW] errechnet sich aus dem gemessenen Drehmoment.

Der Leistungsverlust im DU wird wie folgt berechnet

$$\textbf{Powerloss,du [\%]} = \left(1 - \frac{Pend,du}{P0,du}\right) * 100$$

Clean up

Die Anfangsleistung **P0,cu** [kW] wird aus dem gemessenen Drehmoment bei 4000/min Volllast direkt nach Teststart und Warmlauf des Motors im CU berechnet. Die Vorgehensweise ist ebenfalls in der Issue 5 der Testprozedur beschrieben.

Die Endleistung (Pend,cu) wird im 12. Zyklus in Stufe 12, (siehe Tabelle Figur 2) bestimmt. Auch hier ist der Betriebspunkt 4000/min Volllast. Pend,cu [kW] errechnet sich aus dem gemessenen Drehmoment.

Der Leistungsverlust im CU-Test wird wie folgt berechnet (negative Zahl beim powerloss im cu-Test bedeutet Leistungszuwachs)

$$\textbf{Powerloss (DU,CU)[\%]} = \left(\frac{Pend,du - pend,cu}{P0,du}\right) * 100$$

Als Kraftstoff wurde ein handelsüblicher Dieselkraftstoff der Fa. Haltermann (RF-06-03) eingesetzt. Diesem wurden zur künstlichen Anregung der Bildung von Ablagerungen an den Injektoren 1 Gew.-ppm Zink in Form einer Zink-Didodecanoat-Lösung zugesetzt.

**3. IDID Test - Bestimmung der Additivwirkung gegen interne Injektorablagerungen**

Die Bildung von Ablagerungen im Inneren des Injektors wurde anhand der Abweichungen der Abgastemperaturen der Zylinder am Zylinderausgang beim Kaltstart des DW10-Motors charakterisiert.

Zur Förderung der Bildung von Ablagerungen wurden dem Kraftstoff 1 mg/l Na Salz einer organischen Säure, 20 mg/l Dodecenylbernsteinsäure und 10 mg/l Wasser zugegeben.

Der Test wird als dirty-up-clean-up Test (DU-CU) durchgeführt.

DU-CU lehnt sich an die an die CEC Test Prozedur F-098-08 Issue 5 an.

Der DU - CU Test besteht aus zwei einzelnen Tests, die hintereinander gefahren werden. Der erste Test dient zur Ablagerungsbildung (DU), der zweite zum Entfernen der Ablagerungen (CU).

Nach dem DU Lauf wird nach einer mindestens achtstündigen Stillstands-Phase ein Kaltstart des Motors mit anschließendem 10-minütigen Leerlauf durchgeführt.

Danach wird mit dem CU Kraftstoff der CU gestartet, ohne die Injektoren auszubauen und zu reinigen. Nach dem CU Lauf über 8h wird nach einer mindestens achtstündigen Stillstands-Phase ein Kaltstart des Motors mit anschließendem 10-minütigen Leerlauf durchgeführt. Die Auswertung erfolgt durch den Vergleich der Temperaturverläufe für die einzelnen Zylinder nach Kaltstart des du und des CU-Laufs.

Der IDID-Test zeigt die interne Ablagerungsbildung im Injektor an. Als Kenngröße dient bei diesem Test die Abgastemperatur der einzelnen Zylinder. Bei einem Injektorsystem ohne IDID erhöhen sich die Abgastemperaturen der Zylinder gleichmäßig. Bei vorhandenem IDID erhöhen sich die Abgastemperaturen der einzelnen Zylinder nicht gleichmäßig und weichen voneinander ab.

Die Temperatursensoren befinden sich hinter dem Zylinderkopfaustritt im Abgaskrümmer. Signifikante Abweichung der einzelnen Zylindertemperaturen (z.B. > 20°C) zeigen das Vorliegen von internen Injektorablagerungen (IDID) an.

Die Tests (DU und CU) werden mit jeweils 8h Laufzeit durchgeführt. Der einstündige Testzyklus aus der CEC F-098-08 (siehe Figur 1) wird dabei jeweils 8-mal durchfahren. Bei Abweichungen der einzelnen Zylindertemperaturen von größer 45°C zum Mittelwert aller 4 Zylinder wird der Test vorzeitig abgebrochen.

Änderung und Besonderheiten: Gereinigte Injektoren wurden vor jedem DU-Testbeginn eingebaut. Die Reinigungsdauer im Ultraschallbad bei 60°C Wasser + 10% Superdecontamine betrug 4h.

**B. Herstellungsbeispiele:**

Verwendete Reagenzien:

Cocoyldimethylamin (N,N-Dimethyl-N-C12/14-amin, CAS 68439-70-3 bzw. 112-18-5) mit einer Gesamtaminzahl von 246 mg KOH/g.
Isononansäure (CAS 3302-10-1) von Fa. BASF.
Dimethyloxalat (CAS 553-90-2) von Fa. Aldrich
2-Propylheptanol (CAS 10042-59-8) von Fa. BASF.
Solvent Naphtha, naphthalene depleted : Hydrosol A 200 ND, CAS 64742-94-5, Fa. DHC Solvent Chemie GmbH
2-Ethylhexylamin (CAS 104-75-6) von Fa. BASF
Oleylamin (CAS 112-90-3) von Fa. Aldrich

**Vergleichsbeispiel 1: C12/14-NMe$_3$-methyloxalat (Vergleich)**

In einem 1 l Doppelmantelgefäß wird eine Mischung von Cocoyldimethylamin (300 g), Dimethyloxalat (233 g) und Isononansäure (1,1 g) für 24 h auf 120°C erhitzt. Anschließend wird überschüssiges Dimethyloxalat am Rotationsverdampfer bei 120°C im Vakuum entfernt. Man erhält das Produkt als beigen Feststoff. $^1$H-NMR ($CDCl_3$) bestätigt die Quaternierung ($\delta$ = 3.36 ppm (Singulett, RN($C\underline{H}_3$)$_3$), $\delta$ = 3.71 ppm (Singulett, - $O_2CCO_2CH_3$)).

**Beispiel 1: C12/14-NMe$_3$-(2-propylheptyl)-oxalat (Referenzbeispiel)**

Eine Lösung des Produktes aus Vergleichsbeispiel 1 (314 g) in 2-Propylheptanol (598 g) wird für 6 h auf 120°C erhitzt, wobei flüchtiges Methanol unter Überleiten eines Stickstoffstromes abdestilliert wird. Das Produkt wird als Lösung in 2-Propylheptanol erhalten. $^1$H-NMR ($CDCl_3$; nach Entfernung des Lösungsmittels bei 70°C im Hochvakuum) bestätigt die Umesterung.

**Beispiel 2: C12/14-NMe$_3$-(2-ethylhexl)-aminooxoacetat (Referenzbeispiel)**

Eine Lösung des Produktes aus Vergleichsbeispiel 1 (100 g) in Solvent Naphtha, naphthelene depleted (85 g) und 2-Ethylhexylamin (37,6 g) wird für 4 h auf 120°C erhitzt, wobei flüchtiges Methanol unter Überleiten eines Stickstoffstromes abdestilliert wird. $^1$H-NMR ($CDCl_3$; nach Entfernung des Lösungsmittels bei 70°C im Hochvakuum) bestätigt die Amidierung.

**Beispiel 3: C12/14-NMe$_3$-oleylaminooxoacetat (Referenzbeispiel)**

Eine Lösung des Produktes aus Vergleichsbeispiel 1 (100 g) in Solvent Naphtha, naphthelene depleted (211 g) und Oleylamin (111 g) wird für 4 h auf 120°C erhitzt, wobei flüchtiges Methanol unter Überleiten eines Stickstoffstromes abdestilliert wird. $^1$H-NMR ($CDCl_3$; nach Entfernung des Lösungsmittels bei 70°C im Hochvakuum) bestätigt die Amidierung.

**Beispiel 4:** (Referenzbeispiel)

In einem 1 l Doppelmantelgefäß wird eine Mischung von N-Cocoylmorpholin (Gesamtaminzahl 207 mg KOH/g, 200 g), Dimethyloxalat (261 g) und Isononansäure (9,2 g) für 4 h auf 160°C erhitzt. $^1$H-NMR ($CDCl_3$) bestätigt die Quaternierung ($\delta$ = 3.5 ppm, Singulett, $R_3NC\underline{H}_3$). Anschließend wird überschüssiges Dimethyloxalat am Rotationsverdampfer bei 120°C im Vakuum entfernt. Das Reaktionsprodukt wird in 2-Propylheptanol (460 g) gelöst und für 6 h auf 120°C erhitzt, wobei flüchtiges Methanol unter Überleiten eines Stickstoffstromes abdestilliert wird. Das Produkt wird als Lösung in 2-Propylheptanol erhalten.

**C. Anwendungsbeispiele:**

In den folgenden Anwendungsbeispielen werden die Additive als Reinsubstanz oder als Lösung in einem Lösungsmittel eingesetzt.

**Anwendungsbeispiel 1: Bestimmung der Löslichkeits- und Formulierungseigenschaften**

Die Verbindung gemäß Vergleichsbeispiel 1: C12/14-NMe3-methyloxalat ist ein kristalliner Feststoff und unlöslich in Solvent Naphtha bei einer Konzentration von 50% (w/w). Die Verbindung gemäß Erfindungsgemäßem Beispiel 1: C12/14-NMe3-(2-propylheptyl)-oxalat ist flüssig und lagerstabil bei Zimmertemperatur in 50%-iger Lösung in Solvent Naphtha. Dies verdeutlicht die signifikant verbesserten HandlingEigenschaften der erfindungsgemäßen Verbindung.

Folgende Formulierungen wurden auf Lagerstabilität bei -20 °C getestet:

a) 1:5 Verdünnung in Solvent Naphtha (5 Teile):

Man beobachtet folgendes:
Vergleichsbeispiel 1. Verfestigung nach 44 Tagen bei -20°C
Erfindungsgemäßes Beispiel 1: klare Flüssigkeit nach 60 Tagen bei -20°C

b) 1:5:1 Verdünnung in 2-Ethylhexylnitrate (5 Teile) und Solvent Naphtha (1 Teil)

Man beobachtet folgendes:
Vergleichsbeispiel 1: Verfestigung nach 1 tag bei -20°C
Erfindungsgemäßes Beispiel 1: klare Flüssigkeit nach 14 Tagen bei -20°C

**Anwendungsbeispiel 2: Bestimmung der Motoröl-Kompatibilität**

Bestimmung nach Norm DGMK 531 1-A mit Kraftstoff EN590 B7 von Aral unadditiviert, und Motorenöl Wintershall Multi-Rekord Top 15W-40.

In einen 500mL Erlenmeyerkolben werden je 10g Motorenöl und 10g des zu prüfenden Additivs eingewogen und homogenisiert. Der die Mischung enthaltende Kolben wird mit einem Glasstopfen locker verschlossen und drei Tage bei einer Temperatur von 90 ± 3°C im Trockenschrank konditioniert. Nach der Konditionierung wird die Probe eine Stunde lang bei Raumtemperatur im Abzug abgekühlt. Die Mischung wird nun mit 500mL Dieselkraftstoff aufgefüllt und gut durchmischt. Ein Membranfilter, Durchmesser 50mm, Porenweite 0,8μm wird in die Filtrationsapparatur eingelegt. Mischung wird durch den Membranfilter mit Hilfe von Vakuum mit einem Druck von 200mbar filtriert. Die Filtrationszeit wird gemessen.

Man beobachtet folgendes:

Vergleichsbeispiel : sehr schlechte Filtrierbarkeit, Filtrationszeit > 300 sec. (fail) Erfindungsgemäßes Beispiel 1: gute Filtrierbarkeit, Filtrationszeit 114 sec. (pass)

**Anwendungsbeispiel 3: XUD9 Motorentest (keep clean)**

Test nach obiger Prozedur CEC F-23-01 in Peugeot XUD9 Motor. Kraftstoff EN590 B7 Aral unadditiviert: Testergebnis (flow restriction bei 0.1 mm Nadelhub) für Erfindungsgemäßes Beispiel 1:

| Dosierung [mg/kg] | Flow restriction |
|---|---|
| 0 | 76.8% |
| 24 | 46.6% |
| 36 | 14.5% |
| 48 | 4.5% |

Die erfindungsgemäße Verbindung ist somit sehr wirksam hinsichtlich Vermeidung von Ablagerungen in indirekt einspritzenden Motoren. Verbindung ist auch in der Lage vorhandene Ablagerungen zu entfernen (clean up Effekt).

**Anwendungsbeispiel 4:** DW10 Zn Motorentest (clean up)

Der Test wurde mit einem Peugeot DW10 Motor durchgeführt, der in CEC F-98-08 Prozedur verwendet wird, wobei in Abwandlung jedoch schärfere Bedingungen im Dirty up-Teil verwendet wurden:

I. Dirty up: die schärferen Bedingungen erlauben die deutlich schnellere Bildung von Injektorablagerungen als unter Standard CEC F-98-08 Bedingungen: Der Motor wurde 4 h bei Volllast (4000 rpm) mit EN590 B7 Aral, unadditiviert, enthaltend 3 mg/kg Zn, betrieben.

<u>Testergebnis:</u>

Unter diesen Bedingungen verringerte sich die Motorenleistung nach 4 h von 96.4 kW auf 92.1 kW, was einem Power loss von 5.4% entspricht.

II. Clean up:

Auf 8 h verkürzte CEC F-98-08 Prozedur mit 1 ppm Zn in EN590 B7 Aral unadditiviert, enthaltend 25 mg/kg aktive Verbindung gemäß erfindungsgemäßem Beispiel 1.

<u>Testergebnis:</u>

Motorenleistung

- bei Testbeginn: 90.2 kW,
- nach 3 h Motorenlauf: 97.7 kW (d.h. volle Wiederherstellung der Motorenleistung)
  Die Motorenleistung blieb konstant bei 97.7 kW bis zum Ende des 8 h dauernden Clean up Tests

Die Verbindung gemäß Erfindungsgemäßem Beispiel 1 (C12/14-NMe3-(2-propylheptyl)-oxalat) besitzt besondere Wirksamkeit gegen Ablagerungen in direkt einspritzenden Motoren. Die Verbindung zeigt hohe Wirksamkeit sowohl bei der Verhinderung von Aufbau von Ablagerungen als auch bei der Entfernung von bestehenden Ablagerungen.

**Anwendungsbeispiel 5: DW10 Na soap IDID Test (clean up)**

Zur Untersuchung des Einflusses der Additive auf die Performance von direkteinspritzenden Dieselmotoren wurde als weitere Testmethode der IDID-Motorentest durchgeführt, bei dem die Abgastemperaturen der Zylinder am Zylinderausgang beim Kaltstart des DW10-Motors bestimmt wurden. (vgl. obige Methodenbeschreibung)

Verwendet wurde ein direkteinspritzender Dieselmotor DW10 mit Common-Rail-System des Herstellers Peugeot, der in Testmethode CEC F-098-08 eingesetzt wird. Die Methode die verwendet wurde leitet sich ab von CEC F-098-08 mit der Abwandlung, dass anstatt 1ppm Zn zur künstlichen Anregung der Bildung von Ablagerungen jeweils 1 Gew.-ppm Natriumnaphthenat sowie 20 Gew.-ppm Dodecenylbernsteinsäure zugesetzt wurden. Als Kraftstoff wurde ein handelsüblicher B7 Dieselkraftstoff gemäß EN 590 der Fa. Aral eingesetzt. Die Testdauer wird im Vergleich zu Methode CEC F-098-08 auf 8 Stunden gekürzt. Nach jeweils DU und CU Phasen wird der Motor abgekühlt und anschließend im Leerlauf gestartet.

Testergebnisse:

I. DU (Dirty up)

Es wurden jeweils die Abgastemperaturen der 4 Zylinder (" Z1" bis " Z4") an den Zylinderausgängen nach 0 Minuten (" ϑ 0") und nach 5 Minuten (" ϑ 5")gemessen. Die Ergebnisse der Abgastemperatur-Messungen mit Durchschnittswerten ("Δ") und den größten Abweichungen von Δ nach unten ("-") und oben ("+") für die beiden Testläufe sind in der folgenden Übersicht zusammengefasst:

| | | | | |
|---|---|---|---|---|
| ϑ 0 | Z1: 28°C | Z2: 27°C | Z3: 27°C | Z4: 26°C |
| ϑ 5 | Z1: 36°C | Z2: 68°C | Z3: 108°C | Z4: 121°C |

Δ : 83°C (-47°C / +38°C)

Eine signifikante Abweichung vom Mittelwert der Abgastemperaturen und die signifikanten Unterschiede zwischen einzelnen Zylinder weise auf die Bildung von IDID hin.

II. CU (Clean up) Nach der DU Phase wird der Motor mit additiviertem Kraftstoff betrieben, ohne vorher die Injektoren zu reinigen. Die Additivierung erfolgt mit 70 mg/kg aktive Verbindung gemäß Erfindungsgemäßem Beispiel 1: Es wurden wieder jeweils die Abgastemperaturen der 4 Zylinder ("Z1" bis "Z4") an den Zylinderausgängen nach 0 Minuten ("ϑ 0") und nach 5 Minuten ("ϑ 5") gemessen. Die Ergebnisse der Abgastemperatur-Messungen mit Durchschnittswerten ("Δ") und den größten Abweichungen von Δ nach unten ("-") und oben ("+") für die beiden Testläufe sind in der folgenden Übersicht zusammengefasst:

| | | | | |
|---|---|---|---|---|
| ϑ 0 | Z1: 28°C | Z2: 27°C | Z3: 27°C | Z4: 26°C |
| ϑ 5 | Z1: 78°C | Z2: 82°C | Z3: 77°C | Z4: 69°C |

Δ : 77°C (-8°C / +5°C)

Die Abweichung vom Mittelwert ist nicht signifikant; die gebildeten IDID wurden entfernt.

Die Verbindung gemäß Erfindungsgemäßem Beispiel 1 (C12/14-NMe3-(2-propylheptyl)-oxalat) besitzt besondere Wirksamkeit gegen IDID Bildung Die Verbindung zeigt hohe Wirksamkeit sowohl bei der Verhinderung von Aufbau von Ablagerungen als auch bei der Entfernung von bestehenden Ablagerungen.

**Anwendungsbeispiel 6: DW10 Na Power loss Test (keep clean)**

Zur Untersuchung der Wirksamkeit der erfindungsgemäßen Verbindungen gegen Leistungsverlust, verursacht durch Metalle, wie Na, K und andere (und nicht durch Zn wie oben beschrieben), wurde ein IDID-Motorentest eingesetzt.. Während des Laufs wird die Leistung nach CEC F-098-08 gemessen.

| Test | Additiv | Motorenleistung vor dem Test, [kW] | Motorenleistung nach dem Test, [kW] | Leistungsänderung im Test [%] |
|---|---|---|---|---|
| Keep clean, 8 h | 1 ppm Na + 20 ppm Dodecenylbernsteinsäure | 97,8 | 91,9 | -6,0 |
| Keep clean, 8 h | 1 ppm Na + 20 ppm Dodecenylbernsteinsäure und 150 ppm C12/14-N $Me_3$-(2-propylheptyl)-oxalat | 96,4 | 96,6 | +0,2 |

Die Verbindungen gemäß dieser Erfindung sind gegen Ablagerungen anderen Metallen als Zn in Motoren mit Direkteinspritzung wirksam, wie in dem obigen Na Power loss Test gezeigt Die Verbindungen verhindern in wirksamer Weise Verlustleistung und können auch verwendet werden, um Ablagerungen zu entfernen.

Auf die Offenbarung der hierin zitierten Druckschriften wird ausdrücklich Bezug genommen.

## Patentansprüche

1. Verwendung eines eine quaternisierte Stickstoffverbindung umfassenden Reaktionsprodukts oder einer aus dem Reaktionsprodukt durch Aufreinigung erhaltene, eine quaternisierte Stickstoffverbindung enthaltende Teilfraktion davon,
als Kraftstoffadditiv in einem Anteil von 10 bis 5000 Gew.-ppm, bezogen auf die Gesamtmenge des Kraftstoffs, wobei das Reaktionsprodukt erhalten ist durch

    a) Umsetzung einer quaternisierbaren Stickstoffverbindung, enthaltend wenigstens eine quaternisierbare Aminogruppe mit einem Quaternierungsmittel, das die wenigstens eine quaternisierbare Aminogruppe in eine quaternäre Ammoniumgruppe überführt,
    wobei das Quaternierungsmittel der Methyl- oder Ethylester einer aliphatischen, cycloaromatischen oder cycloaliphatischen Polycarbonsäure ist; und
    b) Umesterung der verbleibenden Estergruppe des Quaternisierungsprodukts aus Stufe a);

    wobei
    die Umesterung erfolgt mittels eines Alkohols, ausgewählt unter

    a) Polyalkoxylaten, der allgemeinen Formel 1

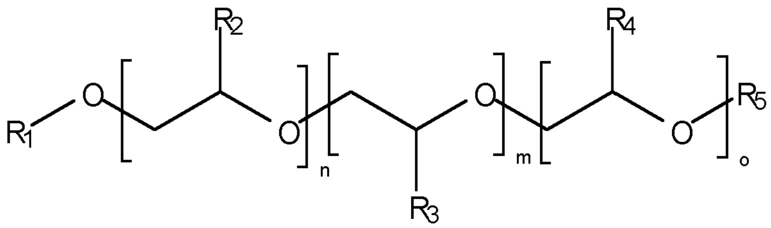

(1)

    worin:

    $R_1$ und $R_5$ unabhängig voneinander für H, Alkyl, Alkenyl oder Aryl stehen, wobei wenigstens einer der beiden Reste für H steht;
    $R_2$, $R_3$ und $R_4$ unabhängig voneinander für H, $C_1$-$C_{30}$-Alkyl, insbesondere $C_1$-$C_5$ Alkyl, stehen; und
    n, m und o unabhängig voneinander für ganzzahlige Werte von 0 bis 100 stehen;
    mit Molekularmassen von 40 bis 10.000 g/mol, ermittelt durch Bestimmung der OH-Zahl (DIN 53240-2).
    b) Polyolgestarteten Polyalkoxylaten mit Molekularmassen im Bereich von 100 bis 10.000 g/mol, ermittelt durch Bestimmung der OH-Zahl (DIN 53240-2).

    wobei die zur Umesterung der verbleibenden Estergruppe verwendete Verbindung a) oder b) einen Verteilungskoeffizient logP von größer als 0,7, insbesondere größer als 0,8, größer als 0,9, größer als 1, oder größer als 1,1 aufweist, wobei der logP- Wert bis zu einem Wert von etwa 6 nach OECD Richtlinie 117 bei 25 °C in n-Octanol/Wasser bestimmt wird und über einem Wert von 6 nach der ACD/LogP Methode bestimmt wird.

2. Verwendung nach Anspruch 1, wobei die Polyalkoxylate der Formel (1) Homo- oder Copolymere sind.

3. Verwendung nach Anspruch 1, wobei die polyolgestarteten Polyalkoxylate Homo- oder Copolymere sind.

4. Verwendung nach Anspruch 3, wobei das Polyalkoxylat ein Homo- oder Copolymer von Alkenyloxiden, ausgewählt unter Ethylenoxid, Propylenoxid und Butylenoxid, ist.

5. Verwendung nach Anspruch 1, wobei die Umesterung mittels einer Polyoxy-$C_2$-bis $C_4$-alkylengruppierungen enthaltenden Verbindung erfolgt, welche durch Umsetzung von $C_2$- bis $C_{60}$-Alkanolen, $C_6$- bis $C_{30}$-Alkandiolen, $C_1$- bis $C_{30}$-Alkyl-cyclohexanolen oder $C_1$- bis $C_{30}$-Alkylphenolen mit 1 bis 30 mol Ethylenoxid und/oder Propylenoxid und/oder Butylenoxid pro Hydroxylgruppe erhältlich sind.

6. Verwendung nach Anspruch 1, wobei die Umesterung mittels eines alkoholgestarteten Polyethers mit 5 bis 35 $C_3$-

bis $C_6$-Alkylenoxideinheiten pro Alkoholmolekül erfolgt.

**7.** Verwendung nach Anspruch 6, wobei der Starteralkohol ein langkettiges Alkanol oder langkettiges Alkyl-substituiertes Phenol ist, wobei der langkettige Alkylrest für einen geradkettigen oder verzweigten $C_6$- bis $C_{18}$-Alkylrest steht.

**8.** Verwendung nach einem der vorhergehenden Ansprüche als Additiv zur Verringerung des Kraftstoffverbrauches von direkteinspritzenden Dieselmotoren und/oder zur Minimierung des Leistungsverlustes (powerloss) in direkteinspritzenden Dieselmotoren.

**9.** Verwendung nach einem der Ansprüche 1 bis 7 als Ottokraftstoffadditiv zur Verringerung von Ablagerungen im Einlasssystem eines Ottomotors.

**10.** Verwendung nach einem der Ansprüche 1 bis 7 als Dieselkraftstoffadditiv zur Verringerung und/oder Vermeidung von Ablagerungen in den Einspritzsystemen und / oder von Ventilkleben in direkteinspritzenden Dieselmotoren.

**11.** Verwendung nach einem der vorhergehenden Ansprüche, wobei die quaternisierbare Stickstoffverbindung ausgewählt ist unter:

a) wenigstens einem Alkylamin der folgenden allgemeinen Formel 3:,

$$R_aR_bR_cN \qquad (3)$$

worin

wenigstens einer der Reste $R_a$,$R_b$ und $R_c$ für einen geradkettigen oder verzweigte, gesättigten oder ungesättigten $C_8$-$C_{40}$-Hydrocarbylrest steht und die übrigen Reste für gleiche oder verschiedene, geradkettige oder verzweigte, gesättigte oder ungesättigten $C_1$-$C_6$-Hydrocarbylreste stehen; oder
worin alle Reste $R_a$,$R_b$ und $R_c$ für gleiche oder verschiedene geradkettige oder verzweigte, gesättigten oder ungesättigten $C_8$-$C_{40}$-Hydrocarbylreste stehen.

b) wenigstens einem Polyalken-substituierten Amin, enthaltend wenigstens eine quaternisierbare Aminogruppe;
c) wenigstens einem Polyether-substituierten Amin, enthaltend wenigstens eine quaternisierbare Aminogruppe; und
d) wenigstens einem Reaktionsprodukt eines hydrocarbylsubstituierten Acylierungsmittels und einer Verbindung, enthaltend ein Stickstoff- oder Sauerstoffatom und zusätzlich enthaltend wenigstens eine quaternisierbare Aminogruppe; und
e) Mischungen davon.

**12.** Verwendung nach einem der Ansprüche 1 bis 11, wobei das Quaternierungsmittel eine Verbindung der allgemeinen Formel 2 ist

$$R_1OC(O)\text{-}A\text{-}C(O)OR_{1a} \qquad (2)$$

worin

$R_1$ und $R_{1a}$ unabhängig voneinander für einen Methyl- oder Ethylrest steht und
A für eine chemische Bindung oder ein gegebenenfalls ein- oder mehrfach substituiertes Hydrocarbylen, oder für einen gegebenenfalls substituierten einkernigen Arylen- oder Cycloalkylenrest steht.

**13.** Verwendung nach einem der vorhergehenden Ansprüche, wobei das quaternisierbare tertiäre Amin eine Verbindung der Formel 3 ist, worin wenigstens zwei der Reste $R_a$, $R_b$ und $R_c$ gleich oder verschieden sind und für einen geradkettigen oder verzweigten, $C_{10}$-$C_{20}$-Alkylrest steht und der übrigen Rest für $C_1$-$C_4$-Alkyl steht.

**14.** Verwendung nach einem der vorhergehenden Ansprüche, wobei das Quaternierungsmittel ausgewählt ist unter Di-methyl- oder ethyl-phthalaten und Di-methyl- oder ethyl-oxalaten.

**15.** Verwendung nach einem der vorhergehenden Ansprüche, wobei der Kraftstoff ausgewählt ist unter Mitteldestillat-Kraftstoffen, wie insbesondere Heizölen und Dieselkraftstoffen, Biodieselkraftstoffen, Ottokraftstoffen, und Alkanol-haltigen Ottokraftstoffen.

**16.** Quaternisierte Stickstoffverbindung gemäß der Definition in einem der Ansprüche 1 bis 15.

**17.** Verfahren zur Herstellung einer quaternisierter Stickstoffverbindung nach Anspruch 16, umfassend

a) die Umsetzung einer quaternisierbaren Stickstoffverbindung, enthaltend wenigstens eine quaternisierbare Aminogruppe mit einem Quaternierungsmittel, das die wenigstens eine Aminogruppe in eine quaternäre Ammoniumgruppe überführt,
wobei das Quaternierungsmittel der Methyl- oder Ethylester einer aliphatischen, cycloaromatischen oder cycloaliphatischen Polycarbonsäure ist; und
b) die Umesterung mit einer wie in einem der Ansprüche 1 bis 7 definierten Verbindung erfolgt.

**18.** Additivkonzentrat, enthaltend in Kombination mit weiteren Diesel- oder Ottokraftstoffadditiven wenigstens eine quaternisierte Stickstoffverbindung gemäß der Definition in Anspruch 16 oder hergestellt nach Anspruch 17.

**19.** Kraftstoffzusammensetzung, enthaltend in einem Anteil von 10 bis 5000 Gew.-ppm, bezogen auf die Gesamtmenge des Kraftstoffs, wenigstens eine quaternisierte Stickstoffverbindung umfassenden Reaktionsprodukts oder eine aus dem Reaktionsprodukt durch Aufreinigung erhaltene, eine quaternisierte Stickstoffverbindung enthaltende Teilfraktion davon, wobei das Reaktionsprodukt erhältlich ist durch

a) die Umsetzung einer quaternisierbaren Stickstoffverbindung, enthaltend wenigstens eine quaternisierbare, Aminogruppe mit einem Quaternierungsmittel, das die wenigstens eine Aminogruppe in eine quaternäre Ammoniumgruppe überführt,
wobei das Quaternierungsmittel der Methyl- oder Ethylester einer aliphatischen, cycloaromatischen oder cycloaliphatischen Polycarbonsäure ist; und
b) die Umesterung oder Amidierung der verbleibenden Estergruppe des Quaternisierungsprodukts aus Stufe a); wobei
die Umesterung mit einer wie in einem der Ansprüche 1 bis 7 definierten Verbindung erfolgt.

**20.** Kraftstoffzusammensetzung nach Anspruch 19, wobei die quaternisierbare Stickstoffverbindung ausgewählt ist unter

a) wenigstens einem Alkylamin der obigen allgemeinen Formel 3
b) wenigstens einem Polyalken-substituierten Amin, enthaltend wenigstens eine quaternisierbare Aminogruppe;
c) wenigstens einem Polyether-substituierten Amin, enthaltend wenigstens eine quaternisierbare Aminogruppe; und
d) wenigstens einem Reaktionsprodukt eines hydrocarbylsubstituierten Acylierungsmittels und einer Verbindung, enthaltend ein Stickstoff- oder Sauerstoffatom und zusätzlich enthaltend wenigstens eine quaternisierbare Aminogruppe; und
e) Mischungen davon.

**21.** Kraftstoffzusammensetzung nach einem der Ansprüche 19 und 20, wobei das Quaternierungsmittel eine Verbindung der obigen allgemeinen Formel 2 ist.

**22.** Kraftstoffzusammensetzung nach einem der Ansprüche 19 bis 21, wobei das quaternisierbare tertiäre Amin eine Verbindung der Formel 3 ist, worin wenigstens zwei der Reste $R_a$, $R_b$ und $R_c$ gleich oder verschieden sind und für einen geradkettigen oder verzweigten, $C_{10}$-$C_{20}$-Alkylrest steht und der übrigen Rest für $C_1$-$C_4$-Alkyl steht.

**23.** Kraftstoffzusammensetzung nach einem der Ansprüche 19 bis 22, wobei das Quaternierungsmittel ausgewählt ist unter Di-methyl- oder ethyl-phthalaten und Di-methyl- oder ethyl-oxalaten.

## Claims

**1.** The use of a reaction product comprising a quaternized nitrogen compound or of a fraction thereof which comprises a quaternized nitrogen compound and is obtained from the reaction product by purification,
as a fuel additive in a proportion of from 10 to 5000 ppm by weight, based on the total amount of the fuel,
where the reaction product has been obtained by

a) reacting a quaternizable nitrogen compound comprising at least one quaternizable amino group with a quaternizing agent which converts the at least one quaternizable amino group to a quaternary ammonium group, where the quaternizing agent is the methyl or ethyl ester of an aliphatic, cycloaromatic or cycloaliphatic polycarboxylic acid; and
b) transesterifying the remaining ester group of the quartenization product from stage a);

where
the transesterification is effected by means of an alcohol selected from

a) polyalkoxylates of the general formula 1

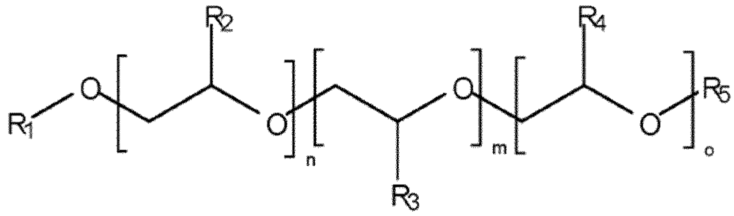

(1)

in which:

$R_1$ and $R_5$ are each independently H, alkyl, alkenyl or aryl, where at least one of the two radicals is H;
$R_2$, $R_3$ and $R_4$ are each independently H, $C_1$-$C_{30}$-alkyl, especially $C_1$-$C_5$ alkyl; and
n, m and o are each independently integer values from 0 to 100;
having molecular masses from 40 to 10 000 g/mol, found by determining the OH number (DIN 53240-2);

b) polyol-started polyalkoxylates having molecular masses in the range from 100 to 10 000 g/mol, found by determining the OH number (DIN 53240-2);

where the compound a) or b) used for transesterification of the remaining ester group has a coefficient of partition logP of greater than 0.7, especially greater than 0.8, greater than 0.9, greater than 1, or greater than 1.1,
where the logP value up to a value of about 6 is determined according to OECD Guideline 117 at 25°C in n-octanol/water, and above a value of 6 by the ACD/LogP method.

**2.** The use according to claim 1, wherein the polyalkoxylates of the formula (1) are homo- or copolymers.

**3.** The use according to claim 1, wherein the polyol-started polyalkoxylates are homo- or copolymers.

**4.** The use according to claim 3, wherein the polyalkoxylate is a homo- or copolymer of alkenyl oxides selected from ethylene oxide, propylene oxide and butylene oxide.

**5.** The use according to claim 1, wherein the transesterification is effected by means of a compound comprising polyoxy-$C_2$- to -$C_4$-alkylene moieties which are obtainable by reacting $C_2$- to $C_{60}$-alkanols, $C_6$- to $C_{30}$-alkanediols, $C_1$- to $C_{30}$-alkylcyclohexanols or $C_1$- to $C_{30}$-alkylphenols with 1 to 30 mol of ethylene oxide and/or propylene oxide and/or butylene oxide per hydroxyl group.

**6.** The use according to claim 1, wherein the transesterification is effected by means of an alcohol-started polyether having 5 to 35 $C_3$- to $C_6$-alkylene oxide units per alcohol molecule.

**7.** The use according to claim 6, wherein the starter alcohol is a long-chain alkanol or long-chain alkylsubstituted phenol, where the long-chain alkyl radical is a straight-chain or branched $C_6$- to $C_{18}$-alkyl radical.

**8.** The use according to any of the preceding claims as additive for reducing the fuel consumption of direct injection diesel engines and/or for minimizing power loss in direct injection diesel engines.

**9.** The use according to any of claims 1 to 7 as gasoline fuel additive for reducing deposits in the intake system of a gasoline fuel engine.

**10.** The use according to any of claims 1 to 7 as diesel fuel additive for reducing and/or preventing deposits in the intake systems and/or valve sticking in direct injection diesel engines.

**11.** The use according to any of the preceding claims, wherein the quaternizable nitrogen compound is selected from:

a) at least one alkylamine of the following general formula 3:

$$R_aR_bR_cN \qquad (3)$$

in which

at least one of the $R_a$, $R_b$ and $R_c$ radicals is a straight-chain or branched, saturated or unsaturated $C_8$-$C_{40}$-hydrocarbyl radical and the other radicals are identical or different, straight-chain or branched, saturated or unsaturated $C_1$-$C_6$-hydrocarbyl radicals; or
in which all the $R_a$, $R_b$ and $R_c$ radicals are identical or different, straight-chain or branched, saturated or unsaturated $C_8$-$C_{40}$-hydrocarbyl radicals;

b) at least one polyalkene-substituted amine comprising at least one quaternizable amino group;
c) at least one polyether-substituted amine comprising at least one quaternizable amino group; and
d) at least one reaction product of a hydrocarbyl-substituted acylating agent and a compound comprising a nitrogen or oxygen atom and additionally comprising at least one quaternizable amino group; and
e) mixtures thereof.

**12.** The use according to any of claims 1 to 11, wherein the quaternizing agent is a compound of the general formula 2

$$R_1OC(O)\text{-}A\text{-}C(O)OR_{1a} \qquad (2)$$

in which

$R_1$ and $R_{1a}$ are each independently a methyl or ethyl radical and
A is a chemical bond or an optionally mono- or polysubstituted hydrocarbylene, or an optionally substituted monocyclic arylene or cycloalkylene radical.

**13.** The use according to any of the preceding claims, wherein the quaternizable tertiary amine is a compound of the formula 3 in which at least two of the $R_a$, $R_b$ and $R_c$ radicals are the same or different and are each a straight-chain or branched $C_{10}$-$C_{20}$-alkyl radical and the other radical is $C_1$-$C_4$-alkyl.

**14.** The use according to any of the preceding claims, wherein the quaternizing agent is selected from dimethyl or diethyl phthalates and dimethyl or diethyl oxalates.

**15.** The use according to any of the preceding claims, wherein the fuel is selected from middle distillate fuels, such as heating oils and diesel fuels in particular, biodiesel fuels, gasoline fuels, and alkanol-containing gasoline fuels.

**16.** A quaternized nitrogen compound as defined in any of claims 1 to 15.

**17.** A process for preparing a quaternized nitrogen compound according to claim 16, comprising

a) the reaction of a quaternizable nitrogen compound comprising at least one quaternizable amino group with a quaternizing agent which converts the at least one amino group to a quaternary ammonium group,
where the quaternizing agent is the methyl or ethyl ester of an aliphatic, cycloaromatic or cycloaliphatic polycarboxylic acid; and
b) the transesterification is effected by means of a compound as defined in any of claims 1 to 7.

**18.** An additive concentrate comprising, in combination with further diesel fuel additives or gasoline fuel additives, at least one quaternized nitrogen compound as defined in claim 16 or prepared according to claim 17.

**19.** A fuel composition comprising, in a proportion of from 10 to 5000 ppm by weight, based on the total amount of the fuel, at least reaction product comprising a quaternized nitrogen compound, or a fraction thereof which comprises

a quaternized nitrogen compound and is obtained from the reaction product by purification, where the reaction product is obtainable by

a) the reaction of a quaternizable nitrogen compound comprising at least one quaternizable amino group with a quaternizing agent which converts the at least one amino group to a quaternary ammonium group,
where the quaternizing agent is the methyl or ethyl ester of an aliphatic, cycloaromatic or cycloaliphatic polycarboxylic acid; and
b) the transesterification or amidation of the remaining ester group of the quaternization product from stage a); where
the transesterification is effected by means of a compound as defined in any of claims 1 to 7.

**20.** The fuel composition according to claim 19, wherein the quaternizable nitrogen compound is selected from

a) at least one alkylamine of the above general formula 3
b) at least one polyalkene-substituted amine comprising at least one quaternizable amino group;
c) at least one polyether-substituted amine comprising at least one quaternizable amino group; and
d) at least one reaction product of a hydrocarbyl-substituted acylating agent and a compound comprising a nitrogen or oxygen atom and additionally comprising at least one quaternizable amino group; and
e) mixtures thereof.

**21.** The fuel composition according to either of claims 19 and 20, wherein the quaternizing agent is a compound of the above general formula 2.

**22.** The fuel composition according to any of claims 19 to 21, wherein the quaternizable tertiary amine is a compound of the formula 3 in which at least two of the $R_a$, $R_b$ and $R_c$ radicals are the same or different and are each a straight-chain or branched $C_{10}$-$C_{20}$-alkyl radical and the other radical is $C_1$-$C_4$-alkyl.

**23.** The fuel composition according to any of claims 19 to 22, wherein the quaternizing agent is selected from dimethyl or diethyl phthalates and dimethyl or diethyl oxalates.

## Revendications

**1.** Utilisation d'un produit de réaction comprenant un composé azoté quaternisé ou d'une fraction partielle de celui-ci contenant un composé azoté quaternisé, obtenue par purification à partir du produit de réaction,
en tant qu'additif de carburant en une proportion de 10 à 5 000 ppm en poids, par rapport à la quantité totale du carburant,
le produit de réaction étant obtenu par

a) transformation d'un composé azoté quaternisable, contenant au moins un groupe amino quaternisable avec un agent de quaternisation qui convertit l'au moins un groupe amino quaternisable en un groupe ammonium quaternaire,
l'agent de quaternisation étant l'ester méthylique ou éthylique d'un poly(acide carboxylique) aliphatique, cycloaromatique ou cycloaliphatique ; et
b) transestérification des groupes ester restants du produit de quaternisation de l'étape a) ;

la transestérification étant réalisée au moyen d'un alcool choisi parmi

a) des polyalcoxylates de formule générale 1

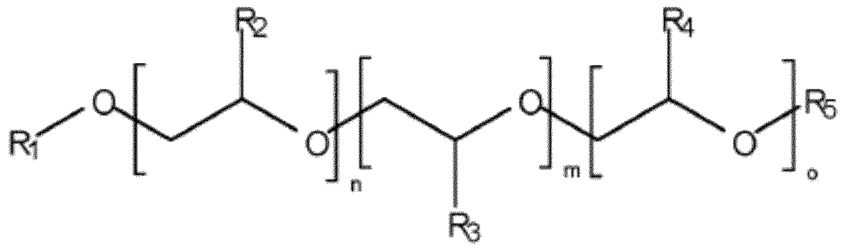

(1)

dans laquelle :

$R_1$ et $R_5$ représentent indépendamment l'un de l'autre H, alkyle, alcényle ou aryle, au moins l'un des deux radicaux représentant H ;
$R_2$, $R_3$ et $R_4$ représentent indépendamment les uns des autres H, $C_{1-30}$-alkyle, en particulier $C_{1-5}$-alkyle ; et n, m et o représentent indépendamment les uns des autres des valeurs entières de 0 à 100 ;
dotés de masses moléculaires de 40 à 10 000 g/mole, établies par détermination de l'indice d'OH (norme DIN 53240-2),

b) des polyalcoxylates amorcés par un polyol dotés de masses moléculaires de 100 à 10 000 g/mole, établies par détermination de l'indice d'OH (norme DIN 53240-2),
le composé a) ou b) utilisé pour la transestérification des groupes ester restants présentant un coefficient de distribution logP supérieur à 0,7, en particulier supérieur à 0,8, supérieur à 0,9, supérieur à 1, ou supérieur à 1,1, la valeur de logP, jusqu'à une valeur d'environ 6, étant déterminée d'après la directive 117 de l'OCDE à 25 °C dans le n-octanol/eau et, pour une valeur supérieure à 6, d'après la méthode ACD/LogP.

**2.** Utilisation selon la revendication 1, les polyalcoxylates de formule (1) étant des homopolymères ou des copolymères.

**3.** Utilisation selon la revendication 1, les polyalcoxylates amorcés par un polyol étant des homopolymères ou des copolymères.

**4.** Utilisation selon la revendication 3, le polyalcoxylate étant un homopolymère ou un copolymère d'oxydes d'alcényle, choisi parmi l'oxyde d'éthylène, l'oxyde de propylène et l'oxyde de butylène.

**5.** Utilisation selon la revendication 1, la transestérification étant réalisée au moyen d'un composé contenant des groupements polyoxy-$C_{2-4}$-alkylène, qui peuvent être obtenus par transformation de $C_{2-60}$-alcanols, de $C_{6-30}$-alcanediols, de $C_{1-30}$)-alkyl-cyclohexanols ou de $C_{1-30}$-alkylphénols avec 1 à 30 moles d'oxyde d'éthylène et/ou d'oxyde de propylène et/ou d'oxyde de butylène par hydroxyle.

**6.** Utilisation selon la revendication 1, la transestérification étant réalisée au moyen d'un polyéther amorcé par un alcool comportant 5 à 35 motifs de type oxyde de $C_{3-6}$-alkylène par molécule d'alcool.

**7.** Utilisation selon la revendication 6, l'alcool d'amorçage étant un alcanol à longue chaîne ou un phénol substitué par un alkyle à longue chaîne, le radical alkyle à longue chaîne représentant un radical $C_{6-18}$-alkyle linéaire ou ramifié.

**8.** Utilisation selon l'une quelconque des revendications précédentes en tant qu'additif pour la réduction de la consommation de carburant de moteurs diesel à injection directe et/ou pour la minimisation de la perte de puissance (powerloss) dans des moteurs diesels à injection directe.

**9.** Utilisation selon l'une quelconque des revendications 1 à 7 en tant qu'additif d'essence pour la réduction de dépôts dans le système d'admission d'un moteur à essence.

**10.** Utilisation selon l'une quelconque des revendications 1 à 7 en tant qu'additif de carburant diesel pour la réduction et/ou la prévention de dépôts dans les systèmes d'injection et/ou d'adhésion de soupape dans des moteurs diesel à injection directe.

**11.** Utilisation selon l'une quelconque des revendications précédentes, le composé azoté quaternisable étant choisi parmi :

a) au moins une alkylamine de formule générale suivante 3 :

$$R_aR_bR_cN \qquad (3)$$

dans laquelle au moins un des radicaux $R_a$, $R_b$ et $R_c$ représente un radical $C_{8-40}$-hydrocarbyle linéaire ou ramifié, saturé ou insaturé et les radicaux restants représentent des radicaux identiques ou différents $C_{1-6}$-hydrocarbyle linéaires ou ramifiés, saturés ou insaturés, ou
tous les radicaux $R_a$, $R_b$ et $R_c$ représentent des radicaux identiques ou différents $C_{8-40}$-hydrocarbyle linéaires ou ramifiés, saturés ou insaturés,

b) au moins une amine substituée par un polyalcène, contenant au moins un groupe amino quaternisable ;
c) au moins une amine substituée par un polyéther, contenant au moins un groupe amino quaternisable ; et
d) au moins un produit de réaction d'un agent d'acylation substitué par hydrocarbyle et d'un composé contenant un atome d'azote ou d'oxygène et contenant de plus au moins un groupe amino quaternisable ; et
e) des mélanges correspondants.

**12.** Utilisation selon l'une quelconque des revendications 1 à 11, l'agent de quaternisation étant un composé de formule générale 2

$$R_1OC(O)\text{-}A\text{-}C(O)OR_{1a} \qquad (2)$$

dans laquelle
$R_1$ et $R_{1a}$ représentent indépendamment l'un de l'autre un radical méthyle ou éthyle et A représente une liaison chimique ou un hydrocarbylène éventuellement monosubstitué ou polysubstitué, ou un radical arylène à un noyau ou cycloalkylène, éventuellement substitué.

**13.** Utilisation selon l'une quelconque des revendications précédentes, l'amine tertiaire quaternisable étant un composé de formule 3 dans laquelle au moins deux des radicaux $R_a$, $R_b$ et $R_c$ sont identiques ou différents et représentent un radical $C_{10}$-$_{20}$-alkyle linéaire ou ramifié et le radical restant représente $C_{1-4}$-alkyle.

**14.** Utilisation selon l'une quelconque des revendications précédentes, l'agent de quaternisation étant choisi parmi des phtalates de diméthyle ou de diéthyle et des oxalates de diméthyle ou de diéthyle.

**15.** Utilisation selon l'une quelconque des revendications précédentes, le carburant étant choisi parmi des carburants de type distillat intermédiaire, comme en particulier des huiles combustibles et des carburants de type diesel, des carburants de type biodiesel, des carburants de type essence et des carburants de type essence contenant un alcanol.

**16.** Composé azoté quaternisé selon la définition dans l'une quelconque des revendications 1 à 15.

**17.** Procédé pour la préparation d'un composé azoté quaternisé selon la revendication 16, comprenant

a) la transformation d'un composé azoté quaternisable, contenant au moins un groupe amino quaternisable avec un agent de quaternisation qui convertit l'au moins un groupe amino en un groupe ammonium quaternaire, l'agent de quaternisation étant l'ester méthylique ou éthylique d'un poly(acide carboxylique) aliphatique, cycloaromatique ou cycloaliphatique ; et
b) la transestérification étant réalisée avec un composé défini comme dans l'une quelconque des revendications 1 à 7.

**18.** Concentré d'additif, contenant en combinaison avec d'autres additifs de diesel ou d'essence, au moins un composé azoté quaternisé selon la définition dans la revendication 16 ou préparé selon la revendication 17.

**19.** Composition de carburant, contenant en une proportion de 10 à 5 000 ppm en poids, par rapport à la quantité totale du carburant, au moins produit de réaction comprenant un composé azoté quaternisé ou une fraction partielle de celui-ci contenant un composé azoté quaternisé, obtenue par purification à partir du produit de réaction, le produit de réaction étant obtenu par

a) la transformation d'un composé azoté quaternisable, contenant au moins un groupe amino quaternisable avec un agent de quaternisation qui convertit l'au moins un groupe amino en un groupe ammonium quaternaire, l'agent de quaternisation étant l'ester méthylique ou éthylique d'un poly(acide carboxylique) aliphatique, cycloaromatique ou cycloaliphatique ; et
b) la transestérification ou l'amidation du groupe ester restant du produit de quaternisation de l'étape a) ;
la transestérification étant réalisée avec un composé défini comme dans l'une quelconque des revendications 1 à 7.

**20.** Composition de carburant selon la revendication 19, le composé azoté quaternisable étant choisi parmi

a) au moins une alkylamine de formule générale 3 ci-dessus
b) au moins une amine substituée par un polyalcène, contenant au moins un groupe amino quaternisable ;
c) au moins une amine substituée par un polyéther, contenant au moins un groupe amino quaternisable ; et
d) au moins un produit de réaction d'un agent d'acylation substitué par hydrocarbyle et d'un composé contenant un atome d'azote ou d'oxygène et contenant de plus au moins un groupe amino quaternisable ; et
e) des mélanges correspondants.

**21.** Composition de carburant selon l'une quelconque des revendications 19 et 20, l'agent de quaternisation étant un composé de formule générale 2 ci-dessus.

**22.** Composition de carburant selon l'une quelconque des revendications 19 à 21, l'amine tertiaire quaternisable étant un composé de formule 3 dans laquelle au moins deux des radicaux $R_a$, $R_b$ et $R_c$ sont identiques ou différents et représentent un radical $C_{10}$-$_{20}$-alkyle linéaire ou ramifié et le radical restant représente $C_{1-4}$-alkyle.

**23.** Composition de carburant selon l'une quelconque des revendications 19 à 22, l'agent de quaternisation étant choisi parmi des phtalates de diméthyle ou de diéthyle et des oxalates de diméthyle ou de diéthyle.

| step | duration (minutes) | engine speed (rpm) +/- 20 | load (%) | torque (Nm) +/-5 | boost air after IC (°C) +/-3 |
|---|---|---|---|---|---|
| 1 | 2' | 1750 | (20) | 62 | 45 |
| 2 | 7' | 3000 | (60) | 173 | 50 |
| 3 | 2' | 1750 | (20) | 62 | 45 |
| 4 | 7' | 3500 | (80) | 212 | 50 |
| 5 | 2' | 1750 | (20) | 62 | 45 |
| 6 | 10' | 4000 | 100 | * | 50 |
| 7 | 2' | 1250 | (10) | 25 | 43** |
| 8 | 7' | 3000 | 100 | * | 50 |
| 9 | 2' | 1250 | (10) | 25 | 43** |
| 10 | 10' | 2000 | 100 | * | 50 |
| 11 | 2' | 1250 | (10) | 25 | 43** |
| 12 | 7' | 4000 | 100 | * | 50 |
| | Σ= 1 hour | | | | |

* for expected range see appendix 06.5

** target only

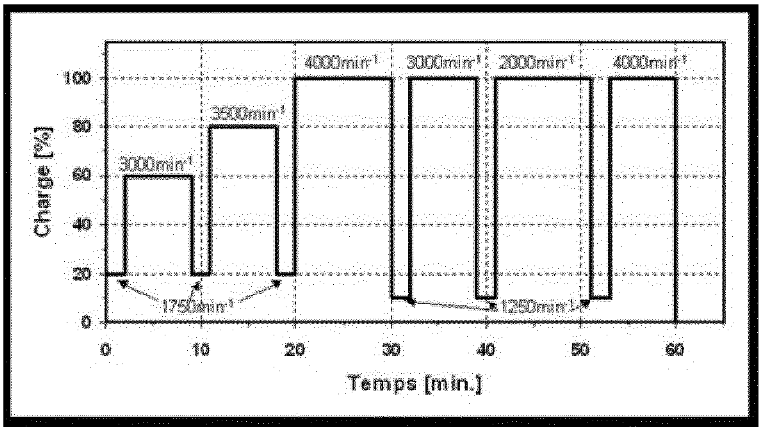


Fig.1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2033945 A
- EP 1254889 A
- JP 61012197 A
- GB 2496514 A
- WO 2013087701 A
- WO 2006135881 A
- WO 2013064689 A
- DE 4325237 A1
- DE 10243361 A1
- WO 2008060888 A
- US 20080113890 A
- US 3275554 A
- US 3438757 A
- US 3454555 A
- US 3565804 A
- US 3755433 A
- US 3822289 A
- US 5567845 A
- US 5496383 A
- US 5350429 A
- US 5492641 A
- US 4832702 A
- WO 2013000997 A
- DE 4319672
- WO 2008138836 A
- WO 9424231 A
- WO 9703946 A
- DE 19620262 A
- WO 9603367 A
- WO 9603479 A
- EP 476485 A
- EP 307815 A
- WO 8701126 A
- EP 639632 A
- EP 310875 A
- EP 356725 A
- EP 700985 A
- US 4877416 A
- DE 3838918 A
- EP 831141 A
- DE 3826608 A
- DE 4142241 A
- DE 4309074 A
- EP 452328 A
- EP 548617 A
- DE 10102913 A
- WO 9929748 A
- WO 2005054314 A
- WO 2004035715 A
- EP 061895 A
- US 4491455 A
- WO 9318115 A
- EP 261957 A
- WO 0044857 A
- WO 98004656 A
- US 6743266 B2
- WO 0047698 A


### In der Beschreibung aufgeführte Nicht-Patentliteratur


- **PERAUSKAS, A.A. et al.** *Perspectives in Drug Discovery and Design,* 2000, vol. 19, 99-116
- Diamine und höhere Amine. **KIRK - OTHEMER.** Encyclopedia of Chemical Technology. Interscience Publishers, 1965, vol. 7, 27-39
- *CHEMICAL ABSTRACTS,* 109-55-7
- **M. LONESCU.** Chemistry and Technology of Polyols for Polyurethanes. 2005
- **VOLLHARDT.** Organische Chemie. Wiley Verlag
- **MORRISON BOYD.** Lehrbuch der organischen Chemie VCh, Otera, Esterification. Wiley Verlag
- **N. A. PLATE ; V. P. SHIBAEV.** Comb-Like Polymers. Structure and Properties. *J. Poly. Sci. Macromolecular Revs.,* 1974, vol. 8, 117-253
- *CHEMICAL ABSTRACTS,* 68439-70-3
- *CHEMICAL ABSTRACTS,* 3302-10-1
- *CHEMICAL ABSTRACTS,* 553-90-2
- *CHEMICAL ABSTRACTS,* 10042-59-8
- *CHEMICAL ABSTRACTS,* 64742-94-5
- *CHEMICAL ABSTRACTS,* 104-75-6
- *CHEMICAL ABSTRACTS,* 112-90-3